(19) Europäisches Patentamt / European Patent Office / Office européen des brevets

(11) **EP 4 219 673 A1**

(12) **EUROPEAN PATENT APPLICATION**
published in accordance with Art. 153(4) EPC

(43) Date of publication:
**02.08.2023 Bulletin 2023/31**

(21) Application number: **21872585.1**

(22) Date of filing: **24.09.2021**

(51) International Patent Classification (IPC):
*C12M 1/00* $^{(2006.01)}$      *C12M 1/04* $^{(2006.01)}$
*C12M 1/26* $^{(2006.01)}$

(52) Cooperative Patent Classification (CPC):
**C12M 1/00; C12M 1/04; C12M 1/26**

(86) International application number:
**PCT/JP2021/035192**

(87) International publication number:
**WO 2022/065456 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2020 JP 2020160251**

(71) Applicant: NIKON CORPORATION
**Minato-ku**
**Tokyo 108-6290 (JP)**

(72) Inventors:
• **MORIYAMA ,Masaki**
**Tokyo 108-6290 (JP)**

• **ISHIZAWA, Naoya**
**Tokyo 108-6290 (JP)**
• **KOBAYASHI, Ryo**
**Tokyo 108-6290 (JP)**
• **TANAKA, Shuhei**
**Tokyo 108-6290 (JP)**
• **NAKAMURA, Taichi**
**Tokyo 108-6290 (JP)**
• **HAYASHI, Seri**
**Tokyo 108-6290 (JP)**
• **TAKUBO, Makiko**
**Tokyo 108-6290 (JP)**

(74) Representative: **Hoffmann Eitle**
**Patent- und Rechtsanwälte PartmbB**
**Arabellastraße 30**
**81925 München (DE)**

(54) **METHOD OF MANIPULATING ORGANISM AND DEVICE FOR MANIPULATING ORGANISM**

(57)    In a first aspect of the present invention, a manipulation method of an organism is provided, comprising: forming an air bubble by introducing gas into liquid in which an organism is submerged; controlling of energy before, during or after the forming of the air bubble, including controlling of difference (E1 - E2) obtained by subtracting, from surface free energy E1 at an interface between the gas and the organism, surface free energy E2 at an interface between the gas and the liquid; and manipulating the organism using the air bubble, by bringing the air bubble into contact with the organism during or after the controlling of energy.

**(Cont. next page)**

EP 4 219 673 A1

START

RECEIVE SAMPLE — S100

CAPTURE IMAGE — S120

RECEIVE MANIPULATION CONDITION — S140

PROCESSING FOR REPLACEMENT OR ADDING OF LIQUID TO BE PERFORMED? — S160
→ Yes → PROCESSING FOR REPLACEMENT OR ADDING OF LIQUID — S600
No

EQUIP WITH NOZZLE — S180

MOVE RELATIVE POSITION BETWEEN NOZZLE AND CELL — S200

FORM AIR BUBBLE — S300

PERFORM MANIPULATION — S400

REMOVE AIR BUBBLE — S500

MANIPULATION TARGET TO BE RELEASED? — S640
→ Yes → RELEASE TO DESIGNATED POSITION — S645
No

IS THERE OTHER MANIPULATION TARGET? — S650
→ Yes → REPLACE NOZZLE? — S660
No / No
Yes → TAKE NOZZLE OUT — S670

TAKE NOZZLE OUT — S680

END

*FIG.6*

2

**Description**

BACKGROUND

1. TECHNICAL FIELD

The present invention relates to a manipulation method of an organism and an organism manipulation device.

2. RELATED ART

In cell biology studies or the like, suction of a particular cell among many cells in a culture dish is performed. Patent document 1 discloses a cell suction assisting system for suction of a target cell by using a chip. Patent document 1: Japanese Patent Application Publication No. 2016-000007

[General disclosure] In a first aspect of the present invention, a manipulation method of an organism is provided. The manipulation method of an organism may include forming an air bubble by introducing gas into liquid in which the organism is submerged. The manipulation method of an organism may include controlling of energy before, during or after the forming of the air bubble, including controlling of difference (E1 - E2) obtained by subtracting, from surface free energy E1 at an interface between the gas and the organism, surface free energy E2 at an interface between the gas and the liquid. The manipulation method of an organism may include manipulating the organism using the air bubble, by bringing the air bubble into contact with the organism during or after the controlling of energy.

In a second aspect of the present invention, the controlling of energy may include decrease the difference (E1 - E2) or suppressing the difference (E1 - E2) from increasing. The manipulating may include allowing an organism to be attached to a gas-liquid interface between the air bubble and the liquid.

In a third aspect of the present invention, the controlling of energy may include increasing the difference (E1 - E2) or suppressing the difference (E1 - E2) from decreasing.

In a fourth aspect of the present invention, the manipulating may include the organism being squeezed by a gas-liquid interface between the air bubble and the liquid.

In a fifth aspect of the present invention, the controlling of energy may include controlling the surface free energy E2.

In a sixth aspect of the present invention, the controlling of energy may include replacing at least a part of the liquid with another liquid.

In a seventh aspect of the present invention, the controlling of energy may include adding another liquid to the liquid.

In an eighth aspect of the present invention, the controlling of energy may include adding a polar organic compound or inorganic salt to the liquid.

In a ninth aspect of the present invention, the controlling of energy may include replacing at least part of the gas with another gas.

In a tenth aspect of the present invention, the controlling of energy may include allowing an air bubble formed in the forming of the air bubble to be attached to the organism within ten seconds after the air bubble being formed.

In an eleventh aspect of the present invention, the controlling of energy may include allowing an air bubble formed in the forming of the air bubble to be attached to the organism after fifteen seconds or more has elapsed since the air bubble was formed.

In a twelfth aspect of the present invention, the controlling of energy may include enlarging or reducing a gas-liquid interface of the air bubble formed in the forming of the air bubble.

In a thirteenth aspect of the present invention, the controlling of energy may include controlling introduction speed or suction speed of the gas in the forming of the air bubble.

In a fourteenth aspect of the present invention, the manipulating may include manipulating the organism by bringing the interface between the liquid and the air bubble into contact with the organism and moving the interface.

In a fifteenth aspect of the present invention, the forming of the air bubble may include submerging an end of a flow channel to which the gas is injectable in liquid, and introducing the gas into the liquid from the end. The forming of the air bubble may further include moving relative positions of the flow channel and the organism to become closer.

In a sixteenth aspect of the present invention, the manipulating may include bringing the interface between the liquid and the air bubble into contact with the organism, and collecting the organism in contact with the interface by capturing the interface into the flow channel.

In a seventeenth aspect of the present invention, the manipulating may include capturing the interface into the flow channel within ten seconds after bringing the interface between the liquid and the air bubble into contact with the organism.

In an eighteenth aspect of the present invention, an organism manipulation device for manipulating an organism is provided. The organism manipulation device may include a flow channel into which gas is introduced from an end arranged in liquid in which the organism is submerged, to form an air bubble at the end. The organism manipulation device may include an energy control unit which controls a difference (E1 - E2) obtained by subtracting, from surface free energy E1 at an interface between the gas and the organism, surface free energy E2 at an interface between the gas and the liquid. The organism manipulation device may include a manipulation unit which manipulates the organism with the air bubble.

In a nineteenth aspect of the present invention, the energy control unit may have a volume control unit which causes the manipulation unit to control a pump connected to the flow channel, thereby controlling volume of the air bubble in the liquid.

In a twentieth aspect of the present invention, an organism manipulation device is provided. The organism manipulation device may include a flow channel having an end arranged in liquid including an organism, which is supplied in a container. The organism manipulation device may include a pump which introduces gas into the flow channel to form an air bubble at the end. The organism manipulation device may include a position control unit which controls a position of the container or the flow channel. The position control unit may move the container or the flow channel to a position at which the air bubble can be brought into contact with the organism. The pump may be form the air bubble at the position and to bring the air bubble into contact with the organism while increasing the air bubble's volume.

The summary clause does not necessarily describe all necessary features of the embodiments of the present invention. The invention may also include a sub-combination of the features described above.

BRIEF DESCRIPTION OF THE DRAWINGS

Fig. 1A illustrates an example of an device configuration of an organism manipulation device 100 in the present embodiment.

Fig. 1B illustrates an example of an device configuration of the organism manipulation device 100 in the present embodiment.

Fig. 2A illustrates an example of a schematic view showing a structure of a nozzle 49 in the present embodiment.

Fig. 2B illustrates an example of a schematic view showing a structure of the nozzle 49 in the present embodiment.

Fig. 3A illustrates an example of a schematic view showing a structure of the nozzle 49 in the present embodiment.

Fig. 3B illustrates an example of a schematic view showing a structure of the nozzle 49 in the present embodiment.

Fig. 4 illustrates an example of a schematic view showing an example of a method for collecting an organism in the present embodiment.

Fig. 5 illustrates an example of a specific configuration of an information processing device 170 in the present embodiment.

Fig. 6 illustrates an example of the flow of a method for manipulating an organism in the present embodiment.

Fig. 7A illustrates an example of a GUI image displayed on an output unit 160 in the present embodiment.

Fig. 7B illustrates an example of a GUI image displayed on the output unit 160 in the present embodiment.

Fig. 7C illustrates an example of a GUI image displayed on the output unit 160 in the present embodiment.

Fig. 7D illustrates an example of a GUI image displayed on the output unit 160 in the present embodiment.

Fig. 7E illustrates an example of a GUI image displayed on the output unit 160 in the present embodiment.

Fig. 8 illustrates an example of the flow for replacement or adding processing of liquid at S600 in the present embodiment.

Fig. 9A illustrates an example of the flow for moving relative positions between the nozzle 49 and a cell at S200 in the present embodiment.

Fig. 9B illustrates an example of the flow of moving relative positions between the nozzle 49 and the cell at S200 in the present embodiment.

Fig. 9C illustrates an example of the flow of moving relative positions between the nozzle 49 and the cell at S200 in the present embodiment.

Fig. 10A illustrates an example of the flow of forming an air bubble at S300 in the present embodiment.

Fig. 10B illustrates an example of the flow of forming an air bubble at S300 in the present embodiment.

Fig. 11A illustrates an example of the flow of performing manipulation at S400 in the present embodiment.

Fig. 11B illustrates an example of the flow of collecting cytoplasm and cytoplasmic membrane from a cell in the present embodiment.

Fig. 11C illustrates an example of exfoliating a cell by allowing it to be attached to the air bubble in the present embodiment.

Fig. 11D illustrates an example of a schematic view showing a method for collecting a cell in the present embodiment.

Fig. 11E illustrates an example of cells subjected to passage in the present embodiment.

Fig. 11F illustrates an example of cells subjected to passage in the present embodiment.

Fig. 11G illustrates an example of an analysis of collected cells in the present embodiment.

Fig. 11H illustrates an example of a schematic view showing retained cells in the present embodiment.

Fig. 11I illustrates an example of squeezed cells in the present embodiment.

Fig. 12A is an example of the flow of removing the air bubble at S500 in the present embodiment.

Fig. 12B is an example of the flow of removing the air bubble at S500 in the present embodiment.

Fig. 13A illustrates surface free energy variation when the cell is attached to the air bubble.

Fig. 13B illustrates a method for varying the surface free energy of a gas-liquid interface 255.

Fig. 13C illustrates a mechanism by which surface free energy of the gas-liquid interface 255 may be varied by a solute.

Fig. 13D illustrates surface free energy variation when the cell breaks in by being attached to the air bubble.

Fig. 13E illustrates that the surface free energy falls over time in the air bubble formed in liquid including contaminant.

Fig. 13F illustrates an example in which it is made easier for the cell to be attached to the gas-liquid interface 255 by replacing the liquid.

Fig. 13G illustrates an example in which the gas-liquid interface 255 is allowed to be attached to the cell while enlarging a surface area of the gas-liquid interface 255.

Fig. 14 illustrates an example of a hardware configuration of a computer.

DESCRIPTION OF EXEMPLARY EMBODIMENTS

Hereinafter, the invention will be described through embodiments of the invention, but the following embodiments do not limit the invention according to claims. In addition, not all combination of the features described in the embodiments are necessary for the solution of the invention. Note that in the drawings, same or similar parts are assigned with same reference signs, and duplicated descriptions may be omitted.

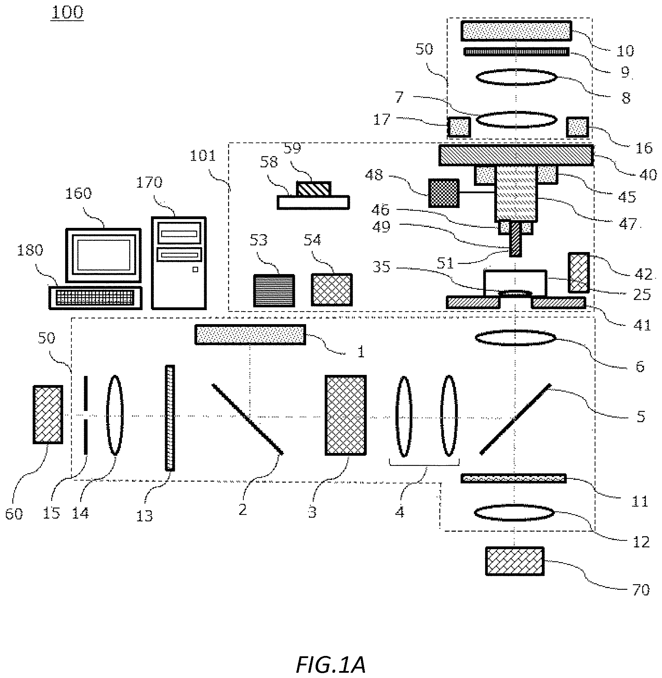

Fig. 1A illustrates an example of a device configuration of an organism manipulation device 100 in the present embodiment. The organism manipulation device 100 according to the present invention manipulates a minute organism such as a cell by using an interface between a gas and a liquid. For example, the organism manipulation device 100 can perform, on an organism, various manipulations such as attaching the organism to the interface and then separating the organism adhered on a solid phase. The organism manipulation device 100 includes a microscope unit 50, a camera 60, a camera 70, a manipulation unit 101, an output unit 160, an information processing device 170, and an input unit 180.

The microscope unit 50 is a device for observing or displaying a manipulation target 35 in an enlarged manner by using a microscope. The manipulation target 35 is an organism. The organism may be an organic creature. For example, the organism may be a cell. As an example, the cell may be an animal cell or a plant cell. As an example, the cell may be a living cell or a dead cell. In addition, for example, the organism may be a minute organism other than a cell. As an example, the minute organism may be a microorganism, a fungus, an alga, a biological tissue, a spheroid, or the like. In addition, the organism may contain an intracellular organelle.

The microscope unit 50 includes a fluorescence image observation light source 1, a dichroic mirror 2, an optical deflector 3, a relay lens 4, a dichroic mirror 5, an objective lens 6, a condenser lens 7, a condensing lens 8, a band-pass filter 9, a transmission image observation light source 10, a barrier filter 11, a projection lens 12, a barrier filter 13, a projection lens 14, a pinhole 15, a light source 16, and a light source 17.

The fluorescence image observation light source 1 is a light source used when a fluorescence image observation of the manipulation target 35 is performed. The manipulation target 35 may be labeled with one type or two or more types of fluorescent substances, or may not be fluorescent-labeled. The fluorescence image observation light source 1 emits, to the manipulation target 35, light to be excited or reflected.

The transmission image observation light source 10 is a light source used when a transmission image observation of the manipulation target 35 is performed. The transmission image observation light source 10 emits light to be transmitted through the manipulation target 35. The light to be transmitted through the manipulation target 35 may pass through the outside of the nozzle or may pass through the inside of the nozzle.

The configuration of the microscope unit 50 other than that described above will be described below. Note that the present invention is not limited to the example of the above description, and the microscope unit 50 may have a known configuration. For example, the configuration of the microscope unit 50 may have the configuration described in Japanese Patent Application Publication No. H7-13083 or Japanese Patent No. 3814869.

The camera 60 captures a fluorescence image of the manipulation target 35 to generate an image. The image data generated by the camera 60 may be recorded in the information processing device 170 (for example, a recording unit 190 to be described below) and/or output to the output unit 160. For example, the camera 60 may be a camera that captures a fluorescence image, but is not limited thereto. In the following description, the camera 60 is a camera that captures a fluorescence image.

The camera 70 captures a transmission image of a manipulation target 35, and generates an image. Data of the image generated by the camera 70 may be recorded inside the information processing device 170 (for example, a recording unit 190 described below), and/or output to an output unit 160. For example, the camera 70 may be a camera for capturing a transmission image, but it is not limited thereto. In the description below, the camera 70 is a camera for capturing a transmission image.

The camera 60 and the camera 70 each has an imaging sensor (not illustrated). The camera 60 and the camera 70 may each be a cooling camera. The cooling camera is a camera that can cool the imaging sensor to suppress the noise generated by heat. The imaging sensor may be a CMOS imaging sensor (Complementary Metal Oxide Semiconductor) or a CCD imaging sensor (Charge Coupled Device). The camera 60 and the camera 70 may be accommodated in an enclosure that is different from that of the microscope unit 50.

The manipulation unit 101 manipulates the manipulation target 35 by using a gas-liquid interface between gas and liquid. For example, the manipulation unit 101 forms an air bubble in liquid, and manipulates an organism (for example, a cell) in the liquid. The manipulation unit 101 has all or at least a part of a nozzle actuator 40, a sample actuator 41, a flow channel capturing camera 42, a light source 45, a light source 46, a pressure generation unit 47, a sensor unit 48, a nozzle 49, a flow channel 51, a flow channel replacement unit 53, a liquid storage unit 54, a sample lid 58, and a sample lid retaining unit 59.

The nozzle actuator 40 has a nozzle 49 mounted thereon via the pressure generation unit 47 to move the nozzle 49. As described below, the flow channel 51 is formed inside the nozzle 49, and a gas-liquid interface 255 such as an air bubble is form at the tip portion of or inside the flow channel 51. The nozzle actuator 40 may be operable in any direction of a vertical direction, a lateral direction, and an upward/downward direction. The nozzle actuator 40 may only be operable in the upward/downward direction. In this case, the movement of the nozzle actuator 40 in the vertical direction and the lateral direction may be operated by a stage of the microscope unit 50. The nozzle actuator 40 may only be operable in a vertical/lateral direction. In this case, the movement of the nozzle actuator 40 in the upward/downward direction may be operated by the stage of the microscope unit 50. The nozzle actuator 40 may be fixed without being operated. In this case, the movement of the nozzle actuator 40 in the vertical/lateral direction and the upward/downward direction may be operated by the stage of the microscope unit 50. The operation of the nozzle actuator 40 is controlled by a nozzle position control unit (not illustrated) of a volume control unit in the information processing device 170.

The sample actuator 41 moves the stage (not illustrated) on which the container 25 is mounted. The sample actuator 41 may be operable in any directionof a vertical direction, a lateral direction, and an upward/downward direction. The stage may have a transparent container 25 for accommodating the manipulation target 35 mounted thereon. The container 25 may be a culture dish filled with liquid. The sample actuator 41 may have one or more containers and/or tubes mounted thereon, but it is not limited thereto. The operation of the sample actuator 41 is controlled by a stage position control unit (not illustrated) of the volume control unit in the information processing device 170. Note that, the stage may be provided in the manipulation unit 101 or may be provided in the microscope unit 50.

The flow channel capturing camera 42 captures the tip portion of the nozzle 49. The flow channel capturing camera 42 may capture the air bubble formed at the tip portion of the nozzle 49. The captured image may be sent to an image processing unit in the information processing device 170. Based on the captured image, a volume control unit 200 may instruct the nozzle actuator 40 and/or the sample actuator 41 to move to relative positions with the nozzle 49 and the manipulation target 35. Note that, instead of the flow channel capturing camera 42, the camera 60 or the camera 70 or the like may capture the tip portion of the nozzle 49. It is not limited thereto, and in the descriptions below, the flow channel capturing camera 42 may be a microscope-accessory camera provided in the microscope unit 50. The camera provided in the microscope unit 50 may use a fluorescence image observation light source 1, a transmission image observation light source 10, a light source 16, a light source 17, a light source 45, and a light source 46 as lighting. The light source 16 and the light source 17 may be ring lighting, but it is not limited thereto.

The light source 45 and the light source 46 illuminate the nozzle 49 and/or the manipulation target 35. The light source 45 and the light source 46 may be ring lighting, but it is not limited thereto.

The pressure generation unit 47 generates pressure to be loaded onto the flow channel 51. The pressure generation unit 47 is connected to one oned of the flow channel 51 that is not in contact with the liquid, and supplies preset gas to said one end. For example, the pressure generation unit 47 may have an actuator for reciprocating a syringe pump and a plunger of the syringe pump. The plunger of the syringe pump may be pushed toward the flow channel 51 side by the actuator to charge gas to the flow channel 51, and the plunger of the syringe pump is pulled from the flow channel 51 side by the actuator to intake gas from the flow channel 51. Control of the pressure generation unit 47 is performed by the volume control unit in the information processing device 170.

Liquid in which the manipulation target 35 is submerged may be a complete medium, a basal medium, or a buffer, but it is not limited thereto. The complete medium is a medium including maintenance/growth factor required for maintenance and growth of a cell. The basal medium is a medium including very small amout of protein, amino acid or salt. The buffer is liquid in which the pH and osmotic pressure suitable for survival of a cell is maintained. Well known liquid, complete medium, basal medium, and buffer can be used.

Gas may be air. Gas may include moisture.

The sensor unit 48 has one or a plurality of sensors, to sense a state of liquid and gas in the nozzle 49 and the nozzle 49. For example, the sensor unit 48 may detect a position, speed, and acceleration of the nozzle 49. The sensor unit 48 may detect a position of the nozzle actuator 40, a pressure that is generated in the pressure generation unit 47, and a position of the plunger of the syringe pump in the pressure generation unit 47, or the like. The sensor unit 48 may detect the environmental temperature and the temperature of the liquid in the container 25. The sensor unit 48 may detect the humidity in the environment. In addition, the sensor unit 48 may detect the pH of the liquid in the container 25. The sensor unit 48 may detect the temperature and humidity of the gas in the nozzle 49. The sensor unit 48 sends these pieces of information to the information processing device 170 (for example, the volume control unit 200 described below). A sensor used for the sensor unit 48 can be a well known sensor. Note that, the sensor unit 48 may be an enclosure that is different from that of the pressure generation unit 47, or may be stored inside the pressure generation unit 47.

The nozzle 49 is equipment provided with the flow channel 51 described below. The nozzle 49 may be bar-shaped or may be flat plate-shaped.

The flow channel 51 passes therethrough liquid and gas being sucked (intake) or discharged (charged), and introduces the gas from an end 254 arranged in the liquid in which the manipulation target 35 is submerged. The flow channel 51 is provided inside the nozzle 49 to penetrate the nozzle 49 in the longitudinal direction. The pressure generation unit 47 is connected to the other end side of the flow channel 51.

The flow channel replacement unit 53 is equipment for retaining and disposing of the nozzle 49. When replacing the nozzle 49, the flow channel replacement unit 53 may remove the nozzle 49 that is equipped on the nozzle actuator 40 and dispose it to a nozzle disposal unit (not illustrated) of the flow channel replacement unit 53, and alternatively equip the nozzle actuator 40 with a nozzle 49 retained in a nozzle retaining unit (not illustrated) of the flow channel replacement unit 53. The flow channel replacement unit 53 may be omitted, and in that case, the nozzle 49 may be replaced manally by an operator.

The liquid storage unit 54 is equipment for retaining the liquid to be supplied to the container 25, and collecting the liquid from the container 25 to perform disposal. When replacing the liquid, the liquid storage unit 54 may collect, from the container 25, the liquid accommodated in the container 25 to dispose it to a liquid disposal unit (not illustrated) of the liquid storage unit 54, and refill the container 25 with liquid retained in a liquid retaining unit (not illustrated) of the liquid storage unit 54. The replacement of liquid may be replacement of liquid of the same type. The replacement of liquid may be replacement of liquid of different types. The liquid storage unit 54 may be omitted, and in that case, the liquid may be replaced manually by the operator.

The sample lid 58 is a lid that is attached to the container 25. The sample lid 58 may be attached to the container 25, or may be stored sample lid retaining unit 59. As required, the sample lid 58 may be taken out from the sample lid retaining unit 59 to be attached to the container 25, and may be removed from the container 25 to be stored in the sample lid retaining unit 59, by a sample actuator lid (not illustrated). In this case, the operation of the sample actuator lid may be controlled by a sample lid control unit (not illustrated) of the volume control unit 200 in the information processing device 170. The sample lid 58 and the sample lid retaining unit 59 may be omitted, and in that case, the sample lid 58 may manually be attached to the container 25 and removed from the container 25 by the operator.

The liquid storage unit 54 may store liquid that may change the difference (E1 - E2) of surface free energy for the liquid that is filled in the container 25, and the details thereof will be described below. Refill and/or disposal of liquid may be performed via a liquid flow channel (not illustrated) that couples the container 25 and the liquid storage unit 54. For example, the liquid flow channel may be one that couples the container 25 and the liquid retaining unit to allow the liquid to flow therethrough. For example, the liquid flow channel may couple the container 25 and the liquid disposal unit to allow the liquid to flow therethrough. The liquid flow channel may be a pippet. The replacement of liquid may be replacement of liquid of the same type. The replacement of liquid may be replacement of liquid of different types. The liquid storage unit 54 may be omitted, and in that case, the liquid may be replaced manually by the operator.

The output unit 160 outputs a processing result of the information processing device 170. For example, the output unit 160 outputs an image obtained by performing image processing by an inside (for example, the image processing unit 300 described below) of of the information processing device 170. For example, the output unit 160 is a monitor connected to the information processing device 170.

The information processing device 170 exchanges instructions and data with a microscope unit 50, the camera 60, the camera 70, the manipulation unit 101, the output unit 160, and the input unit 180. For example, the information processing device 170 is connected to the microscope unit 50 and the manipulation unit 101, to control the microscope unit 50 and the manipulation unit 101.

Specifically, the information processing device 170 switches the combination of the type of an objective lens 6 and/or the type of filter cube of a fluorescent filter arranged in the light path of the microscope unit 50. For example, both the type of the filter cube and the type of the objective lens 6 arranged in the light path are different in transmission image observation and fluorescence image observation. In addition, two types of fluorescence image observation only differ in the type of the filter cube arranged in the light path. In addition, the light source used is different for transmission image observation and fluorescence image observation (a transmission image observation light source 10 and a fluorescence image observation light source 1, respectively). Thus, the inside (for example, the imaging control unit described below) of the information processing device 170 may switch among one or more of the filter block, the objective lens 6, and the light source, depending on which of the at least any one or more of transmission image observation and one type or two or more types of fluorescence image observation is to be performed.

When fluorescence image observation is to be performed, the information processing device 170 turns the fluorescence image observation light source 1 on and turns the transmission image observation light source 10 off, in order to enable the light path of the fluorescence image observation light source 1. When fluorescence image observation is to be performed, the light incident from the fluorescence image observation light source 1 illuminates the manipulation target 35, via a dichroic mirror 2, a optical deflector 3, a relay lens 4, a dichroic mirror 5, and an objective lens 6.

When the manipulation target 35 is fluorescent-labeled, the fluorescent substance of the manipulation target 35 is excited, and emits fluorescence. The fluorescence emitted from the manipulation target 35 reaches the light receiving surface of the camera 60, via the objective lens 6, the dichroic mirror 5, the relay lens 4, the optical deflector 3, the dichroic mirror 2, a barrier filter 13, a projection lens 14, and a pinhole 15 (when the microscope unit 50 is a confocal microscope). At this time, a fluorescence image of the manipulation target 35 is formed at the camera 60. Note that, even when the manipulation target 35 is not fluorescent-labeled, the light incident from the fluorescence image observation light source 1 hits the manipulation target 35, and the manipulation target 35 can be observed by using the light reflected from the manipulation target 35.

When transmission image observation is to be performed, the information processing device 170 turns the transmission image observation light source 10 on and turns the fluorescence image observation light source 1 off, in order to enable the light path of the transmission image observation light source 10. When transmission image observation is to be performed, the light incident from the transmission image observation light source 10 illuminates the manipulation target 35, via a band-pass filter 9, a condenser lens 8, and a condenser lens 7. The light having transmitted through the manipulation target 35 reaches the light receiving surface of the camera 70, via the objective lens 6, the dichroic mirror 5, the barrier filter 11, and the projection lens 12. At this time, a transmission image of the manipulation target 35 is formed at the camera 70. Note that, when it is difficult to see the end of the nozzle 49 in fluorescence observation, transmission image observation may be performed as well.

In addition, the information processing device 170 controls relative positions of the nozzle 49 and the stage of the manipulation unit 101. Further, in addition to control of the microscope unit 50 and the manipulation unit 101, the information processing device 170 may perform image processing such as receiving the manipulation target 35 captured by the camera 60 and/or the camera 70, and/or an image captured by the flow channel capturing camera 42 of the manipulation unit 101, and generating one composite image from a plurality of images. The information processing device 170 may perform control of other operations or data processing or the like of the organism manipulation device 100, as required. The configuration of the information processing device 170 will be described below.

The input unit 180 inputs instructions, data, or the like from the operator to the information processing device 170. For example, the input unit 180 inputs instructions related to selection of a manipulation application for the manipulation target 35 from the operator. In addition, the input unit 180 inputs operating amount of the nozzle actuator 40 and/or the sample actuator 41 from the operator to the information processing device 170. For example, the input unit 180 is a keyboard or a mouse connected to the organism manipulation device 100.

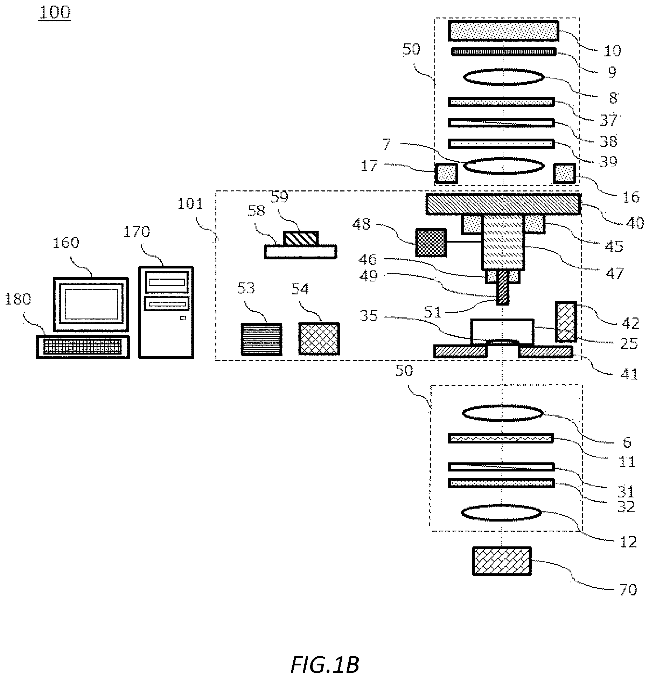

Fig. 1B is another example of an device configuration of the organism manipulation device 100 in the present embodiment. In Fig. 1B, the organism manipulation device 100 is illustrated in a case where the microscope unit 50 is a phase microscope or a differential interference microscope. When the microscope unit 50 is a phase microscope, the microscope unit 50 may include an objective lens 6 (which may include a phase plate), a condenser lens 7, a condenser lens 8, a band-pass filter 9, a transmission image observation light source 10, a barrier filter 11, a projection lens 12, a light source 16, a light source 17, and a ring diaphragm 39. When the microscope unit 50 is a differential interference microscope, the microscope unit 50 may include an objective lens 6, a condenser lens 7, a condenser lens 8, a band-pass filter 9, a transmission image observation light source 10, a barrier filter 11, a projection lens 12, a light source 16, a light source 17, a Nomarski prism 31, an analyzer (polarizing plate) 32, a polarizer (polarizing plate) 37, and a Nomarski prism 38. In addition, it is not limited thereto, and the microscope unit 50 may include components other than those described above. For example, the phase microscope may include the Nomarski prism 31 or the like, and the differential interference microscope may include the ring diaphragm 39 or the like. Description of Fig. 1A may be applied to components of the organism manipulation device 100 other than the microscope unit 50.

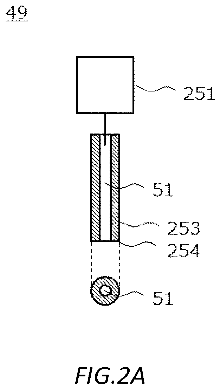

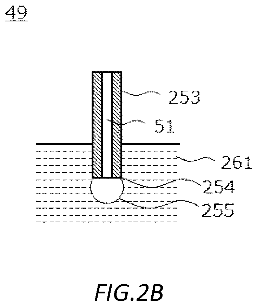

Fig. 2A and Fig. 2B are examples of a schematic view showing a structure of the nozzle 49 in the present embodiment. In Fig. 2A, the nozzle 49 includes a cylindrical portion 253 having a flow channel 51. The cylindrical portion 253 may have a hollow cylincrial shape. In this case, the shape of a cross section of the cylindrical portion 253 orthogonal to the axial direction is a circle. In addition, the flow channel 51 may be connected to a pump 251 (for example, a syringe pump of the pressure generation unit 47) at one end side. The pump 251 receives an instruction from the information processing device 170 (for example, the volume control unit 200 described below), and regulates the pressure and/or volume of the air bubble by regulating the amount of gas that is charged to the flow channel 51 or taken in from the flow channel 51.

In Fig. 2B, when the end 254 on the side to which the pump (not illustrated; for example, a syringe pump of the pressure generation unit 47) of the cylindrical portion 253 is not connected is arranged in the liquid 261, the pump can form the air bubble at the end 254 by charging gas to the flow channel 51. In this case, a gas-liquid interface 255 is formed at a boundary between the gas of the air bubble and the liquid 261. Note that, the shape of the air bubble is not limited to a spherical shape, and may be deformed according to the shape of the end 254. Here, when the gas is retained at the end 254 of the flow channel 51, the gas-liquid interface 255 is formed at the end 254 of the flow channel 51, but when both gas and liquid exist inside the flow channel 51, gas-liquid interface 255 may be formed at the interface between them inside the flow channel 51.

When the gas-liquid interface 255 is brought into contact with an organism which is adhered to a solid phase such as a inner bottom surface of the container 25 in the liquid 261, by moving the gas-liquid interface 255, force can be applied to the organism by the gas-liquid interface 225, and the organism can be exfoliated from the solid phase to be attached to the gas-liquid interface 255. The movement of the gas-liquid interface 255 may be performed by moving the nozzle 49 at which air bubble is formed by the nozzle actuator 40, may be performed by moving the liquid, or may be performed by changing the volume of the air bubble. Here, the solid phase may be a surface to which an adherent cell can be adhered and on which it can be cultured. For example, the solid phase may be glass; resin such as polystylene; metal; a surface coated with one or more types of extracellular matrix component selected from collagen, fibronectin, laminin, polylysine; a surface coated with various polymers (as an example, a polymer for which hydrophilicity or adsorption to a cell can be controlled), or the like, but it is not limited thereto. Note that, although the gas-liquid interface 255 is formed by an interface between gas and liquid in the present embodiment, it is not limited thereto, and it may be changed according to the phase or substance that is in contact with the interface. Further, when the gas-liquid interface 255 is brought into contact with an organism that is adhered to a solid phase such as the inner bottom surface of the container 25 in the liquid 261, the organism may be exfoliated from the solid phase by moving the gas-liquid interface 255 after allowing the organism to be attached to the gas-liquid interface 255, and the organism may be pressed by the gas-liquid interface 255 while the organism is not attached to the gas-liquid interface 255. Details of the method for exfoliating the organism from the solid phase will be described below.

An opening area of the flow channel 51 at the end 254 is not particularly limited, as long as it has a size in which the organism can be manipulated. For example, the opening area may be greater than the adhering area per organism. The shape of the end 254 is not particularly limited. In addition, the inner diameter of the flow channel 51 may be the same across the entire length of the cylindrical portion 253. In addition, instead of the air bubble formed at the end 254, the gas-liquid interface 255 may be formed inside the flow channel 51.

In addition, the flow channel 51 may be one that captures the gas-liquid interface 225 into the flow channel 51 to further collect the organism by taking in gas in the air bubble to which the organism is attached by the pump 251. Alternatively, the nozzle 49 may be one that further includes another flow channel for collecting the organism, apart from the flow channel 51.

In the embodiments of Fig. 2A and Fig. 2B, since only one flow channel 51 is formed in the nozzle 49 and only one pump 251 is connected to the flow channel 51, the configuration is very simple and minimal, which allows maintenance and cost of the organism manipulation device 100 to be reduced.

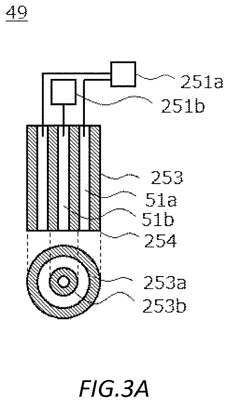

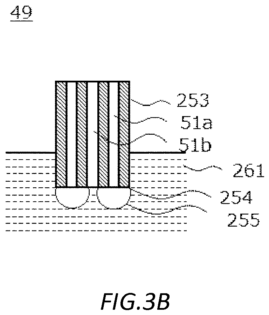

Fig. 3A and Fig. 3B are examples of a schematic view showing a structure of the nozzle 49 in another embodiment. In the examples of Fig. 2A and Fig. 2B, a case is shown where the flow channel for forming the air bubble and the flow channel for collecting the organism are the same, but in the examples of the Fig. 3A and Fig. 3B, a case in shown where the flow channel for forming the air bubble and the flow channel for collecting the organism are different.

In Fig. 3A, the cylindrical portion 253 of the nozzle 49 has a double structure including an outer cylinder 253a and an inner cylinder 253b. There is a first flow channel 51a through which gas flows between the outer cylinder 253a and the inner cylinder 253b, and there is a second flow channel 51b inside the inner cylinder 253b. For example, the first flow channel 51a may be a gas-supplying flow channel, and the second flow channel 51b may be a gas-collecting flow channel.

In addition, the first flow channel 51a and the second flow channel 51b of the nozzle 49 may be connected to the first pump 251a and the second pump 251b, respectively, at one end side. For example, the pressure generation unit 47 may have a first pump 251a and a second pump 251b, as syringe pumps, each of which may be controlled by a separate actuator. The actuator having received an instruction from the volume control unit described below regulates the amount of gas to be charged to or taken in from the first flow channel 51a and the second flow channel 51b, and thereby the first pump 251a and the second pump 251b respectively regulate the pressure and/or volume of the air bubble. The shape of the cross section of the cylindrical portion 253 orthogonal to the axial direction is a doughnut shape in the first flow channel 51a, and a circle in the second flow channel 51b.

In Fig. 3B, when the end 254 on the side to which the first pump 251a and the second pump 251b of the cylindrical portion 253 are not connected is arranged in the liquid 261, the first pump 251a can form the air bubble at the end 254 by charging gas to the first flow channel 51a. In this case, a gas-liquid interface 255 is formed at a boundary between the gas of the air bubble and the liquid 261.

When the gas-liquid interface 255 is brought into contact with an organism which is adhered to a solid phase in the liquid 261, by moving the gas-liquid interface 255, the organism can be exfoliated from the solid phase so that the organism is attached to the gas-liquid interface 255. The second pump 251b may capture the gas-liquid interface 225 into the flow channel 51 and collect the organism by taking the air bubble to which the organism is attached in via the second flow channel 51b.

Note that, although, in the above-described embodiment, the first pump 251a forms the air bubble by charging gas into the first flow channel 51a, and the second pump 251b collects the organism by taking in the gas at the second flow channel 51b, the second pump 251b may form the air bubble by charging gas to the second flow channel 51b and the first pump 251a may collect the organism by taking in gas at the first flow channel 51a. In addition, the first pump 251a and the second pump 251b may be the same syringe pump provided in the pressure generation unit 47. One of the first pump 251a and the second pump 251b may be omitted.

In the embodiment of Fig. 3A and Fig. 3B, since it is possible to simultaneously allow the organism to be attached to the gas-liquid interface 255 by forming an air bubble at one flow channel and collect the attached organism at the other flow channel, there is an effect that the time for collecting the cell is reduced. Note that, although, in Fig. 3A and Fig. 3B, an embodiment in which the shape of the cross section of the cylindrical portion 253 orthogonal to the axial direction is a doughnut shape in the first flow channel 51a and a circle in the second flow channel 51b shown, the shape of the cross section is not limited to a doughnut shape or a circle, and as long as there are two flow channels, attaching collecting of the organism can be simultaneously performed.

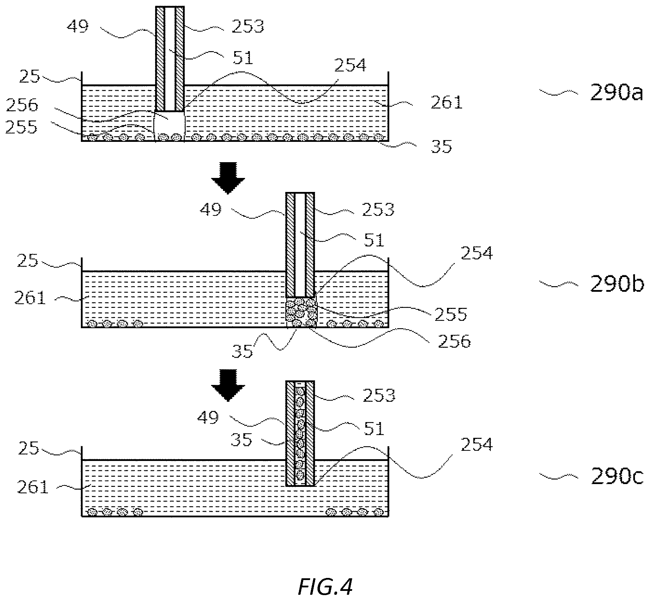

Fig. 4 is a schematic view illustrating an example of a method for collecting the manipulation target 35 in the present embodiment. At 290a, the manipulation target 35 is cultured at a solid phase on the inner bottom surface of the container 25. The manipulation target 35 may be cultured in liquid 261. For example, the manipulation target 35 is an adherent cell. For example, the liquid may be a complete medium.

At 290a, the pump 251 charges gas to the flow channel 51 of the nozzle 49, and forms an air bubble 256 at the end 254 of the nozzle 49. By bringing the air bubble 256 into contact with the manipulation target 35, the gas-liquid interface 255 between gas and the liquid 261 contacts the manipulation target 35. In this case, the pump 251 adjusts the charging and intaking of gas to maintain the formed air bubble 256. In this manner, manipulation of the manipulation target 35 by the air bubble 256 can be easily performed.

Then, at 290b, the nozzle actuator 40 moves the nozzle 49 along the surface of the solid phase with the air bubble 256 remained in contact with the manipulation target 35. Although description is made in Fig. 4 of how the nozzle actuator 40 moves the nozzle 49 from the left-to-right direction, the direction in which the nozzle 49 is moved is not limited as long as it is in a direction parallel to the surface of the solid phase. By moving the nozzle 49 with the nozzle actuator 40, the gas-liquid interface 255 is moved to allow the manipulation target 35 to be attached to the gas-liquid interface 255, and the manipulation target 35 can be exfoliated from the solid phase. At this time, the exfoliated manipulation target 35 is attached to the gas-liquid interface 255 of the air bubble 256. The volume control unit 200 controls the pump 251 to regulate the pressure and/or volume of the gas charged or taken in to change the size of the air bubble 256, and thereby the manipulation target 35 in a desired range can be exfoliated. Note that, instead of moving the nozzle 49 along the surface of the solid phase, the stage may be moved.

Then, at 290c, the pump 251 takes in the gas in the flow channel 51, and thereby the manipulation target 35 attached to the gas-liquid interface 255 may be collected. In this manner, by using the air bubble 256 formed at the nozzle 49, the manipulation target 35 can be selectively exfoliated from the solid phase to be collected.

Note that, the when the manipulation target 35 is an adherent cell that is strongly adhered to the solid phase, adhesion of the adherent cell may be relaxed in advance before performing the method of Fig. 4. Relaxing the adhesion of the adherent cell can be performed by using a well known method as described below.

In Fig. 4, although an example in which the gas-liquid interface 255 is moved by moving the nozzle 49 and the cell is exfoliated to be collected has been described, the movement of the gas-liquid interface 255 is not limited to the above-described example. For example, the volume of the air bubble 256 may be increased after bringing the air bubble 256 into contact with the manipulation target 35. In this case, the contact surface between the air bubble 256 and the solid phase is expanded, and the manipulation target can be selectively exfoliated from the solid phase to be collected. For example, the nozzle actuator 40 may move the nozzle 49 to become closer to the solid phase after the air bubble 256 is brought into contact with the manipulation target 35. Also in this case, the contact surface between the air bubble 256 and the solid phase is expanded due to the air bubble 256 being pressed against the solid phase, and the manipulation target can be selectively exfoliated from the solid phase to be collected.

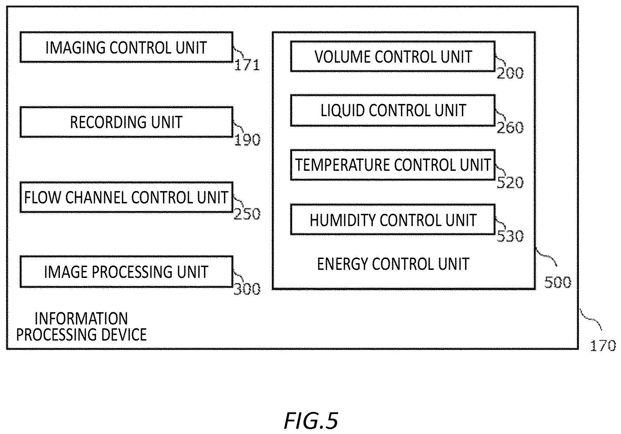

Fig. 5 illustrates an example of a specific configuration of an information processing device 170 in the present embodiment. The information processing device 170 includes an imaging control unit 171, a recording unit 190, a flow channel control unit 250, an image processing unit 300, and an energy control unit 500.

The imaging control unit 171 performs control of the fluorescence image observation light source 1, the objective lens 6, the fluorescent filter, the transmission image observation light source 10, the flow channel capturing camera 42, the light source 45, the light source 46, the camera 60, and the camera 70, or the like, described in Fig. 1A and Fig. 1B. For example, when an image capturing condition of the manipulation target 35 is input to the input unit 180, in accordance with the input image capturing condition, the imaging control unit 171 performs an adjustment required for each image-capturing, among switching of cameras, switching of the types of the objective lens 6 in the microscope unit 50, switching of the light sources, switching of the types of the fluorescent filter, the position of the stage and the height of the objective lens 6. After the imaging control unit 171 performed an adjustment required, the one or more cameras of the flow channel capturing camera 42, the camera 60, and the camera 70 performs image-capturing of the manipulation target 35 or the nozzle 49 to generate an image of the manipulation target 35 or the nozzle 49. One or more cameras send the data of the generated image to the image processing unit 300. In addition, the generated image data may also be recorded in the recording unit 190 and/or output to the output unit 160.

The recording unit 190 may be a memory, a hard disk drive, or an external recording medium, but it is not limited thereto. The information processing device 170 has a central processing unit (CPU), and said CPU executes a computer program recorded on the recording unit 190 to achieve the information processing device 170 or the like.

The flow channel control unit 250 controls retaining, equipping, and disposing of the nozzle 49. The flow channel control unit 250 receives, from the input unit 180, an instruction on manipulation related to equipping and disposing of the nozzle 49 from the operator. In accordance with the instruction received, the flow channel control unit 250 sends, to the flow channel replacement unit 53, an instruction to take the nozzle 49 out from a flow channel retaining unit of the flow channel replacement unit 53, equip the nozzle actuator 40 with the nozzle 49 or remove the nozzle 49 equipped on the nozzle actuator 40, and dispose it to a flow channel disposal unit of the flow channel replacement unit 53.

The image processing unit 300 receives images captured by the flow channel capturing camera 42, the camera 60, and the camera 70, from these cameras. The image processing unit 300 may use a plurality of the images received to synthesize them into one composite image. For example, the image processing unit 300 may synthesize a fluorescence image captured by the camera 60 and a transmission image captured by the camera 70 to generate a composite image. The image processing unit 300 may record the images received from these cameras and/or the composite image in the recording unit 190, and/or output them to the output unit 160.

The energy control unit 500 controls the gas supplied to the flow channel 51, the liquid filled in the container 25, a relative positional relationship between the manipulation target 35 and the nozzle 49, and the environment of the manipulation unit 101 such as the temperature and humidity. In this manner, the energy control unit 500 controls the difference (E1 - E2) obtained by subtracting the surface free energy E2 at the interface between gas and liquid from the surface free energy E1 at the interface between gas and the organism. The value of the difference (E1 - E2) may be a positive value, zero, or a negative value. The smaller the value of the difference (E1 - E2), the better the organism attaches to the air bubble. Here, since the value of the surface free energy E1 is constant, there is less room for the energy control unit 500 to control. Therefore, the value of this difference (E1-E2) can be controlled to a preset value by controlling the surface free energy E2 at the interface between gas and liquid with the energy control unit 500. The energy control unit 500 may include all or some of the volume control unit 200, a liquid control unit 260, a temperature control unit 520, and a humidity control unit 530.

The volume control unit 200 controls the pressure and/or volume of the gas charged to or taken in from the flow channel 51, movement of the nozzle 49, and movement of the stage. The volume control unit 200 controls the actuator of the pressure generation unit 47 in the manipulation unit 101 to charge gas to the syringe pump connected to the flow channel 51 or take in gas to the syringe pump, thereby controlling the pressure and/or volume of the air bubble formed at the flow channel 51. For example, the volume control unit 200 controls the actuator such that the syringe pump is pressed or pullled at a predetermined pressure or distance.

The volume control unit 200 may control the introduction speed (air-charge speed) and/or suction speed (air intake speed) of the gas. Specifically, the volume control unit 200 may control the actuator of the pressure generation unit 47 to regulate the speed at which the syringe pump is moved or the pressure at which the syringe pump is pressed, thereby control the speed at which the air bubble is formed and/or the speed at which the manipulation target 35 is collected. For example, by increasing the speed by which the syringe pump is pressed or increasing the pressure at which the syringe pump is pressed to increasing the speed at which gas is taken in to the syringe pump, the speed at which the manipulation target 35 is collected can be increased to facilitate collection of the manipulation target 35.

In addition, the volume control unit 200 may receive information on the introduction speed and suction speed of the gas from the pressure generation unit 47 or the sensor unit 48. The volume control unit 200 may calculate, from these pieces of information, the pressure and volume of the air bubble formed at the flow channel 51. The volume control unit 200 may include all or some of a nozzle position control unit, a stage position control unit, an air-charge control unit, and an air-intake control unit.

The nozzle position control unit controls the nozzle actuator 40 to control the operation of the nozzle 49, the air stream in the air bubble associated with the operation of the nozzle 49, and the movement of the gas-liquid interface 255 associated with the operate of the nozzle 49. In addition, the nozzle position control unit receives location information of the nozzle 49 from the sensor unit 48 or the nozzle actuator 40.

The stage position control unit controls the sample actuator 41, to control the operationg of the stage on which the container 25 accommodating the manipulation target 35 is mounted, air stream in the air bubble associated with the operation of the stage, and movement of the gas-liquid interface 255 associated with the operation of the stage. In addition, the stage position control unit receives location information of the stage and the manipulation target 35 from the sensor unit 48 or the sample actuator 41.

In addition, as illustrated in Fig. 3A or the like, when the nozzle 49 includes a gas-supplying flow channel for supplying (charge air) gas and a gas-collecting flow channel for collecting (intaking) gas, the air-charge control unit may control the first pump 251a connected to the gas-supplying flow channel, thereby controlling the volume of gas charged to the gas-supplying flow channel. The volume control unit 200 or the air-charge control unit receives information on the amount of gas charged to the gas-supplying flow channel to the nozzle actuator 40, the pressure generation unit 47, or the sensor unit 48.

In addition, as illustrated in Fig. 3A or the like, when the nozzle 49 includes a gas-supplying flow channel for supplying (charging) gas and a gas-collecting flow channel for collecting (intaking) gas, the air-intake control unit may control the second pump 251b connected to the gas-collecting flow channel, thereby controlling the amount (volume) of gas taken in from the gas-collecting flow channel. The volume control unit 200 or the air-intake control unit receives information on the amount of gas taken in from the gas-collecting flow channel to the nozzle actuator 40, the pressure generation unit 47, or the sensor unit 48.

The liquid control unit 260 controls retaining, refilling, and disposing of the liquid. The liquid control unit 260 receives, from the input unit 180, instruction from the operator on manipulation related to refilling and disposing of the liquid. In accordance with the instruction received, the liquid control unit 260 sends, to the liquid storage unit 54, an instruction to refill the container 25 with liquid retained in the liquid retaining unit of the liquid storage unit 54 or collect, from the container 25, the liquid accommodated in the container 25 to dispose it to the liquid disposal unit of the liquid storage unit 54.

Specifically, the liquid control unit 260 may send, to the liquid storage unit 54, an instruction to dispose all or at least some of the liquid accommodated in the container 25 and refill the container 25 with liquid retained in the liquid retaining unit of the liquid storage unit 54, thereby replacing all or at least some of the liquid accommodated in the container 25. In addition, the liquid control unit 260 may send, to the liquid storage unit 54, an instruction to add liquid to the container 25 by refilling the container 25 with the liquid retained in the liquid retaining unit of the liquid storage unit 54 without disposing the liquid accommodated in the container 25. The replacing or adding of the liquid may be performed via the liquid flow channel with which the liquid retaining unit and the container 25 are coupled.

The temperature control unit 520 controls the temperature of the liquid. For example, the temperature control unit 520 may control the temperature of the liquid accommodated in the container 25. For example, the temperature control unit 520 may control the temperature of the liquid retained in the liquid retaining unit of the liquid storage unit 54. The temperature control unit 520 may be connected to a temperature adjustment device such as a heater or a cooler connected to the liquid storage unit 54 and/or the container 25. For example, the temperature adjustment device may be an electric heater or a Peltier-type cooler. In this case, the temperature control unit 520 may control the temperature adjustment device such that the temperature adjustment device regulates the liquid to be at a preset temperature. The temperature control unit 520 may control the value of surface free energy at the gas-liquid interface 255 by regulating the temperature of the liquid. Details of the mechanism by which the value of surface free energy at the gas-liquid interface 255 is controlled by regulating the temperature of the liquid will be described below.

The humidity control unit 530 controls humidity of the gas. For example, the humidity control unit 530 may control the humidity of gas supplied from the syringe pump. The humidity control unit 530 may be connected to a humidifier connected to the pressure generation unit 47. In this case, the humidity control unit 530 may control the humidifier such that the humidifier regulates the gas to be at a preset humidity. The humidity control unit 530 may control the value of surface free energy at the gas-liquid interface 255 by regulating the humidity of the gas. Details of the mechanism by which the value of surface free energy at the gas-liquid interface 255 is controlled by regulating the humidity of the gas will be described below.

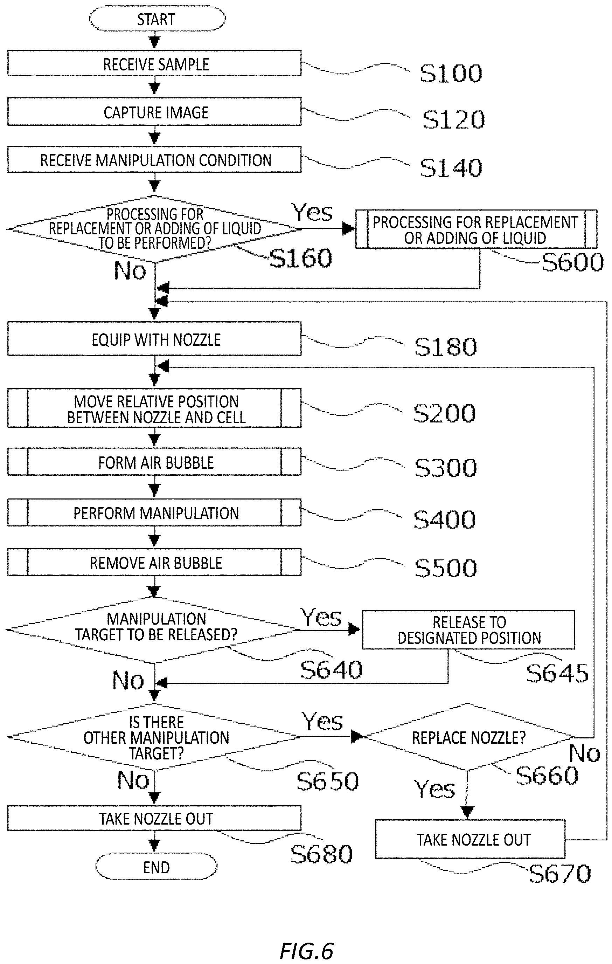

Fig. 6 is an example of the flow of a method for manipulating an organism in the present embodiment. The organism which is manipulation target 35 in the present embodiment can be manipulated by performing processing of S100 - S680 in Fig. 6. Note that, for convenience of description, the processing of S100 to the processing of S680 will be described in order; however, at least some processing may be executed in parallel, and each step may be interchanged and executed within a range not deviating from the spirit of the present invention.

First, at S100, the sample actuator 41 receives the organism to be the manipulation target 35. For example, at S100, the sample actuator 41 has a container 25 accommodating the manipulation target 35 along with the liquid mounted on the stage. In order to perform manipulation on the manipulation target 35, the lid of the container 25 may be removed. The lid may be replaced by an actuator for replacing the lid, or may be replaced manually by an operator. After the sample actuator 41 received the organism to be the manipulation target 35, the information processing device 170 advances the processing to S120.

Then, at S120, the camera 60 or the camera 70 performs image-capturing of a wide-range observation field including the manipulation target 35 to generate an image. The imaging control unit 171 sets the observation method to low-magnification transmission image capturing, and sends an instruction to the camera 70 to perform image-capturing of the observation field. The imaging control unit 171 may set the observation method to fluorescence image capturing and send an instruction to the camera 60 to perform image-capturing of the observation field. The imaging control unit 171 may receive input of the image capturing condition from the operator via the input unit 180. The camera 60 or the camera 70 performs image-capturing of the observation field. The image processing unit 300 may record the captured image to the recording unit 190 and/or output it to the output unit 160. After the camera 60 or the camera 70 performed image-capturing of the observation field, the imaging control unit 171 advances the processing to S140.

Then, at S140, the information processing device 170 receives an input related to the manipulation target 35 and the type of the manipulation from the operator via the input unit 180. The manipulation target 35 may be a sigle cell, may be a cell population (colony), may be cytoplasm of a cell and/or cytoplasmic membrane, or may be a spheroid, but it is not limited thereto. The type of the manipulation may be collection of the manipulation target 35, may be removal of the manipulation target 35, may be retaining of the manipulation target 35, or may be squeezing of the manipulation target 35, but it is not limited thereto. In addition, the information processing device 170 may receive an input related to the manipulation target 35 the type of manipulation after receiving an input related to the difference (E1-E2) obtained by subtracting, from the surface free energy E1 at the interface between gas and the organism, the surface free energy E2 at the interface between gas and liquid. Further, when an input related to the manipulation target 35 and the type of manipulation is received, the information processing device 170 may output a manipulation candidate for which the difference (E1-E2) is to be controlled.

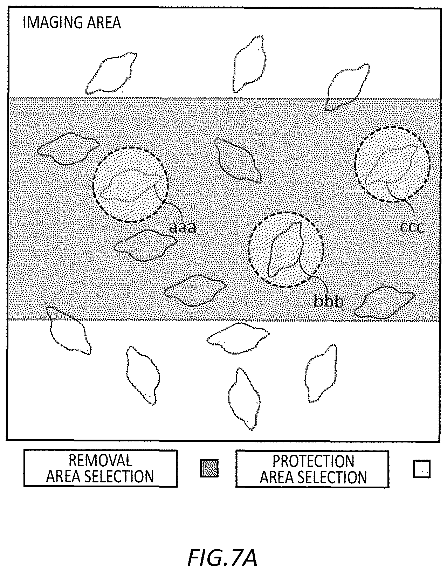

Fig. 7A is an example of a GUI (graphical user interface) image displayed on the output unit 160, which is obtained by capturing an observation field by the camera 60 or the camera 70. In Fig. 7A, cells aaa, bbb, and ccc, which are the manipulation target, are designated as the manipulation target 35 via the input unit 180. As shown in Fig. 7A, an organism that is to be the manipulation target 35 is arbitrarily designated via the input unit 180. As shown in Fig. 7A, a removal area and/or a protection area may be provided in the observation field such that the removal area and/or protection area is selected in the GUI image. By providing a removal area, a risk that a cell other than the cell to be collected is collected can be reduced. In addition, by providing a protection area, the risk of accidentally removing a cell to be collected, when removing a cell in the removal area, can be reduced.

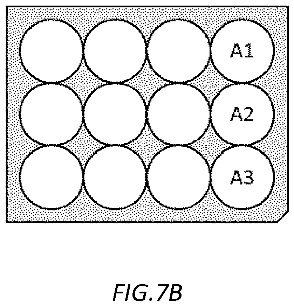

Fig. 7B is an example of a GUI image in which the collection destination and movement destination of the cells aaa, bbb, and ccc, which are the manipulation target 35, displayed on the output unit 160 is respectively designated as A1, A2, and A3 in the twelve-hole plate. As shown in Fig. 7B, the movement destination is arbitrarily designated via the input unit 180. For example, the movement destination may be a same plate, may be a different plate, may be a petri dish, may be a micro test tube, may be a PCR tube, or may be a conical tube.

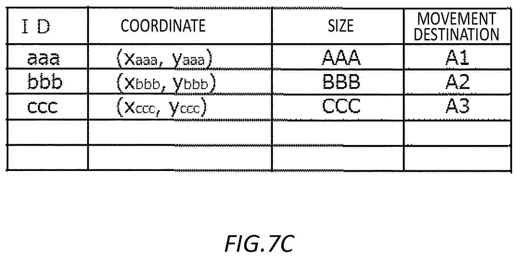

Fig. 7C is an example of a GUI image, that is displayed on the output unit 160, in which a table including a list of ID numbers of the cells to be the manipulation target 35, the xy coordinates of the manipulation targets 35 on the sample actuator 41, the size of the manipulation targets 35, and the movement destinations of the manipulation target 35 is displayed. In the table shown in Fig. 7C, a case in which the ID number, the xy coordinates, the size, and the movement destination are listed as the item, bu the items to be displayed is not limited to these. In this manner, by designating the manipulation target 35, the movement destination or the like via the input unit 180, the image processing unit 300 may output the talbe to the output unit 160.

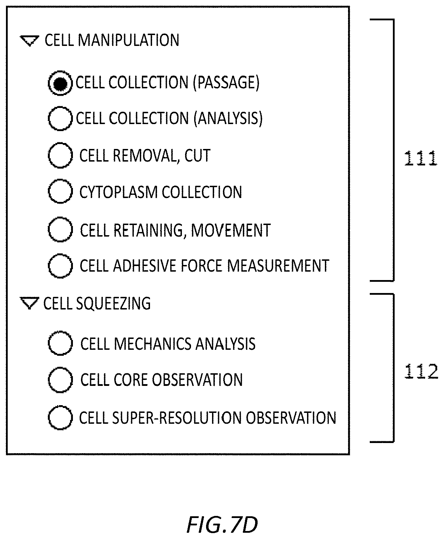

Fig. 7D is an example of a GUI screen displayed on the output unit 160, and in which the type of manipulation of the manipulation target 35 is to be selected. The input unit 180 receives an instruction, from the operator, on what kind of manipulation they wicsh to perform on the manipulation target 35, and inputs it to the information processing device 170. For example, as shown in the display area 111, the manipulation on the cell may be passage, or may be retain or moving of the cell, but it is not limited thereto. For example, as shown in the display area 112, the manipulation on the cell may be one in which the cell is squeezed and the core of the cell is observed, but it is not limited thereto. In Fig. 7D, an example in which the type of manipulation is selected by a radio button, but the method of selection is not limited to a radio button.

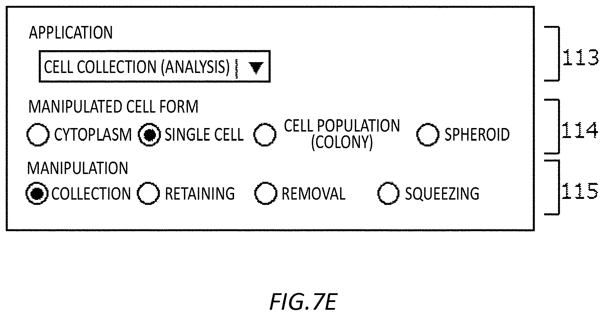

Fig. 7E is another example of a GUI screen displayed on the output unit 160, and in which the type of manipulation of the manipulation target 35 is to be selected. For example, as shown in the display area 113, the type of manipulation on the cell may be selected by a pull -down menu. For example, as shown in the display area 113, the display area 114, and the display area 115, a pull -down menu and a radio button may be used together for the selection of the type of manipulation.

The input unit 180 may send an instruction input from the operator to the information processing device 170, based on the screens shown in Fig. 7A to Fig. 7E. After the information processing device 170 has received the instruction, the imaging control unit 171 advances the processing to S160.

Note that, at S160, when the manipulation target 35 is an adherent cell that is strongly adhered to the solid phase, a step of relaxing the adhesion of the adherent cell in advance may additionally be performed. In this case, at the step of receiving a manipulation condition at S140, the information processing device 170 may receive an input on whether the processing of relaxing the adhesion is to be performed.

Relaxing the adhesion of the adherent cell can be performed by using a well known method. For example, relaxing the adhesion of the adherent cell may be performed by processing the adherent cell with adhesion relaxing solution, after removing liquid (for example, the medium) and washing it with a buffer. For example, the adhesion relaxing solution may be protease solution, may be solution containing no metal ion, or may be solution of chelating agent. As an example, the adhesion relaxing solution is trypsin-EDTA solution. Relaxing of adhesion of the adherent cell may be performed by the liquid control unit 260, or may be performed manually by the operator. After the adherent cell is processed with the adhesion relaxing solution and the adhesive force is weakened, the processing may be advanced to S160. Note that, when a manual step is performed by the operator, which is not limited to relaxing the adhesion of the adherent cell, the processing may be started again from the step of receiving the sample at S100. In addition, instead of the processing by adhesion relaxing solution, the adhesive force may be weakened by using a substrate for relaxing adhesion. For example, a substrate for relaxing adhesion may be one in which adhesion is relax by reacting to temperature or irradiation of light.

Then, at S160, the information processing device 170 receives an input related to processing for replacement or adding of liquid from the operator via the input unit 180. For example, when it is desired to adjust the attaching force between the organism that is the manipulation target 35 and an air bubble, the information processing device 170 may receive an input to perform processing for replacement or adding of liquid. When the information processing device 170 receives an instruction to perform processing for replacement or adding of liquid, the information processing device 170 may advance the processing to S600. When the information processing device 170 receives an instruction not to perform processing for replacement or adding of liquid, the information processing device 170 may advance the processing to S180.

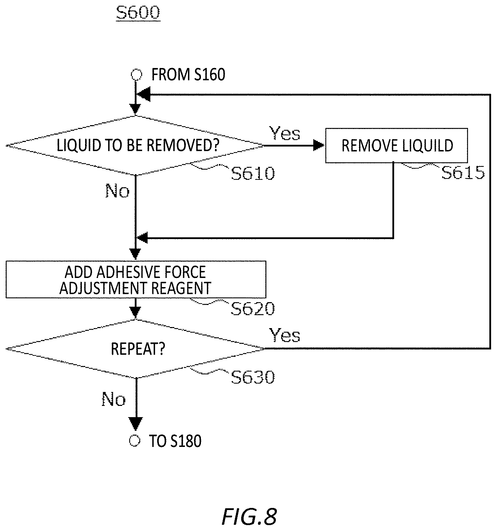

At S600, the liquid control unit 260 replaces the liquid in the container 25 in which the manipulation target 35 is accommodated, or adds another liquid to the liquid in the container 25. At S600, the step of performing processing for replacement or adding of liquid includes the steps of S610 to S630, as shown in Fig. 8.

First, at S610, the information processing device 170 receive an input related to whether the liquid is to be removed from the operator via the input unit 180. When the information processing device 170 receives an instruction to remove the liquid, the processing is advanced to S615. When the information processing device 170 receives an instruction not to remove the liquid, the processing is advanced to S620.

At S615, the liquid control unit 260 controls the liquid storage unit 54 to remove the liquid. For example, the liquid control unit 260 sends an instruction to the liquid storage unit 54 to collect, from the container 25, a preset amount of liquid accommodated in the container 25, and to dispose it to the liquid disposal unit of the liquid storage unit 54. At this time, the liquid storage unit 54 may collect dispose the entire amount of the liquid. Alternatively, the liquid storage unit 54 may collect and dispose a part (for example, half the amount) of the liquid. After the liquid storage unit 54 has collected the liquid, the liquid control unit 260 advances the processing to S620.

At S620, the liquid control unit 260 sends an instruction to the liquid storage unit 54 to add an attaching force adjustment reagent to the container 25. The attaching force adjustment reagent is a reagent for adjusting the attaching force between the organism and the air bubble. For example, the attaching force adjustment reagent may change the concentration of inorganic salt in the liquid and/or concentration of amphipathic substances. As an example, the attaching force adjustment reagent may be a buffer containing or not containing at least one of calcium ion or magnesium ion, may be a basal medium, may be a complete medium, or may be a chelating agent. At this time, the liquid storage unit 54 refills the container 25 with the attaching force adjustment reagent retained in the liquid retaining unit of the liquid storage unit 54.

Note that, altthough an example in which the container 25 is refilled with the attaching force adjustment reagent has be described in the above-described example, instead of adding the attaching force adjustment reagent to the container 25, the attaching force between the organism and the air bubble may be adjusted by causing the inorganic salt, the amphipathic substance or the like included in the liquid to be attached to a filter or the like for removal.

At S630, the information processing device 170 receives an instruction related to whether a series of the above-described manipulation is to be repeated from the operator via the input unit 180. When the information processing device 170 receives an instruction to repeat a series of the manipulation, the information processing device 170 advances the processing to S610, and the liquid control unit 260 instructs the liquid storage unit 54 to remove the liquid in the container 25. When the information processing device 170 receives an instruction not to repeat a series of the manipulation, the information processing device 170 advances the processing to S180. Note that, the step and sub-step of S600 may be manually performed by the operator, and in this case, the processing may be started again from the step of receiving the sample at S100.

At S180, the nozzle actuator 40 is equipped with the nozzle 49. For example, the information processing device 170 receives an instruction to equip the nozzle actuator 40 with the nozzle 49 from the operator via the input unit 180. In accordance with the instruction, the flow channel control unit 250 sends an instruction to the flow channel replacement unit 53 to take out the nozzle 49 from the flow channel retaining unit of the flow channel replacement unit 53 and equip the nozzle actuator 40 with the nozzle 49. At this time, a suitable nozzle 49 may be selected according to the size of the manipulation target 35, the type of manipulation, or the like. The select of the nozzle 49 may be designated by the operator via the input unit 180, or may be automatically designated by the flow channel control unit 250. After the nozzle actuator 40 is equipped with the nozzle 49, the flow channel control unit 250 advances the processing to S200. Note that, nozzle actuator 40 it is not necessary to equip the nozzle actuator 40 with the nozzle 49, such as when the nozzle actuator 40 is previously equipped with the nozzle 49, or when the nozzle 49 is integrally formed, the step of S180 may be omitted.

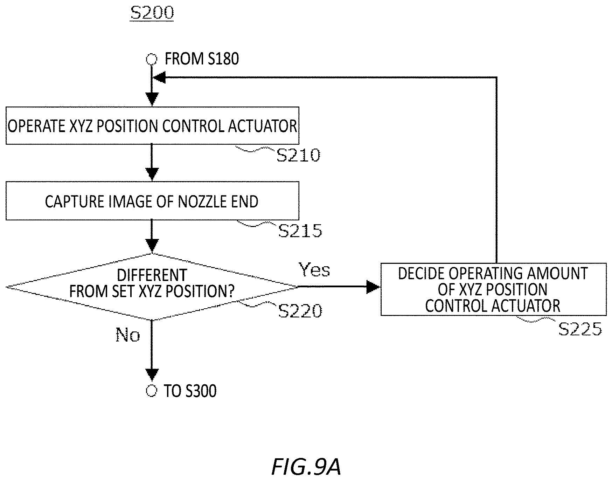

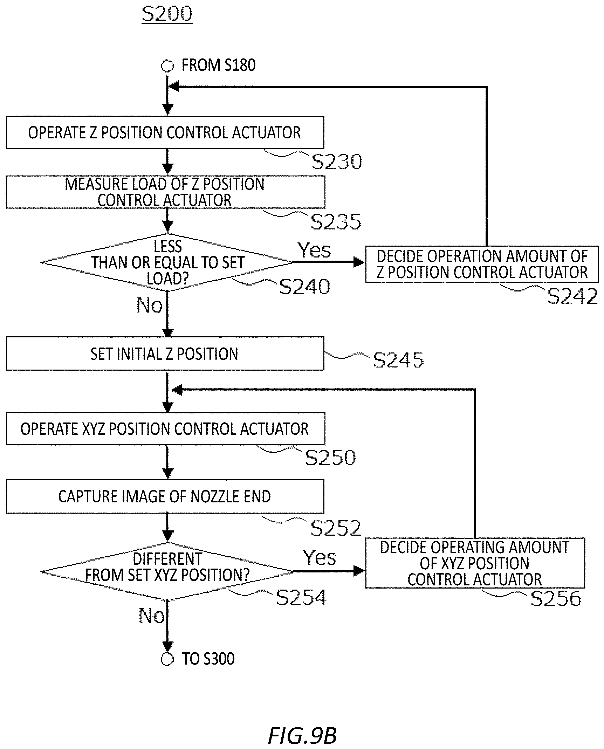

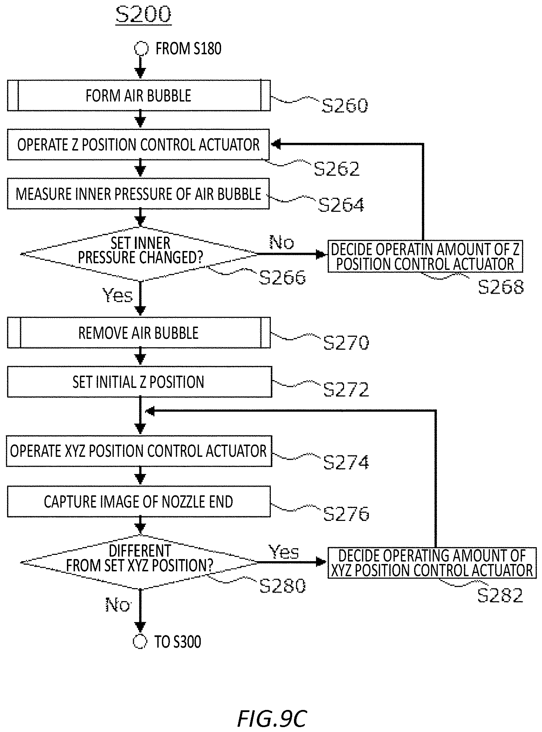

Then, at S200, the nozzle actuator 40 moves the relative position between the nozzle 49 and the manipulation target 35. For example, the volume control unit 200 sends an instruction to the nozzle actuator 40 to move the relative position between the nozzle 49 and the manipulation target 35. As an example, the volume control unit 200 may send an instruction to the nozzle actuator 40 to move the relative position between the nozzle 49 and the manipulation target 35 to become closer, such that an air bubble can be formed in proximity with the manipulation target 35. At S200, the step of moving the relative position includes the steps of S210 to S225 as shown in Fig. 9A, includes the steps of S230 to S256 as shown in Fig. 9B, or includes the steps of S260 to S282 as shown in Fig. 9C.

Fig. 9A is an example of a flow for moving the relative position between the nozzle 49 and the manipulation target 35 based on an image obtaied by capturing the position of an end 254 of the nozzle 49.

First, at S210, the volume control unit 200 sends an instruction to the nozzle actuator 40 to operate nozzle 49 to be a preset position. The nozzle actuator 40 may be an actuator for controlling the xyz position. Here, the z position may be the position in a vertical direction (also referred to as a direction along gravity, an upward/downward direction, or the z direction), the x position may be a position in any x direction (also referred to as a longitudinal direction) perpendicular to the z direction, and the y position may be a position in y direction (also referred to as a lateral direction) perpendicular to the x direction and the z direction.

The z position for the position of the nozzle 49 may be set by firstly focusing the camera 60 and/or the camera 70 to the bottom surface of the container 25, subsequently moving the focus of the camera 60 and/or the camera 70 upwardly by any distance, and then focusing the camera 60 and/or the camera 70 to the tip of the nozzle 49 by the nozzle actuator 40. For example, any distance may be the radius of the air bubble formed at the end 254 of the nozzle 49 or less. When the distance between the tip of the nozzle 49 and the bottom surface of the container 25 is equal to or less than the radius of the air bubble formed, since the air bubble is in contact with the bottom surface, the organism positioned on the bottom surface can be manipulated by using the interface of the air bubble. In this case, the xy position of the nozzle 49 can be set by using the nozzle actuator 40 or the sample actuator 41 based on an image obtained by capturing the manipulation target 35 using the camera 60 and/or the camera 70.

In addition, instead of the camera 60 and/or the camera 70, or together with the camera 60 and/or the camera 70, the position of the nozzle 49 may be set by using the flow channel capturing camera 42. For example, for the adjustment of the z position of the nozzle 49, the z position may be set by using the flow channel capturing camera 42 to laterally from the side of the nozzle 49 to capture the tip of the nozzle 49 and the bottom surface of the container 25. Further, the shape of the air bubble or the amount of fluid of the flow channel 51 or the like may be confirmed by using the flow channel capturing camera 42 to laterally capture the nozzle 49. After the nozzle actuator 40 has moved the nozzle 49 to a preset position, the volume control unit 200 advances the processing to S215.

Then, at S215, the flow channel capturing camera 42 captures an image of the end 254 of the nozzle 49. The flow channel capturing camera 42 sends the captured image to the image processing unit 300. The image processing unit 300 may record the image to the recording unit 190, and/or output the image to the volume control unit 200.

Then, at S220, the volume control unit 200 determines whether the position of the nozzle 49 is different from the preset position based on the captured image of the end 254 of the nozzle 49. For example, the volume control unit 200 calculates the difference of the positions from the captured image of the end 254 of the nozzle 49 and an image of the end 254 of the nozzle 49 at a set xyz position that is preset (that is, the initial position), and determines that the position of the nozzle 49 is different from the initial position is the difference is equal to or more than a threshold.

When it is determined that the position of the nozzle 49 is different from the initial position, the volume control unit 200 advances the processing to S225, and if not, advances the processing to S300.

At S225, the volume control unit 200 decides the operating amount of the nozzle actuator 40. For example, the volume control unit 200 sends an instruction to the nozzle actuator 40 to decide an operating amount of the nozzle actuator 40 and to operate by the decided operating amount, in order to operate the nozzle 49 to the preset xyz position (that is, the initial position). For example, the volume control unit 200 may decide the operating amount according to the size of the difference calculated at S220. The nozzle actuator 40 receives the instruction, and advances to S210. At the second and subsequent S210, the volume control unit 200 sends an instruction to the nozzle actuator 40 to perform an operation for an amount according to to the operating amount.

Fig. 9B is an example of a flow for moving the relative position between the nozzle 49 and the manipulation target 35 based on a load sensed by the nozzle actuator 40.

First, at S230, the volume control unit 200 sends an instruction to the nozzle actuator 40 to operate nozzle 49 to be a preset position. The nozzle actuator 40 may be an actuator for controlling the z position. In this case, the z position is controlled based on the value of the load sensed by the nozzle actuator 40, contact or proximity information. The nozzle 49 may be positioned above a region where no organism exists on the bottom surface of the container 25. After the nozzle actuator 40 has moved the nozzle 49 to a preset position, the volume control unit 200 advances the processing to S235.

Then, at S235, the sensor unit 48 measures the load applied by the nozzle actuator 40, the contact or proximity information, and sends the measurement value to the volume control unit 200. As an example of load detection, when the nozzle 49 reaches the bottom portion of the container 25, the load sensed by the nozzle actuator 40 increases rapidly. Therefore, the volume control unit 200 can determine whether the nozzle 49 has reached the bottom portion of the container 25 by measuring the value of the load sensed by the nozzle actuator 40. In addition, as another example of load detection, the sensor unit 48 may sense the load with the nozzle actuator 40 moving the nozzle 49 in a downward direction. Note that, instead of the sensor unit 48, the nozzle actuator 40 may send the value of the sensed load to the volume control unit 200.

Then, at S240, the volume control unit 200 determines whether the value of the measured load is equal to or less than a set load. When the value of the measured load is equal to or less than the set load, the volume control unit 200 advances the processing to S242, and if not, advances the processing to S245. As described above, the volume control unit 200 calculates the difference between the set load and the measured load, and when the value of the difference is equal to or more than the threshold, it is determined that the nozzle 49 has not reached the bottom portion of the container 25.

At S242, the volume control unit 200 decides the operating amount of the nozzle actuator 40. For example, the volume control unit 200 sends an instruction to the nozzle actuator 40 to decide an operating amount of the nozzle actuator 40 and to operate by the decided operating amount, in order to operate the nozzle 49 to the preset position. For example, the volume control unit 200 may decide the operating amount according to the size of the difference calculated at S240. The nozzle actuator 40 receives the instruction, and advances to S230. At the second and subsequent S230, the volume control unit 200 sends an instruction to the nozzle actuator 40 to perform an operation for an amount according to to the operating amount.

At S245, the volume control unit 200 sets the initial z position of the nozzle 49. For example, the volume control unit 200 may not move the z position of the nozzle 49 after the last S230, and set this as the initial z position. Alternatively, the volume control unit 200 may move the nozzle 49 in the z direction by any preset distance from the bottom surface of the container 25, and set this position as the initial z position. In this manner, the initial z position of the nozzle 49 will be positioned above the bottom surface by any distance. For example, any distance may be the radius of the air bubble formed at the end 254 of the nozzle 49 or less. After the volume control unit 200 has set the initial z position of the nozzle 49, the volume control unit 200 advances the processing to S250.

Then, at S250, the volume control unit 200 sends an instruction to the nozzle actuator 40 to operate the nozzle 49 to a set xyz position that is preset (that is, the initial position). The movement of the nozzle 49 may be movement on the xy plane. In this case, the z position is controlled based on the value of the load sensed by the nozzle actuator 40. In addition, the movement of the nozzle 49 may include movement in the z direction, as required, in addition to the movement on the xy plane. After the nozzle actuator 40 has moved the nozzle 49 to a set xyz position, the volume control unit 200 advances the processing to S252.

Then, at S252, the flow channel capturing camera 42 captures an image of the end 254 of the nozzle 49. The flow channel capturing camera 42 sends the captured image to the image processing unit 300. The image processing unit 300 may record the image to the recording unit 190, and/or output the image to the volume control unit 200.

Then, at S254, the volume control unit 200 determines whether the position of the nozzle 49 is different from the preset position based on the captured image of the end 254 of the nozzle 49. For example, the volume control unit 200 calculates the difference of the positions from the captured image of the end 254 of the nozzle 49 and an image of the end 254 of the nozzle 49 at a set xyz position that is preset (that is, the initial position), and the volume control unit 200 determines that the position of the nozzle 49 is different from the initial position is the difference is equal to or more than a threshold.

When it is determined that the position of the nozzle 49 is different from the initial position, the volume control unit 200 advances the processing to S256, and if not, advances the processing to S300.

At S256, the volume control unit 200 decides the operating amount of the nozzle actuator 40. For example, the volume control unit 200 sends an instruction to the nozzle actuator 40 to decide an operating amount of the nozzle actuator 40 and to operate by the decided operating amount, in order to operate the nozzle 49 to the set xyz position that is preset (that is, the initial position). For example, the volume control unit 200 may decide the operating amount according to the size of the difference calculated at S254. The nozzle actuator 40 receives the instruction, and the volume control unit 200 advances the processing to S250. At the second and subsequent S250, the volume control unit 200 sends an instruction to the nozzle actuator 40 to perform an operation for an amount according to to the operating amount.

Fig. 9C is an example of a flow for moving the relative position between the nozzle 49 and the manipulation target 35 based on inner pressure of the air bubble formed at the end 254 of the nozzle 49.

First, at S260, the volume control unit 200 controls the pressure generation unit 47 to form an air bubble at the end 254 of the nozzle 49. Prior to forming the air bubble, the volume control unit 200 may send an instruction to the nozzle actuator 40 to operate the end 254 of the nozzle 49 into liquid. The step and sub-step of forming the air bubble at S260 may be the same as the step and sub-step of S300 described below.

Then, at S262, the volume control unit 200 sends an instruction to the nozzle actuator 40 to operate nozzle 49 to be a preset position. The nozzle actuator 40 may be an actuator for controlling the z position. In this case, the z position is controlled based on the value of inner pressure measured by the sensor unit 48. The nozzle 49 may be positioned above a region where no organism exists on the bottom surface of the container 25. After the nozzle actuator 40 has moved the nozzle 49 to a preset position, the volume control unit 200 advances the processing to S264.

Note that, at S262, the tip of the nozzle 49 may sense the fluid level to control the z position, based on the value of inner pressure measured by the sensor unit 48. The volume control unit 200 maintains the inner pressure of the nozzle to be equal to or higher or equal to or lower than the atmospheric pressure, and when the tip of the nozzle 49 reaches the fluid level, the value of inner pressure measured by the sensor unit 48 is varied because of the external force caused by deformation of the gas-liquid interface due to the nozzle 49 touching the fluid level. Therefore, the volume control unit 200 can determine whether the tip of the nozzle 49 has reached the fluid level by measuring the value of inner pressure of the air bubble. In this manner, even when the position to which the nozzle 49 is to be moved is not preset, the volume control unit 200 nozzle actuator 40 can send an instruction to control the z position with the fluid level as a reference position, and move the position of the nozzle 49.

Then, at S264, the sensor unit 48 measures the inner pressure of the air bubble formed, and sends the measured value of inner pressure of the air bubble to the volume control unit 200. When the air bubble reaches the bottom portion of the container 25, the shape of the air bubble is deformed due to interaction with the bottom portion, and the inner pressure of the air bubble is rapidly varied. Therefore, the volume control unit 200 can determine whether the air bubble has reached the bottom portion of the container 25 by measuring the value of inner pressure of the air bubble. In addition, as another example of inner pressure measurement, while the nozzle actuator 40 moves the nozzle 49 in a downward direction, the sensor unit 48 may measure the inner pressure, the volume control unit 200 may control the pressure, or the pressure generation unit 47 may be actuated. By simultaneously performing operations in this manner, it is possible to accelerate detection or the like of the bottom portion, or set the course of variation of the pressure in the course of forming an air bubble as a detection indicator. Note that, instead of the sensor unit 48, the nozzle actuator 40 may measure the inner pressure of the air bubble and send the measured value of the inner pressure to the volume control unit 200.

Then, at S266, the volume control unit 200 determines whether the measured value of the inner pressure of the air bubble is within the preset range of inner pressure. When the measured value of inner pressure is outside the preset range of inner pressure, the volume control unit 200 advances the processing to S268, and if not, advances the processing to S270. As described above, the absolute value of the difference between the inner pressure set by the volume control unit 200 and the measured inner pressure of the air bubble is calculated, and if the difference is equal to or more than the threshold, the volume control unit 200 determines that the nozzle 49 has reached the bottom portion of the container 25.

At S268, the volume control unit 200 decides the operating amount of the nozzle actuator 40. For example, the volume control unit 200 sends an instruction to the nozzle actuator 40 to decide an operating amount of the nozzle actuator 40 and to operate by the decided operating amount, in order to operate the nozzle 49 to the preset position. For example, the volume control unit 200 may decide the operating amount according to the size of the difference calculated at S266. The nozzle actuator 40 receives the instruction, and advances to S262. At the second and subsequent S262, the volume control unit 200 sends an instruction to the nozzle actuator 40 to perform an operation for an amount according to to the operating amount.

At S270, the volume control unit 200 controls the pressure generation unit 47 to remove the air bubble from the end 254 of the nozzle 49. The step and sub-step of removing the air bubble at S270 may be the same as the step and sub-step of S500 described below.

Then, at S272, the volume control unit 200 sets the initial z position of the nozzle 49. The step of S272 may be the same as the step of S245. After the volume control unit 200 has set the initial z position of the nozzle 49, the volume control unit 200 advances the processing to S274.

Then, at S274, the volume control unit 200 sends an instruction to the nozzle actuator 40 to operate the nozzle 49 to a set xyz position that is preset (that is, the initial position). The movement of the nozzle 49 may be movement on the xy plane. In this case, the z position is controlled based on the value of the inner pressure measured by the nozzle actuator 40. In addition, the movement of the nozzle 49 may include movement in the z direction, as required, in addition to the movement on the xy plane. After the nozzle actuator 40 has moved the nozzle 49 to a set xyz position that is preset, the volume control unit 200 advances the processing to S276.

Then, at S276, the flow channel capturing camera 42 captures an image of the end 254 of the nozzle 49. The flow channel capturing camera 42 sends the captured image to the image processing unit 300. The image processing unit 300 may record the image to the recording unit 190, and/or output the image to the volume control unit 200.

Then, at S280, the volume control unit 200 determines whether the position of the nozzle 49 is different from the preset position based on the captured image of the end 254 of the nozzle 49. For example, the volume control unit 200 calculates the difference of the positions from the captured image of the end 254 of the nozzle 49 and an image of the nozzle end 254 at a set xyz position that is preset (that is, the initial position), and the volume control unit 200 may determine that the position of the nozzle 49 is different from the initial position is the difference is equal to or more than a threshold.

When it is determined that the position of the nozzle 49 is different from the initial position, the volume control unit 200 advances the processing to S282, and if not, advances the processing to S300.

At S282, the volume control unit 200 decides the operating amount of the nozzle actuator 40. For example, the volume control unit 200 sends an instruction to the nozzle actuator 40 to decide an operating amount of the nozzle actuator 40 and to operate by the decided operating amount, in order to operate the nozzle 49 to the set xyz position that is preset (that is, the initial position). For example, the volume control unit 200 may decide the operating amount according to the size of the difference calculated at S280. The nozzle actuator 40 receives the instruction, and the volume control unit 200 advances the processing to S274. At the second and subsequent S274, the volume control unit 200 sends an instruction to the nozzle actuator 40 to perform an operation for an amount according to to the operating amount.

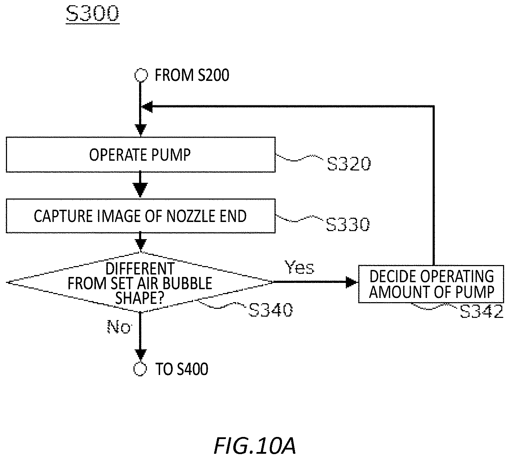

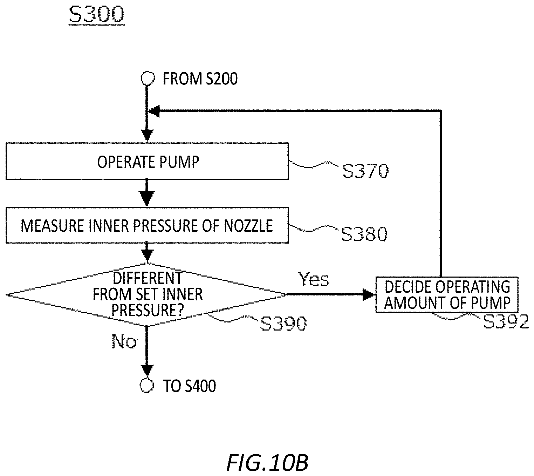

At S300, the volume control unit 200 controls the pressure generation unit 47 such that the gas-liquid interface 255 is enlarged. For example, enlarging the gas-liquid interface 255 may include to form an air bubble. Prior to forming the air bubble, the volume control unit 200 may send an instruction to the nozzle actuator 40 to operate the end 254 of the nozzle 49 into liquid. At S300, the step of enlarging the gas-liquid interface 255 includes the steps of S320 to S342 as shown in Fig. 10A, or includes the steps of S370 to S392 as shown in Fig. 10B.

Fig. 10A is an example of a flow for enlarging the gas-liquid interface 255 based on an image obtained by capturing the position of the end 254 of the nozzle 49.

At S320, the volume control unit 200 sends an instruction to the pressure generation unit 47 connected to the flow channel 51 to enlarge the gas-liquid interface 255 (form an air bubble) at the tip of the flow channel 51. For example, the volume control unit 200 sends an instruction to the pressure generation unit 47 to press the plunger of the syringe pump by a preset distance, or to the actuator of the pressure generation unit 47 to press the plunger of the syringe pump until a preset pressure is reached. As a result, the gas propelled from the syringe pump is charged to the flow channel 51, and the gas-liquid interface 255 at the tip of the flow channel 51 is enlarged (an air bubble is formed). After the gas-liquid interface 255 has been enlarged (the air bubble has been formed), the volume control unit 200 advances the processing to S330.

Then, at S330, the flow channel capturing camera 42 captures an image of the air bubble formed at the end 254 of the nozzle 49. The flow channel capturing camera 42 sends the captured image to the image processing unit 300. The image processing unit 300 may record the image to the recording unit 190, and/or output the image to the volume control unit 200.

Then, at S340, the volume control unit 200 determines whether the shape of the formed air bubble is different from a preset shape of the air bubble based on the captured image of the air bubble. The volume control unit 200 predicts the shape of the air bubble to be formed at the end 254 of the nozzle 49 from information such as set inner pressure in the nozzle 49, the inner diameter of the end 254 of the nozzle 49, wettability of the nozzle 49 (contact angle of liquid), the type of liquid, and the type of gas, or the like. For example, the volume control unit 200 may determine whether the shape of the formed air bubble is different from the set shape of the air bubble by comparing the captured image of the air bubble and the shape of the air bubble predicted from the above-described information.

When the shape of the formed air bubble is different from the set shape of the air bubble, the volume control unit 200 advances the processing to S342, and if not, advances the processing to S400.

At S342, the volume control unit 200 decides the operating amount of a plunger of a syringe pump of the pressure generation unit 47. For example, the volume control unit 200 decides the operating amount (for example, the distance by which the plunger of the syringe pump is pressed or pulled) of the plunger of the syringe pump of the pressure generation unit 47, in order to form the air bubble to be formed at the tip of the nozzle 49 in a preset shape. The volume control unit 200 sends an instruction to the pressure generation unit 47 to operate by the decided operating amount. For example, the volume control unit 200 may decide the operating amount according to the size of the difference calculated at S340.

The operating amount may be an operating amount of the actuator of the pressure generation unit 47, or may be additional pressure to be loaded on the syringe pump. The pressure generation unit 47 receives the instruction, and the volume control unit 200 advances the processing to S320. At second and subsequent S320, the pressure generation unit 47 performs operation by an amount according to the operating amount.

Fig. 10B is an example of a flow for enlarging the gas-liquid interface 255 based on the inner pressure in the nozzle 49.

The step of S370 may be the same as the step of S320. Having finished S370, the volume control unit 200 advances the processing to S380.

Then, at S380, the sensor unit 48 measures the inner pressure in the nozzle 49 and sends the measured value of the inner pressure in the nozzle 49 to the volume control unit 200. Note that, instead of the sensor unit 48, the nozzle actuator 40 may measure the inner pressure in the nozzle 49 and send the measured value of inner pressure to the volume control unit 200.

Then, at S390, the volume control unit 200 determines whether the measured value of inner pressure in the nozzle 49 is within the preset range of inner pressure. When the measured value of inner pressure is outside the set range of inner pressure, the volume control unit 200 advances the processing to S392, and if not, advances the processing to S400. For example, the volume control unit 200 may calculate the difference between the preset inner pressure and the measured inner pressure in the nozzle 49, and if the difference is equal to or more than a threshold, determine that the set inner pressure is not reached.

At S392, the volume control unit 200 decides the operating amount (for example, the distance by which the plunger of the syringe pump is pushed or pulled) of a plunger of a syringe pump of the pressure generation unit 47, in order to achieve the set inner pressure in the nozzle 49. The volume control unit 200 sends an instruction to the pressure generation unit 47 to operate by the decided operating amount. For example, the volume control unit 200 may decide the operating amount according to the size of the difference calculated at S390.

The operating amount may be an operating amount of the actuator of the pressure generation unit 47, or may be additional pressure to be loaded on the syringe pump. The pressure generation unit 47 receives the instruction, and the volume control unit 200 advances the processing to S370.

At second and subsequent S370, the pressure generation unit 47 performs operation by an amount according to the operating amount.

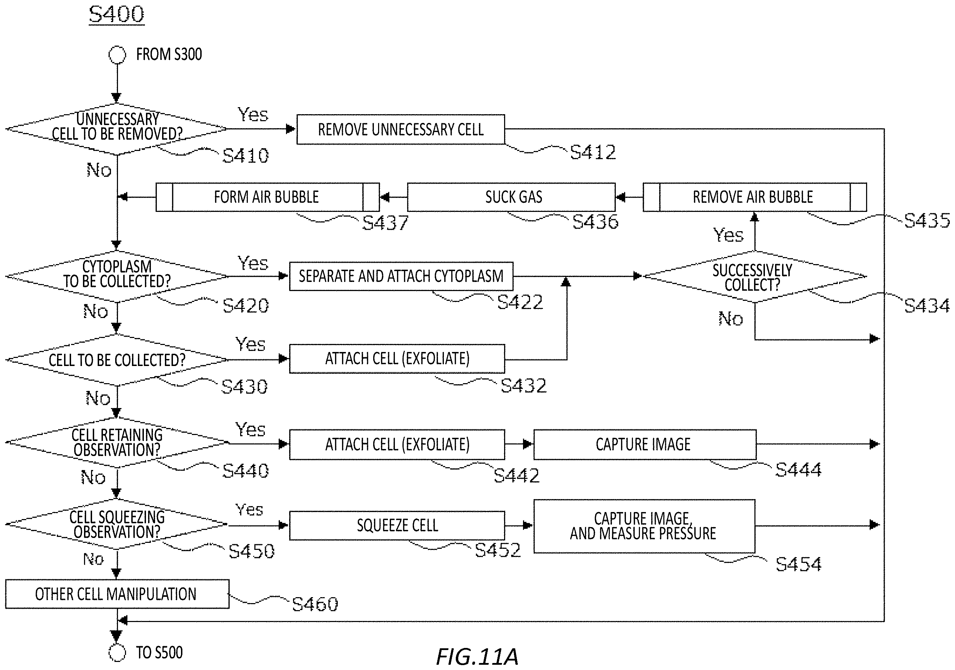

At S400, the manipulation unit 101 performs manipulation on the manipulation target 35. For example, the volume control unit 200 sends an instruction to the manipulation unit 101 to perform manipulation on the manipulation target 35, based on an instruction received via the input unit 180. At S400, the step of performing the manipulation includes the steps of S410 to S460 as shown in Fig. 11A. For example, the manipulation may be removal of unnecessary cells, collecting or moving of a cytoplasmic membrane and/or a cytoplasmic membrane, collecting cells, retaining cells, or squeezing of cells.

Fig. 11A is an example of a flow for performing manipulation on the manipulation target 35. A case in which the manipulation target 35 is a cell is described in Fig. 11A. Note that, the manipulation target 35 is not limited to a cell, and may be another organism.

First, at S410, when an instruction is to remove unnecessary cells is received at S140, the information processing device 170 advances the processing to S412. At S410, when an instruction not to remove unnecessary cells is received at S140, the information processing device 170 advances the processing to S420.

At S412, the volume control unit 200 causes the cell to be attached to the formed air bubble for removal.

For example, the volume control unit 200 sends an instruction to the nozzle actuator 40 to move the position of the nozzle 49 in the x, y and z directions to a position where the target cell exists. After the nozzle actuator 40 has moved the nozzle 49 to a target position, the volume control unit 200 may move the nozzle 49 and/or the stage to bring the gas-liquid interface 255 of the air bubble and the cell into contact with each other.

As an example, the nozzle actuator 40 or the sample actuator 41 identifies the position where the target cell exists, from an image obtained by performing image-capturing of the target cell with the camera 60 or the camera 70, and moves the center of the nozzle 49 to be aligned with the target position. After the cell has been brought into contact with the gas-liquid interface 255 of said air bubble, the nozzle actuator 40 may collect the target cells in the flow channel 51, as shown in 290a to 290c in Fig. 4, and the liquid storage unit 54 may dispose these cells in the liquid disposal unit of the liquid storage unit 54.

In addition, as another example, the volume control unit 200 may form a gas-liquid interface 255 of a predetermined size at the step and sub-step of S300, and causes the cell to be attached to be gas-liquid interface 255 for exfoliation by controlling the gas-liquid interface 255 to be enlarged. After the liquid control unit 260 has removed unnecessary cells, the volume control unit 200 advances the processing to S500.

At S420, when an instruction to collect cytoplasm and/or cytoplasmic membrane is received at S140, the volume control unit 200 advances the processing to S422, and if not, advances the process to S430.

At S422, the volume control unit 200 uses the formed air bubble to separate the cytoplasm and/or the cytoplasmic membrane, and cause them to be attached to said air bubble for collection. For example, the volume control unit 200 controls the pressure generation unit 47 to form an air bubble at the tip of the flow channel 51, and causes the target cell to be attached to said air bubble. Then, the volume control unit 200 squeezes the cell with the air bubble to detach only the cytoplasm and/or the cytoplasmic membrane portion, and causes it to be attached to the air bubble. For example, the volume control unit 200 squeezes the cell by controlling the pressure generation unit 47 to raise the inner pressure of the air bubble, enlarging the air bubble, or by controlling the nozzle actuator 40 to move the nozzle 49 toward the cell and pressing the air bubble against the cell. Subsequently, the volume control unit 200 moves the part of the cell that is bulged out to the outside of the cell by the squeezing to be detached from the cell at the gas-liquid interface, to detach the cytoplasm and/or the cytoplasmic membrane portion from the cell. In this manner, the volume control unit 200 controls the nozzle actuator 40 or the pressure generation unit 47 to cut out necessary cytoplasm and/or cytoplasmic membrane among the manipulation target 35.

For example, the volume control unit 200 may send an instruction to the nozzle actuator 40 to move the nozzle 49 in the x, y and z directions from an initial position to a position where the target cell exists. After the nozzle actuator 40 has moved the nozzle 49 to a target position, the volume control unit 200 may move the nozzle 49 and/or the stage with the nozzle actuator 40 or the sample actuator 41, to bring the gas-liquid interface 255 of said air bubble and the cell into contact with each other. As an example, the volume control unit 200 identifies the position where the target cell exists from an image obtained by performing image-capturing of the target cell by the camera 60 or the camera 70, and controls the nozzle actuator 40 to align the center of the nozzle 49 with the target position to move the nozzle 49. Here, identifying the position where the target cell exists may be performed by the operator. In this case, the volume control unit 200 may receive, from the input unit 180, an input by the operator related to the position where the target cell exists, to identify the position.

It is known that in the cytoplasmic membrane, there exists a portion that exhibits relatively soft physical properties and a portion that exhibits relatively hard physical properties due to the difference in lipid composition that composes the membrane component. The volume control unit 200 controls the nozzle actuator 40 or the pressure generation unit 47 to squeeze the cell with the air bubble. Here, the squeezing of the cell by the air bubble may be perfomed by the volume control unit 200 forming a gas-liquid interface 255 of a predetermined size at the step and sub-step of S300, and causing the cell to be attached to be gas-liquid interface 255 for squeezing by controlling the gas-liquid interface 255 to be enlarged. Then, by using the fact that the portion of the cytoplasmic membrane that exhibit relatively soft physical properties expands outside, the gas-liquid interface 255 may be used to have the expanded portion attached thereto, cytoplasmic membrane may be moved in a direction away from the cell to be detached, and the cytoplasmic membrane may be collected in the flow channel 51 while remaining to be attached to the gas-liquid interface 255. In addition, when cutting the cytoplasmic membrane out in this manner, since the cytoplasmic membrane is expanded by being pressed from the inside by the cytoplasm, the detached cytoplasmic membrane includes cytoplasmic component on the inside, and it is also possible to collect the cytoplasmic component in the flow channel 51. After the nozzle actuator 40 has detached necessary cytoplasm and/or cytoplasmic membrane portions, the volume control unit 200 advances the processing to S434.

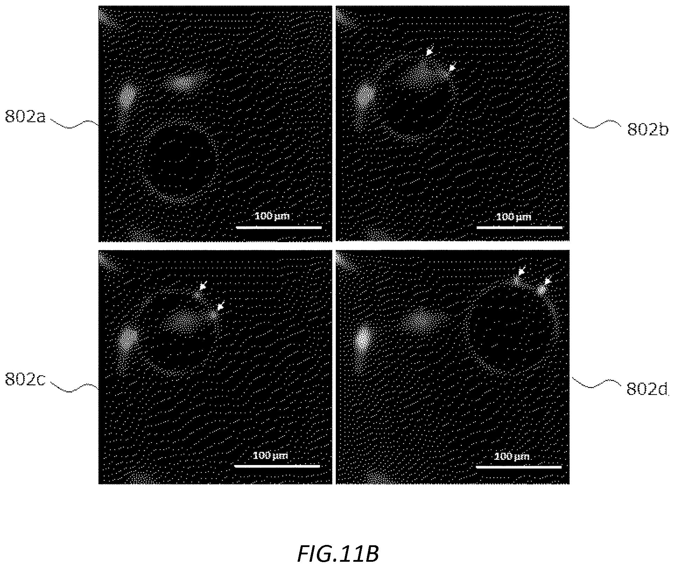

Fig. 11B illustrates how cytoplasm and cytoplasmic membrane is collected from a cell in the present embodiment. The volume control unit 200 controls the pressure generation unit 47 and the nozzle actuator 40, and thereby the relative position between the cell and the nozzle 49 are brought closer in order to bring the HeLa cell (human cervical cancer cell) that is cultured in a solid phase of the container 25 into contact with the gas-liquid interface 255 of the air bubble (802a). Then, the volume control unit 200 controls the gas-liquid interface 255 such that it is pressed against the cell, and thereby the cell is squeezed (802b). At this time, due to the squeezing, the soft portion of the cytoplasmic membrane was observed to expand to the outside (the arrow in 802b). Then, this expanded portion and the gas-liquid interface 255 was moved in a direction away from the cell, so that the cytoplasm and the cytoplasmic membrane in the expanded portion is detached (the arrow in 802c). Finally, the detached cytoplasm and cytoplasmic membrane were caused to be attached to the gas-liquid interface 255 to be collected (802d). Note that, when performing the operation of Fig. 11B, the operation may be performed by enlarging the gas-liquid interface 255, the operation may be performed by moving the nozzle 49, or the operation may be performed by moving the stage.

Then, at S434, the volume control unit 200 determines whether the instruction received at S140 includes successively collecting the cytoplasm and/or cytoplasmic membrane, which is the manipulation target 35. The volume control unit 200 advances the processing to S435 when the determination is positive, and advances the processing to S500 when the determination is negative.

At S435, the volume control unit 200 controls the pressure generation unit 47 to remove the air bubble formed at the end 254 of the nozzle 49. Here, when removing the air bubble, collection of the manipulation target 35 may be simultaneously performed. For example, the manipulation target 35 may be collected in the liquid existing in the flow channel 51. The step and sub-step of removing the air bubble at the volume control unit S435 may be the same as the step and sub-step of S500, and details thereof will be described below.

Then, at S436, the volume control unit 200 controls the pressure generation unit 47 nozzle actuator 40 to perform intake of gas in order to form a new air bubble at the end 254 of the nozzle 49. The volume control unit 200 sends an instruction to the nozzle actuator 40 to take the nozzle 49 out from the liquid. After the nozzle actuator 40 has taken the nozzle 49 out from the liquid, the pressure generation unit 47 may pull the plunger of the syringe pump to intake required amount of gas.

Then, at S437, the volume control unit 200 controls the pressure generation unit 47 to form a new air bubble at the end 254 of the nozzle 49. The content of S300 that is already described may be applied to the step and sub-step of S437. After the pressure generation unit 47 has formed an air bubble at the end 254 of the nozzle 49, the volume control unit 200 advances the processing to the step of S420.

At S430, the volume control unit 200 determines whether an instruction to collect the cell is received at S140. The volume control unit 200 advances the processing to S432 when the determination is positive, and the volume control unit 200 advances the processing to S440 when the determination is negative.

At S432, the volume control unit 200 controls the pressure generation unit 47 to form an air bubble, and causes the cell to be attached to said air bubble. The volume control unit 200 may exfoliate the cell attached to the air bubble from the solid phase, as required.

For example, the volume control unit 200 sends an instruction to the nozzle actuator 40 to move the nozzle 49 in the x, y and z directions from an initial position to a position where the target cell exists. After the nozzle actuator 40 has moved the nozzle 49 to a target position, the volume control unit 200 controls the pressure generation unit 47 to charge gas to the flow channel 51 and to form an air bubble at the tip of the flow channel 51. Then, the volume control unit 200 may control the nozzle actuator 40 or the sample actuator 41 to move the nozzle 49 or the stage and to bring the gas-liquid interface 255 of the air bubble into contact with the cell.

As an example, the nozzle actuator 40 or the sample actuator 41 identifies the position where the target cell exists, from an image obtained by performing image-capturing of the target cell with the camera 60 or the camera 70, and moves the center of the nozzle 49 to be aligned with the target position. After the nozzle actuator 40 or the sample actuator 41 has moved the nozzle 49 to a target position, the volume control unit 200 controls the pressure generation unit 47 to charge gas to the flow channel 51 and to form an air bubble at the tip of the flow channel 51. Then, the volume control unit 200 may move the nozzle 49 and/or the stage and and bring the gas-liquid interface 255 of the air bubble into contact with the cell with the nozzle actuator 40 or the sample actuator 41. For example, after bringing the cell into contact with the gas-liquid interface 255 of said air bubble, the nozzle actuator 40 may exfoliate the cell as required by moving the gas-liquid interface 255 or the like.

Here, collection of the cell may be performed by causing the gas-liquid interface 255 that is an interface between liquid and the air bubble to be attached to the cell, and capturing the gas-liquid interface 255 in the flow channel 51. For example, collection of the cell may be performed by causing the gas-liquid interface 255 that is an interface between liquid and the air bubble to be attached to the cell, and intaking gas in the flow channel 51 to capture the gas-liquid interface 255 in the flow channel 51. For example, after bringing the cell into contact with the gas-liquid interface 255, the target cell may be collected by capturing the gas-liquid interface 255 in the flow channel 51 after a preset time has elapsed. As an example, the preset time may be ten seconds, may be 20 seconds, may be 30 seconds, or may be 30 seconds or more.

After the volume control unit 200 has controlled the pressure generation unit 47 to form an air bubble and caused the cell to be attached to said air bubble, the volume control unit 200 advances the processing to S434. The content that is already described may be applied to steps S434 and after. Note that, in this case, at the step of S434, those to be successively collected is not limited to only the cytoplasm or only the cell, and it may be both the cytoplasm and the cell.

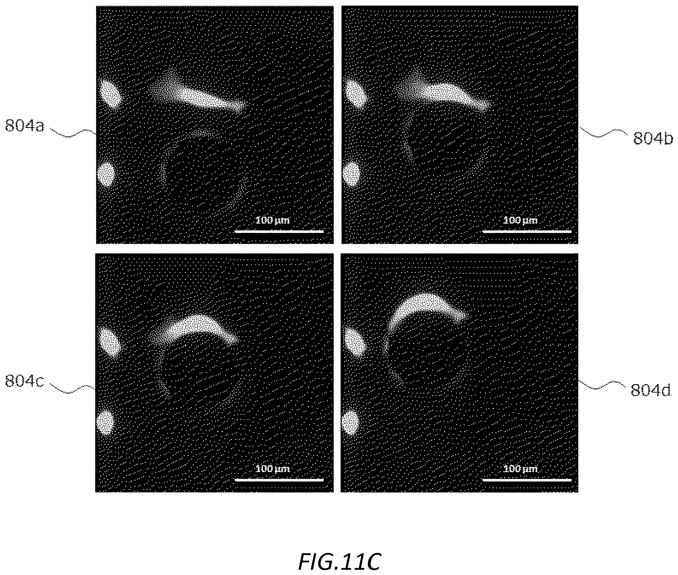

Fig. 11C illustrates how an established cell is exfoliated by allowing it to be attached to the air bubble in the present embodiment. The volume control unit 200 controls the pressure generation unit 47 and the nozzle actuator 40 (or the sample actuator 41 instead of the nozzle actuator 40), and thereby the relative position between the cell and the nozzle 49 is brought closer in order to bring the HeLa cell cultured in a solid phase of the container 25 into contact with the gas-liquid interface 255 of the air bubble (804a). Then, the cell is brought into contact with the gas-liquid interface 255 of the air bubble (804b). Then, the nozzle actuator 40 moved the gas-liquid interface 255 (804c), and the cell was allowed to be attached to the gas-liquid interface 255 to be exfoliated (804d). Note that, when performing the operation of Fig. 11C, the operation may be performed by enlarging the gas-liquid interface 255, the operation may be performed by moving the nozzle 49, or the operation may be performed by moving the stage.

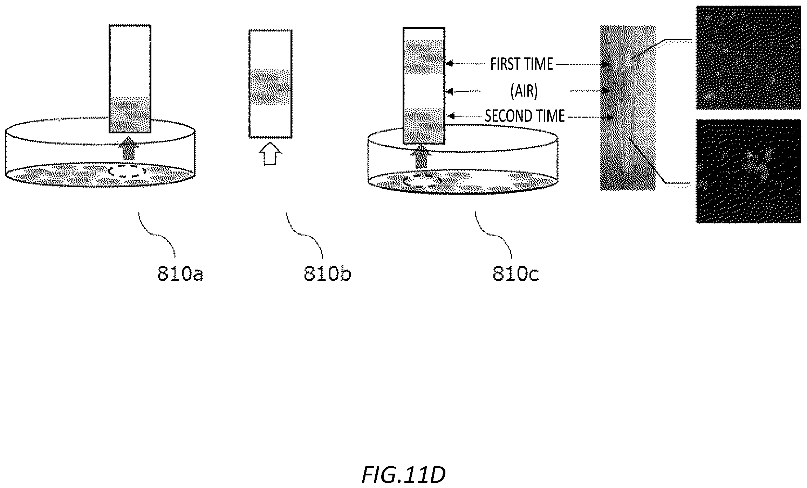

Fig. 11D illustrates a schematic view of a case where S430 -> S432 -> S434 -> S435 -> S436 -> S437 are repeated. When the cytoplasm and/or cytoplasmic membrane is successively collected, and when the cell is successively collected, it may be performed as illustrated in the schematic view of Fig. 11D. At 810a, after the nozzle actuator 40 has put the nozzle 49 in the liquid, the volume control unit 200 controls the pressure generation unit 47 to form an air bubble at the end 254 of the nozzle 49 by causing the syringe pump (not illustrated) to charge air. Then, the cell that is adhered to the bottom surface of the container 25 is caused to be attached to the gas-liquid interface 255 of said air bubble to be exfoliated, and is collected in the flow channel 51 by causing the syringe pump to intake gas (for example, air). Then, at 810b, the nozzle actuator 40 pulls the nozzle 49 up from the liquid and the syringe pump sucks gas into the flow channel 51. Then, at 810c, after the nozzle actuator 40 has put the nozzle 49 in the liquid, the syringe pump charges air to the end 254 of the nozzle 49, thereby forming a new air bubble, and collects the cell that is adhered to the bottom surface of the container 25 by causing it to be attached to the gas-liquid interface 255 of said air bubble. In this manner, the volume control unit 200 controls the pressure generation unit 47 and the nozzle actuator 40, thereby allowing two cells (population) to be successively collected in the flow channel 51 via the gas without being mixed. A photograph is also shown where two cells (population) are actually collected by this method with air interposed therebetween. Note that, although a case where the nozzle 49 is moved is described as an example in Fig. 11D, the operation of Fig. 11D may be performed by moving the stage instead of moving the nozzle 49.

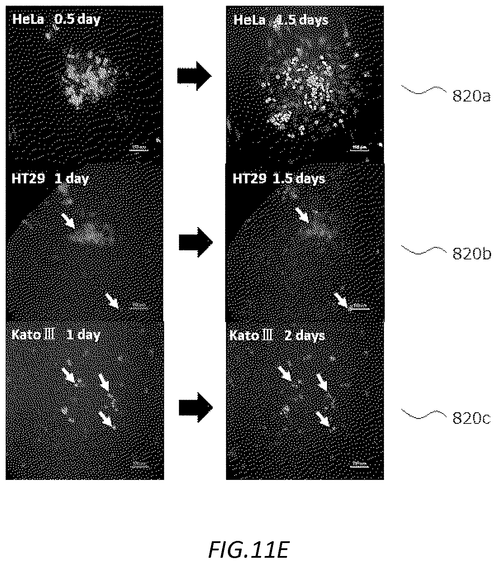

Fig. 11E illustrates how the established cell is collected, and how passage is performed in which the established cell collected is cultured, according to the present embodiment. The volume control unit 200 controls the pressure generation unit 47 and the nozzle actuator 40, thereby allowing a HeLa cell (human cervical cancer cell, 820a, a HT29 cell (human colon cancer cell, 820b), and a Kato III cell (human gastric signet ring cell carcinoma cell, 820c), which are established cells, to be attached, exfoliated, and collected by using the gas-liquid interface 255 of the air bubble ffrom the solid phase of the container 25, and those cells were released to a medium in another container to be cultured for 1.5 days (820a and 820b) and two days (820c). It was confirmed for all of the cells that the cells grew after passage. That is, the cells can be grown without causing damage to cell viability even when the established cells are exfoliated by using the gas-liquid interface 255 of the air bubble according to the present embodiment. Note that, when performing the operation of Fig. 11E, the operation may be performed by moving the nozzle 49, or the operation may be performed by moving the stage.

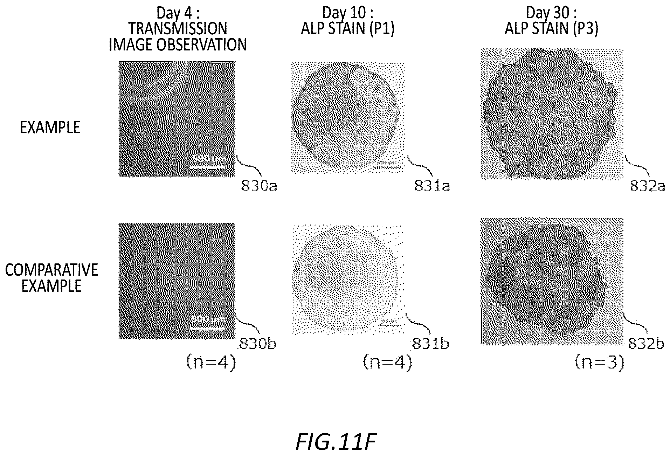

Fig. 11F illustrates how an iPS cell is collected, and how the collected iPS cell was subjected to passage, according to the present embodiment. The volume control unit 200 controls the pressure generation unit 47 and the nozzle actuator 40, thereby allowing a colony of the cultured iPS cells to be attached, exfoliated, and collected by using the gas-liquid interface 255 of the air bubble from the solid phase of the container 25, and after it was released into a liquid medium in another container and cultured for four days, a transmission image was observed. In addition, as a comparative example, an iPS cell that has been subjected to passage by a normal cell passage method (mechanical passage) was used. As a result, no morphological difference was observed between the iPS cell of the present embodiment (830a) and the iPS cell of the comparative example (830b). In addition, the iPS cells of the present embodiment and the comparative example were subjected to alkaline phosphatase stain, after being cultured for ten days. Further, after the iPS cells of the present embodiment and the comparative example were subjected to passage for three times and cultured for 30 days, the iPS cells of the present embodiment and the comparative example were subjected to alkaline phosphatase stain. As a result, no difference in dyeability was observed between the iPS cell of the present embodiment (831a is those cultured for ten days, 832a is those cultured for 30 days) and the iPS cells of the comparative example (831b is those cultured for ten days, 832b is those cultured for 30 days). It is known that alkaline phosphatase is highly expressed in undifferentiated iPS cells that have its replication competence maintained. That is, it was possible to grow the iPS cells remained in their undifferentiated state without affecting the maintainability of the undifferentiation compentence even when the iPS cells are exfoliated to be collected by using the gas-liquid interface 255 according to the present embodiment. Note that, when performing the operation of Fig. 11F, the operation may be performed by moving the nozzle 49, or the operation may be performed by moving the stage.

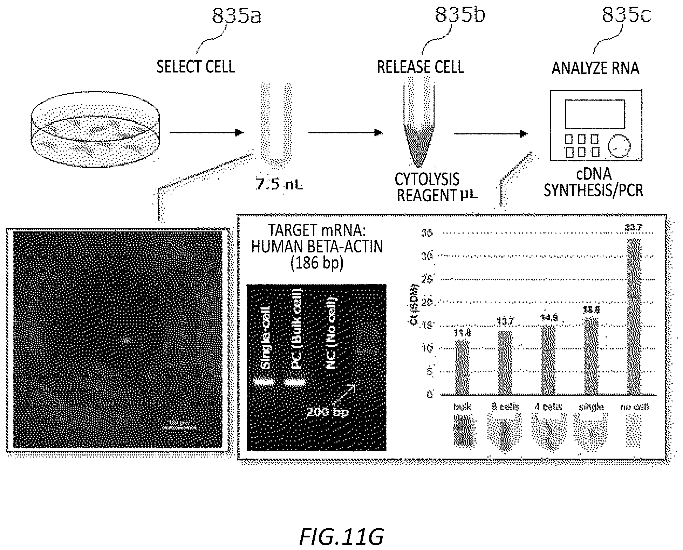

Fig. 11G illustrates how the established cell was collected, and how the collected cell was analyzed according to the present embodiment. A HeLa cell that is an established cell was exfoliated and collected by using the gas-liquid interface 255 of the air bubble from the solid phase of the container 25. The volume control unit 200 controls the pressure generation unit 47 and the nozzle actuator 40, thereby allowing one cell, four cells, and eight cells to be selected to be exfoliated from the solid phase of the HeLa cell by using the gas-liquid interface 255 of the air bubble, and these cells were each collected together with 7.5 nL of liquid medium (835a). Then, the collected HeLa cell were released into 12.5 μl of cytolysis reagent, and lysed the cell (835b). In the cell lysate in which the HeLa cell is lysed, cDNA was synthesized from mRNA of beta-actin to perform PCR reaction (835c). As a result, it was possible to detect an amount of cDNA that is roughly proportional to the collected cell number. That is, it was possible to collect one cell or any number of cells by using the gas-liquid interface 255 according to the present embodiment to perform cell biological analysis. Note that, when performing the operation of Fig. 11G, the operation may be performed by moving the nozzle 49, or the operation may be performed by moving the stage.

Then, at S440, the volume control unit 200 determines whether an instruction to retain and perform image-capturing of the cell is received at S140. The volume control unit 200 advances the processing to S442 when the determination is positive, and advances the processing to S450 when the determination is negative.

At S442, the volume control unit 200 causes the cell to be attached to the formed air bubble, and performs control to retain the attached cell.

For example, the volume control unit 200 sends an instruction to the nozzle actuator 40 to move the nozzle 49 in the x, y and z directions from an initial position to a position where the target cell exists. After the nozzle actuator 40 has moved the nozzle 49 to a target position, the volume control unit 200 controls the pressure generation unit 47 to charge gas to the flow channel 51 and to form an air bubble at the tip of the flow channel 51. The volume control unit 200 may move the nozzle 49 and/or the stage and and bring the gas-liquid interface 255 of said air bubble into contact with the cell with the nozzle actuator 40 or the sample actuator 41. As an example, the nozzle actuator 40 or the sample actuator 41 identifies the position where the target cell exists, from an image obtained by performing image-capturing of the target cell with the camera 60 or the camera 70, and moves the center of the nozzle 49 to be aligned with the target position. After the cell is brought into contact with the gas-liquid interface 255 of said air bubble, the volume control unit 200 advances the processing to S444.

Here, identifying the position where the target cell exists may be performed by the operator. In this case, the volume control unit 200 may receive, from the input unit 180, an input by the operator related to the position where the target cell exists, to identify the position. In addition, although a case where the edge surface of the air bubble is brought into contact with the cell is described in the above-described example, the contact between the air bubble and the cell may be performed by bringing the side surface of the air bubble into contact with the cell. In this case, the nozzle actuator 40 or the sample actuator 41 may move the center of the nozzle 49 to be aligned near the target cell. In addition, for example, after bringing the cell into contact with the gas-liquid interface 255 of said air bubble, the nozzle actuator 40 may exfoliate the cell as required by moving the gas-liquid interface 255 or the like.

Then, at S444, the imaging control unit 171 sends an instruction to the camera 60 or the camera 70 to perform image-capturing of the cell retained at the air bubble. The camera 60 or the camera 70 captures an image, and sends the image to the image processing unit 300. The image processing unit 300 may record the image to the recording unit 190, and/or output the image to the volume control unit 200.

After the camera 60 or the camera 70 has performed image-capturing of the retained cell, the volume control unit 200 advances the processing to S500.

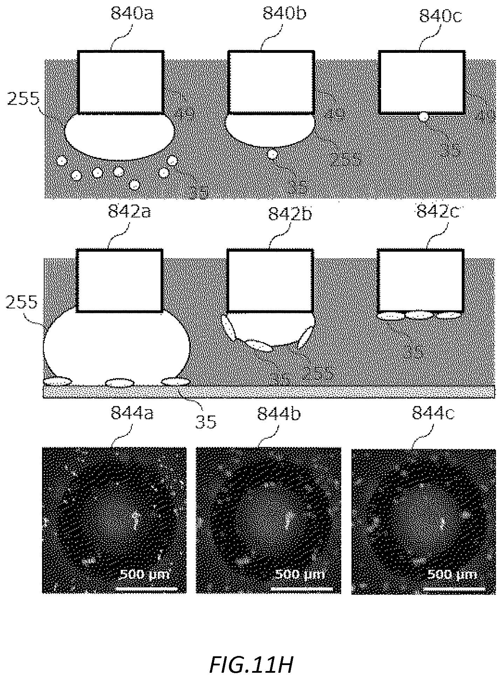

Fig. 11H is a schematic view illustrating how the established cell is retained to be observed at the gas-liquid interface 255 of the air bubble. Since floating cell or a weakly adhered cell moves freely in culture solution due to slight vibration or the like, it is difficult to observe it as it is by using a microscope or the like. At 840a, the volume control unit 200 controls the nozzle actuator 40, and thereby the end 254 of the nozzle 49 is put into liquid medium in the container 25 in which the floating cell (manipulation target 35) is cultured. Then, the pressure generation unit 47 charges gas to the flow channel 51, and thereby an air bubble is formed at the tip of the nozzle 49 and a gas-liquid interface 255 is formed. The volume control unit 200 controls the nozzle actuator 40 or the pressure generation unit 47, and thereby causing the floating cell which is to be the manipulation target 35 to be attached to the formed gas-liquid interface 255. Then, at 840b, the pressure generation unit 47 controls the inner pressure of the air bubble to temporarily retain the cell at the gas-liquid interface 255 of said air bubble. Then, at 840c, the pressure generation unit 47 intakes the gas from the flow channel 51, thereby reducing the air bubble. The cell retained in this state can be observed by using a microscope or the like. Alternatively, the pressure generation unit 47 ma retain the cell without reducing the air bubble, and the retained cell may be observed by using a microscope or the like. In this manner, the cell can be observed without being moved by retaining the cell at the gas-liquid interface 255 of the air bubble according to the present embodiment.

In addition, in a case of adherent cells dispersed in the solid phase of the container 25, in order to perform observation with a microscope or the like, it is necessary to perform the observation in a broad field of view by moving the stage. At 842a, the volume control unit 200 controls the nozzle actuator 40, and thereby the end 254 of the nozzle 49 is put into liquid medium in the container 25 in which the adherent cell (manipulation target 35) is cultured. Then, the pressure generation unit 47 charges gas to the flow channel 51, and thereby an air bubble is formed at the tip of the nozzle 49 and a gas-liquid interface 255 is formed. Then, the volume control unit 200 controls the nozzle actuator 40 or the pressure generation unit 47, thereby controlling the gas-liquid interface 255 to cause the cell to be attached to the gas-liquid interface 255 to be exfoliated. Then, at 842b, the pressure generation unit 47 temporarily retains the cell at the gas-liquid interface 255 of said air bubble. Then, at 842c, the pressure generation unit 47 intakes the gas from the flow channel 51, thereby reducing the air bubble. Cells retained in such state can be observed at once by using a microscope or the like since the cells exist in a narrow range in a plane at the same z position. In this manner, the field of view to be observed can be reduced by retaining the cell at the gas-liquid interface of the air bubble according to the present embodiment.

As an example of such observation methods, Kato III cells which are floating cells were dispersed in the solid phase, and some of the cells were caused to be attached to the gas-liquid interface 255 (844a). Subsequently, the volume control unit 200 controlled the inner pressure of the air bubble via the pressure generation unit 47, thereby retaining the cells at the gas-liquid interface 255 while reducing the air bubble, and when focus was made on the retained cells, the surrounding cells were take out of focus (844b). At this time, since the surrounding cells move but the cells retained at the gas-liquid interface 255 do not move when the stage is moved, the retained cells can be easily observed with a microscope (844c).

At S450, the volume control unit 200 determines whether an instruction to squeeze and perform image-capturing of the cell is received at S140. The volume control unit 200 advances the processing to S452 when the determination is positive, and advances the processing to S460 when the determination is negative.

At S452, the volume control unit 200 controls the pressure generation unit 47 and the nozzle actuator 40 to squeeze the cells by using the air bubble. For example, the volume control unit 200 controls the pressure generation unit 47 to form an air bubble at the tip of the flow channel 51, and brings said air bubble into contact with the cell which is to be the manipulation target 35. Note that, when performing the operation of Fig. 11H, the operation may be performed by moving the nozzle 49, or the operation may be performed by moving the stage.

As an example, the volume control unit 200 sends an instruction to the nozzle actuator 40 to move the nozzle 49 in the x, y and z directions from an initial position to a position where the target cell exists. As an example, the volume control unit 200 identifies the position where the target cell exists from an image obtained by performing image-capturing of the target cell by the camera 60 or the camera 70, and controls the nozzle actuator 40 to align the center of the nozzle 49 with the target position to move the nozzle 49.

Here, identifying the position where the target cell exists may be performed by the operator. In this case, the volume control unit 200 may receive, from the input unit 180, an input by the operator related to the position where the target cell exists, to identify the position. In addition, the contact between the air bubble and the cell may be performed by bringing the edge surface of the air bubble into contact with the cell, or may be performed by bringing the side surface of the air bubble into contact with the cell. When bringing the edge surface of the air bubble into contact with the cell, the nozzle actuator 40 or the sample actuator 41 may move the center of the nozzle 49 to be aligned right above the target cell. When bringing the side surface of the air bubble into contact with the cell, the nozzle actuator 40 or the sample actuator 41 may move the center of the nozzle 49 to be aligned near the target cell, and in this case, an air bubble is formed next to the cell and by moving the nozzle 49, the cell can be squeezed gradually from the side.

Then, after the nozzle actuator 40 has moved the nozzle 49 to a target position, the volume control unit 200 controls the pressure generation unit 47 to charge gas to the flow channel 51 and to form an air bubble at the tip of the flow channel 51 at a target position. The volume control unit 200 may move the nozzle 49 and/or the stage and and bring the gas-liquid interface 255 of said air bubble into contact with the cell with the nozzle actuator 40 or the sample actuator 41. When the position of the nozzle 49 is to be fixed, the gas-liquid interface 255 and the cell may be brought into contact with each other by moving the stage. The volume control unit 200 may perform control to enlarge the gas-liquid interface 255 via the pressure generation unit 47, thereby bringing the cell into contact with the gas-liquid interface 255.

As an example, after the volume control unit 200 operates the plunger of the syringe pump of the pressure generation unit 47 at a preset operating amount to form an air bubble of a preset volume, it moves the nozzle 49 and/or the stage with the nozzle actuator 40 or the sample actuator 41 such that said nozzle 49 is positioned at a position at which the air bubble squeezes the cell. Then, the volume control unit 200 may squeeze the cell by controlling the pressure generation unit 47 to enlarge the air bubble formed at the tip of the flow channel 51, or by controlling the nozzle actuator 40 to move the nozzle 49 toward the cell and press the air bubble against the cell.

In addition, as an example, the volume control unit 200 may squeeze the cell controlling the pressure generation unit 47 to form an air bubble of a preset volume at the tip of the flow channel 51, after the nozzle 49 is moved to be extremely close to the cell.

Then, at S454, the imaging control unit 171 sends an instruction to the camera 60 or the camera 70 to perform image-capturing of the squeezed cell. The camera 60 or the camera 70 captures an image, and sends the image to the image processing unit 300. The image processing unit 300 may record the image to the recording unit 190, and/or output the image to the output unit 160.

In addition, instead of this/in addition to this, the sensor unit 48 or the nozzle actuator 40 may measure the pressure by which the cell is squeezed by the air bubble, and send the measured value of pressure to the volume control unit 200. The camera 60 or the camera 70 may repeatedly perform image-capturing while varying the pressure by which the cell is squeezed with the volume control unit 200. The pressure by which the cell is squeezed can be varied by controlling the pressure generation unit 47 by the volume control unit 200 to vary the inner pressure and/or volume of the air bubble.

As an example, after the nozzle 49 is moved to be extremely close to the cell, the volume control unit 200 may control the pressure generation unit 47 to form an air bubble at the tip of the flow channel 51, and varies the inner pressure and/or volume of the air bubble to squeeze the entire cell or various portions of the cell, while maintaining or varying the pressure at which the cell is squeezed. It is expected that the various portions of the cell have different hardness due to the composition or distribution cytoplasmic membrane or organelle in the cell. In this manner, the composition or distribution of cytoplasmic membrane in the cell or organelle in the cell can be analyzed with the pressure and/or the observed image when being squeezed by the air bubble as an indicator. By squeezing the cell, the thickness of the cell is reduced, which allows the structure at the core of the cell to be clearly observed, and the cell is laterally expanded, which allows the structure in proximity to be separately and individually observed. By peforming observation while squeezing the cell, information related to the force applied to the cell and the amount of morphological change inside and ouside the cell can be obtained, which allows analysis of the information related to the mechanics of the cell. After the camera 60 or the camera 70 has performed image-capturing of the squeezed cell, the volume control unit 200 advances the processing to S500.

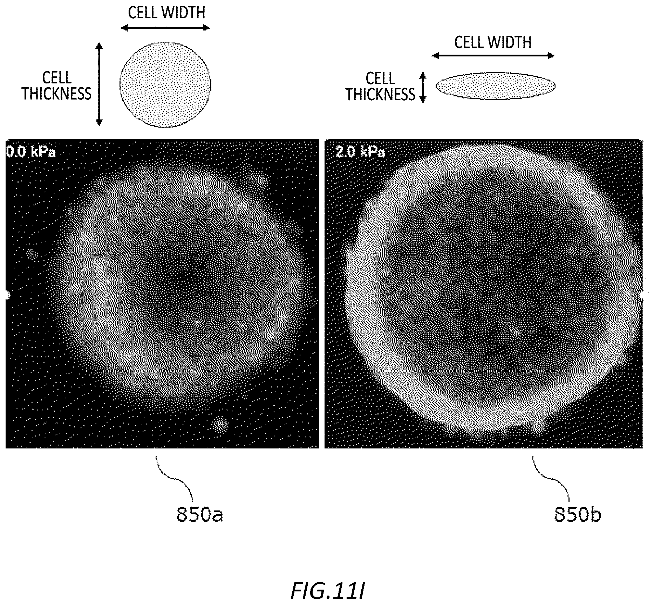

Fig. 11I illustrates how established cells were squeezed by using the air bubble, and how the core of the cell was observed according to the present embodiment. A nucleus and cytoplasm of spheroid (850a) formed from a HT29 cell in a living state was dyed and squeezed by using the air bubble, thereby allowing the structure at the core of the cell such as the nucleus to be observed (850b). Compared at the center portion where it is particularly thick, the nucleus cannot be seen due to the center portion in 850a before the squeezing, but the nucleus can be confirmed at the center portion in 850b, which was observed while being squeezed. Conventionally, in order to observe a structure at the core of the cell, observation was performed by fixing the cell with formalin, methanol or the like and thinly slicing the cell for observation, or by using a specific microscope for core observation. According to the present embodiment, a structure at the core of the cell can be subjected to core observation in a living state without using a specific microscope. Further, although it is difficult to recognize individual nucleus due to the tight connection of nulei with one another for spheroid before the squeezing (850a), with the spheroid observed while being squeezed (850b), due to expansion of spheroid in the vertical/lateral direction, sufficient space was generated between the nuclei, which allowed the nuclei to be independently recognized. Conventionally, in order to independently recognize two or more organelles in proximity inside the cell, research and development are carried out for microscopic system in which optical systems or fluorescent labeling method has been improved, which are called super-resolution microscopes. The resolution is improved in these microscopic techniques, but the observation field is narrower and the capturing time is longer. According to the present embodiment, two or more organelles in proximity inside the cell can be independently recognized, without using a specific microscope, in a living state and within a short image-capturing time while maintaining an observation field. In addition, from the observed image of the squeezed cell and mechanics information, it is also possible to replicate a three-dimensional structure of the original cell.

At S460, the volume control unit 200 controls the manipulation unit 101 such that, among the instructions received at S140, necessary manipulation other than S410 to S450 is performed on the manipulation target 35. For example, the manipulation may be evaluation of adhesiveness of the cell, induction of cell differentiation or the like, which will be described below. Having finished S460, the volume control unit 200 advances the processing to S500.

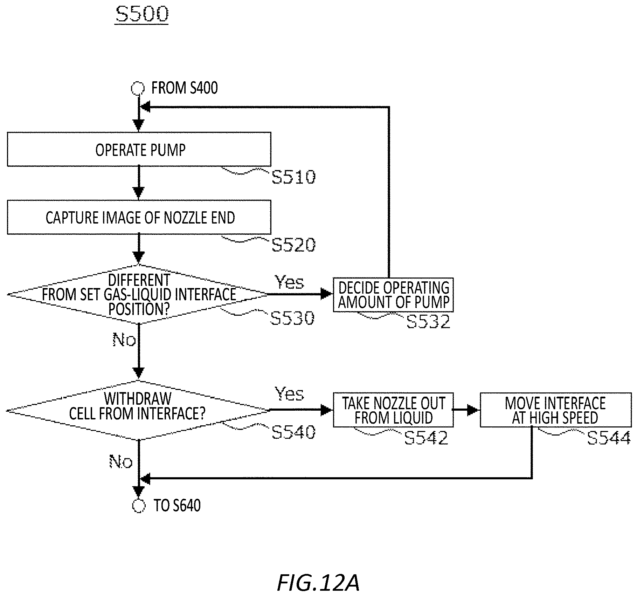

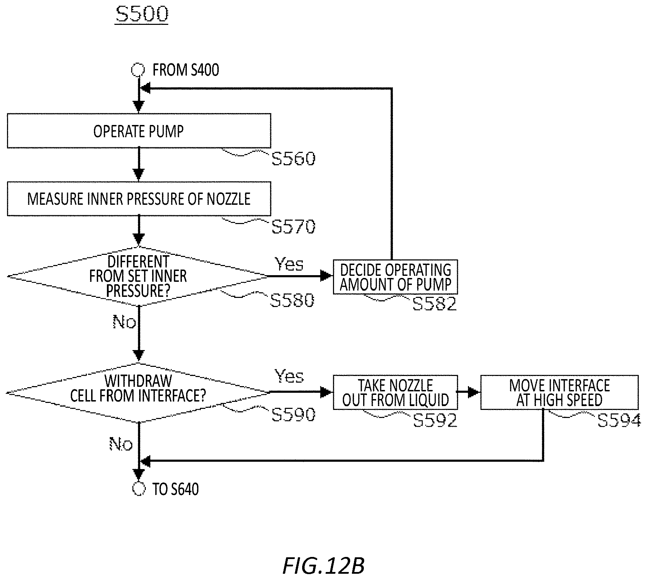

At S500, the volume control unit 200 controls the pressure generation unit 47 such that the gas-liquid interface 255 is reduced. Reducing the gas-liquid interface 255 may include removing the air bubble. Here, when reducing or removing the air bubble, collection of the manipulation target 35 may be performed simultaneously. At S500, the step of reducing the gas-liquid interface 255 includes the steps of S510 to S544 as shown in Fig. 12A, or includes the steps of S560 to S594 as shown in Fig. 12B.

Fig. 12A is an example of a flow for reducing the gas-liquid interface 255 based on an image obtained by capturing the position of the end 254 of the nozzle 49.

At S510, the volume control unit 200 controls the pressure generation unit 47 to perform suction operation on the flow channel 51, and to capture the gas-liquid interface 255 from the tip of the flow channel 51. At this time, liquid is also simultaneously captured. The liquid to be captured may be used to withdraw the manipulation target 35 from the gas-liquid interface 255. Note that, withdrawal includes returning the state of the manipulation target 35 and the gas-liquid interface 255 to a state in which the manipulation target 35 and the gas-liquid interface 255 are in contact with each other to a state in which they are not in contact with each other. The liquid to be captured may be liquid or the like (for example, medium) accommodated in the container 25, or may be another liquid retained in the liquid storage unit 54.

For example, the volume control unit 200 sends an instruction to the pressure generation unit 47 to pull the plunger of the syringe pump by a preset distance, or to the actuator of the pressure generation unit 47 to pull the plunger of the syringe pump until a preset pressure is reached. Having received an instruction from the volume control unit or the air-intake control unit in the volume control unit 200, the pressure generation unit 47 intakes gas. As a result, the gas-liquid interface 255 is reduced (the air bubble is removed), and the gas-liquid interface 255 is captured in the flow channel 51. After the gas-liquid interface 255 is reduced (the air bubble is removed), the volume control unit 200 advances the processing to S520.

Then, at S520, the flow channel capturing camera 42 captures an image of the end 254 of the nozzle 49, and sends the image to the image processing unit 300. The image processing unit 300 may record the image to the recording unit 190, and/or output the image to the volume control unit 200.

Then, at S530, the volume control unit 200 determines whether the position of the gas-liquid interface 255 captured by the flow channel 51 is different from the position preset to the volume control unit 200, based on the captured image of the end 254 of the nozzle 49. The volume control unit 200 advances the processing to S532 when the position is different, and if not, advances the processing to S540. For example, the volume control unit 200 may calculate a difference between the position of the gas-liquid interface 255 calculated based on a captured image of the end 254 of the nozzle 49 and a preset position, and if the difference is threshold or more, the volume control unit 200 may determine that the position of the gas-liquid interface 255 captured by the flow channel 51 is different from the set position.

At S532, the volume control unit 200 decides the operating amount of a plunger of a syringe pump of the pressure generation unit 47. For example, in oder to capture the gas-liquid interface 255 to a preset position of the gas-liquid interface 255, the volume control unit 200 decides the operating amount of the plunger of the syringe pump of the pressure generation unit 47 (for example, a distance by which the plunger of the syringe pump is pushed or pulled). The volume control unit 200 sends an instruction to the pressure generation unit 47 to operate by the decided operating amount. For example, the volume control unit 200 may decide the operating amount according to the size of the difference calculated at S530.

The operating amount may be an operating amount of the actuator of the pressure generation unit 47, or may be additional pressure to be loaded on the syringe pump. The pressure generation unit 47 receives the instruction, and the volume control unit 200 advances the processing to S510. At second and subsequent S510, the pressure generation unit 47 performs operation by an amount according to the operating amount.

At S540, when the instruction received at S140 includes withdrawing the cell from the interface (for example, cell recovery), the volume control unit 200 advances the processing to S542, and if not, the volume control unit 200 advances the processing to S640.

At S542, the volume control unit 200 sends an instruction to the nozzle actuator 40 to take the nozzle 49 out from the liquid. After the nozzle actuator 40 has taken the nozzle 49 out from the liquid by moving the nozzle 49 upwardly by a preset distance, the volume control unit 200 advances the processing to S544.

Then, at S544, the volume control unit 200 controls the pressure generation unit 47 to move the gas-liquid interface 255 between gas and liquid in the flow channel 51 at high speed. In this manner, the cell attached to the gas-liquid interface 255 is withdrawn from the gas-liquid interface 255, and is moved to the liquid. For example, by causing the pressure generation unit 47 to rapidly perform reciprocating operation of the plunger of the syringe pump, the volume control unit 200 causes repeated charging and intaking of air in the flow channel 51 to move the gas-liquid interface 255 at high speed. Also, in addition to/instead of this, the volume control unit 200 may cause the nozzle actuator 40 to perform high-speed reciprocating movement of the nozzle 49 in an upward/downward direction ($\pm$ z direction) and/or a vertical/lateral direction ($\pm$ xy direction) to move the gas-liquid interface 255 in the flow channel 51 at high speed.

The volume control unit 200 may cause the high-speed movement of the gas-liquid interface 255 on the spot (where S542 is performed), or may cause it after causing the nozzle actuator 40 to put the nozzle 49 in the liquid designated as the movement destination. From the information of the inner pressure in the nozzle 49 or the like received from the sensor unit 48, the volume control unit 200 can appropriately withdraw the cell attached to the gas-liquid interface 255 from the gas-liquid interface 255 by controlling the movement velocity of the liquid or the like. In addition, the volume control unit 200 may cause the gas-liquid interface 255 to vibrate by forming an electric field for the nozzle 49.

Further, the volume control unit 200 may control the cell to be withdrawn from the gas-liquid interface 255 by bringing the air bubble into contact with a filter. The volume control unit 200 may withdraw the cell from the gas-liquid interface 255 by controlling the liquid storage unit 54 to add liquid that reduces free energy of the interface. Otherwise, the volume control unit 200 may control the nozzle actuator 40 and the pressure generation unit 47 to cause an air bubble to be formed at the tip of the nozzle 49 at the designated movement destination, and withdraw the cell by rubbing it against the bottom surface of the container 25, which is the designated movement destination. In addition, the volume control unit 200 may cause the cell to be propelled to be withdrawn by controlling the pressure generation unit 47 to raise the inner pressure of the air bubble. In addition, the cell may be withdrawn from the gas-liquid interface 255 by using liquid that reduces the surface free energy as the liquid to be the movement destination. After withdrawing the cell from the interface, the volume control unit 200 advances the processing to S640.

Fig. 12B is an example of a flow for reducing the gas-liquid interface 255 based on the inner pressure in the nozzle 49.

At S560, the volume control unit 200 controls the pressure generation unit 47 to cause the flow channel 51 to perform suction operation, and to capture the gas-liquid interface 255 from the tip of the flow channel 51. The step of S560 may be the same as the step of S510. Then, the volume control unit 200 advances the processing to S570.

Then, at S570, the sensor unit 48 measures the inner pressure in the nozzle 49 and sends the measured value of the inner pressure in the nozzle 49 to the volume control unit 200. Note that, instead of the sensor unit 48, the nozzle actuator 40 may measure the inner pressure in the nozzle 49 and send the measured value of inner pressure to the volume control unit 200.

Then, at S580, the volume control unit 200 determines whether the measured value of inner pressure in the nozzle 49 is within the preset range of inner pressure. The volume control unit 200 advances the processing to S582 when the measured value of the inner pressure is outside the preset range of the inner pressure, and if not, advances the processing to S590. For example, the volume control unit 200 may calculate the difference between the preset inner pressure and the measured inner pressure in the nozzle 49, and if the difference is equal to or more than a threshold, determine that the set inner pressure is not reached.

At S582, the volume control unit 200 decides the operating amount (for example, the distance by which the plunger of the syringe pump is pushed or pulled) of a plunger of a syringe pump of the pressure generation unit 47, in order to achieve the set inner pressure in the nozzle 49. The volume control unit 200 sends an instruction to the pressure generation unit 47 to operate by the decided operating amount. For example, the volume control unit 200 may decide the operating amount according to the size of the difference calculated at S580.

The operating amount may be an operating amount of the actuator of the pressure generation unit 47, or may be additional pressure to be loaded on the syringe pump. The pressure generation unit 47 receives the instruction, and the volume control unit 200 advances the processing to S560. At second and subsequent S560, the pressure generation unit 47 performs operation by an amount according to the operating amount.

At S590, when the instruction received at S140 includes withdrawing the cell from the gas-liquid interface 255 (for example, cell recovery), the volume control unit 200 advances the processing to S592. The steps of S590 to S594 may be the same as the steps of S540 to S544. Having finished S594, the volume control unit 200 advances the processing to S640. When the instruction received at S140 does not include withdrawing from the gas-liquid interface 255 the cell from the gas-liquid interface 255, the volume control unit 200 advances the processing to S640.

Then, at S640, the information processing device 170 receives an input related to releasing of the manipulation target 35 from the operator via the input unit 180. When the information processing device 170 receives an instruction to release the manipulation target 35, the information processing device 170 advances the processing to S645, and if not, advances the processing to S650.

At S645, the volume control unit 200 may send an instruction related to the movement destination of the collected manipulation target 35 to the nozzle actuator 40. For example, the movement destination of the manipulation target 35 may be one designated with a display area in the GUI by the operator, as shown in Fig. 7B. The nozzle actuator 40 may release submerge the nozzle 49 including the manipulation target 35 attached to the gas-liquid interface 255 or withdrawn from the gas-liquid interface 255 in the liquid which is the movement destination, and release it in the liquid which is the movement destination. After the nozzle actuator 40 has released the collected cell in the liquid which is the movement destination, the volume control unit 200 advances the processing to S650. Note that, the cell released in the liquid which is the movement destination may be observed by using the microscope unit 50.

At S650, when there is another manipulation target 35, the volume control unit 200 advances the processing to S660. At S650, when there is no other manipulation target 35, the volume control unit 200 advances the processing to S680.

At S660. In manipulating another manipulation target 35, when it is required to replace the nozzle 49, the volume control unit 200 advances the processing to S670, and when it is not required to replace the nozzle 49, advances the processing to S200.

At S670, the flow channel control unit 250 sends an instruction to the flow channel replacement unit 53 to remove the nozzle 49 with which the nozzle actuator 40 is equipped, and to dispose it to a nozzle disposal unit of the flow channel replacement unit 53. Note that, the nozzle may be retained with the cell captured in the nozzle 49 without being released, and analysis of the captured cell may be performed subsequently. In this case, the nozzle 49 may be retained in the nozzle retaining unit of the flow channel replacement unit 53 without being disposed. After the flow channel replacement unit 53 has disposed the nozzle 49, the processing is advanced to S180.

At S680, disposal of the nozzle 49 may be performed in a procedure similar to S670. The flow channel replacement unit 53 disposes the nozzle 49, and the flow is ended.

In the above-described flow, as an example of manipulating the manipulation target 35, cases of removal of unnecessary cells, collection of cytoplasm and/or cytoplasmic membrane, collection and passage of the cell, retaining of the cell, and squeezing of the cell have been described. Examples of manipulation include several others besides those listed above.

An example of manipulation includes applying culture substrate or medicament to the solid phase at the bottom surface of the container 25 and evaluating the adhesiveness of these culture substrate or medicament to the cell. Since adhesiveness to the cell can be evaluated with the inner pressure of the air bubble, the movement velocity or the load of the nozzle when the cells are exfoliated, as an indicator, the effectiveness of the culture substrate or the medicament on cell adhesion can be evaluated.

Another example of manipulation includes sorting out of cells. Squeezing of the cell is performed by the air bubble according to the flow described above. It is assumed that consitituents or physical properties in the cytoplasmic membrane or the cell may be different depending on the type of the cell. Therefore, it is assumed that the course of change in the shape of the cell or the shape of the cell may be different, after the volume control unit 200 controlled the nozzle actuator 40 and/or the pressure generation unit 47 to start squeezing of the cell with the air bubble, when it is stopped, and when it is released. In addition, it is also assumed that some cell may be ruptured due to the squeezing, depending on the type of the cell. Cells can sorted out with these as indicators.

Another example of manipulation includes observing change in the shape over the course of squeezing of the cell. Squeezing of the cell is performed by the air bubble according to the flow described above. For example, over the course of squeezing the cell, the entire cell or various portions of the cell may be squeezed while varying the pressure by which the cell is squeezed, and perform image-capturing of the variation in the shape of the cell.

Another example of manipulation includes rupture or cutting due to squeezing of the cell. By applying large pressure to the cell, the cell can be ruptured or cut. By causeing the cell to be ruptured or cut, the cytoplasmic membrane, cytoplasm, and/or organelle or the like can be collected, and connection between cells (for example, synapse which is connection between nerve cells) can be cut.

Another example of manipulation includes induction of cell differentiation. Differentiation of osteoblast, muscle cells, and endothelial precursor cells or the like are known to be induced by applying mechanical stimulus thereto. The pressure generation unit 47 performs squeezing on these cells by using the air bubble according to the flow described above, thereby allowing differentiation to be induced.

Another example of manipulation includes gene transfer to the cell. The volume control unit 200 uses the air bubble to cause a vesicle-like substance such as a cytoplasmic membrane to be attached to the gas-liquid interface 255 and to touch the cytoplasmic membrane, according to the flow described above, and thereby the content of the vesicle is captured in the cell due to membrane fusion. At this time, by having a gene contained in the vesicle, it is possible to capture a gene in the cell. In addition, not only a gene, but other polymers can be captured in the cell via a pore. In addition, according to the flow described above, it is likely that a tiny gap is generated in a part of the membrane over the course of deformation of the cell when the volume control unit 200 squeezes the cell at the gas-liquid interface 255 by using the air bubble. At this time, by adding a gene in the cell medium, the gene can be captured in the cell via the gap. In addition, not only a gene, but other polymers can be captured in the cell via the gap.

Another example of manipulation includes cooperation with external devices like a cell culture device such as a fermenter, or a cell analysis device such as a cell sorter. The volume control unit 200 may use the air bubble to capture the cell in the flow channel 51 according to the flow described above, and release it to a designated position at a cooperating external device, thereby moving the cell. In addition, since the flow channel 51 is directly connected to the external device, the cell captured in the flow channel 51 may be sent to the external device, thereby moving the cell.

Another example of manipulation includes manipulating emulsion. Emulsion is a drop in oil or oil droplet in aqueous solution. In order to stabilize formed emulsion, surfactant or the like which is an amphipathic substance may be included in the emulsion. The surfactant or the like is arranged to surround the drop or oil droplet, to form a monomolecular film at the interface. Such a monomolecular film can be found in a part of the organelle in the cell, such as endosome or lipid droplet. According to the flow described above, the volume control unit 200 may use the air bubble to cause the emulsion to be attached to the gas-liquid interface 255, or may perform further manipulation.

As a method for exfoliating and/or collecting the cell, there is a method of exfoliating the cell by locally modifying a substrate by using a special substrate that reacts to temperature or light, or a method of exfoliating the cell with ultrasonic wave, but these methods requires means for collecting the cell. However, the method of the present invention has an advantage that it does not require a special substrate, and includes both means for exfoliating the cell and means for collecting the cell. In addition, as means for collecting the exfoliated cell, there is a method of collecting the cell by a liquid current which sucks the liquid, such as an aspirator, but there is a possibility that a large amount of liquid may be simultaneously collected with the cell, or that surrounding cells other than the target cell may be involved. However, in the method of the present invention, there is an advantage that it is possible to collect the cell attached to the gas-liquid interface by capturing the gas-liquid interface in the nozzle, and that the target cell can be easily collected without involving cells other than the target cell with a very small amount of fluid. In addition, it is known that applying a strong liquid current when sucking the liquid causes an adverse impact to the cell, but there is an advantage that by using the method of the present invention, such an adverse impact can be avoided.

Next, the manipulation target 35 can be easily manipulated by controlling the surface free energy of the gas-liquid interface 255 in the method of manipulating the manipulation target 35 by using the gas-liquid interface 255 described above. Details of a method for controlling the surface free energy of the gas-liquid interface 255 will be described below. Although a case where a cell is the manipulation target 35 is described as an example in the description below, the manipulation target 35 may be another organism.

The method for controlling the surface free energy at the gas-liquid interface 255 described below is not limited to be perfomed before the step of forming an air bubble (enlarging the gas-liquid interface 255), that is, to be performed immediately before performing the step of S300. For example, the method for controlling the surface free energy of the gas-liquid interface 255 can be performed at any time, in the middle of the step and sub-steps of forming the air bubble (the sub-steps of S300), or after performing the step of forming the air bubble (immediately before performing the step of S400, and/or in the middle of the sub-steps of S400).

For example, when it is desired to attach the cell to the gas-liquid interface 255, it becomes easier to cause the cell to be attached to the gas-liquid interface 255 by controlling the interface energy of the gas-liquid interface 255 to be increased before the step of forming the air bubble. In addition, when it is desired to withdraw the cell attached to the gas-liquid interface 255, it becomes easier to withdraw the cell from the gas-liquid interface 255 by controlling the interface energy of the gas-liquid interface 255 to be decreased in the middle of the step of forming the air bubble or after performing the step of forming the air bubble. For example, at the step of S645, when releasing the collected cell to a designated position, it may becomes easier for the cell to be withdrawn from the gas-liquid interface 255 by controlling the interface energy of the gas-liquid interface 255 to be decreased. Details will be described below.

In addition, the step of manipulating the manipulation target 35 (the sub-steps of S400) may include controlling the surface free energy of the gas-liquid interface 255. For example, the step of manipulating the manipulation target 35 (the sub-steps of S400) may be performed in the middle of controlling the surface free energy of the gas-liquid interface 255, or may be performed after controlling the surface free energy of the gas-liquid interface 255.

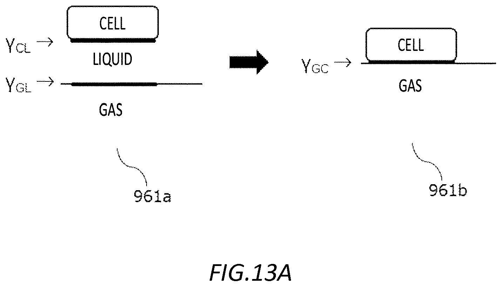

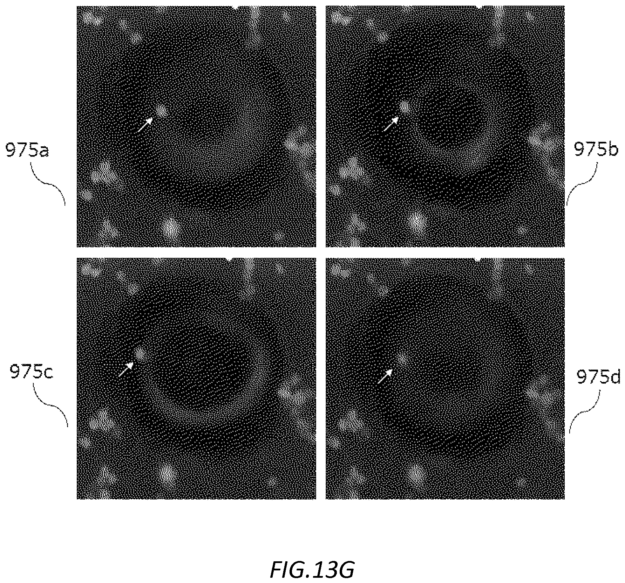

Fig. 13A illustrates the variation in the surface free energy when the manipulation target 35 is attached to the gas-liquid interface 255. 961a in Fig. 13A shows the state before the cell which is the manipulation target 35 is attached to the gas-liquid interface 255, and 961b shows the state after the cell is attached to the gas-liquid interface 255.

In 961a, $\gamma_{CL}$ represents the surface free energy between the cell and liquid in the attachment area S, and $\gamma_{GL}$ represents the surface free energy E2 between gas and liquid in the attachment area S. In 961b, $\gamma_{GC}$ represents the surface free energy E1 between gas and the cell in the attachment area S. When the cell is attached to the gas-liquid interface 255, that is, when the state is transitioned from 961a to 961b, the surface free energy variation is represented by formula 1 below:

$$[Formula\ 1] \qquad \Delta G = \gamma_{GC} \times S - (\gamma_{GL} + \gamma_{CL}) \times S$$

Thermodynamically speacing, as long as the $\Delta G$ is in the above-described formula 1 is a negative value, attachment of the cell to the gas-liquid interface 255 may be spontaneously progressed.

Here, when the manipulation target 35 is an organism having a surface that exhibits hydrophilicity (for example, an animal cell), and the liquid 261 is solution appropriate for growing, maintaining, or retaining the manipulation target 35 therein (for example, culture solution, buffer solution, or the like), the values of $\mu_{GC}$ and $\gamma_{GL}$ are in the order of $10^{-2}$ J/$m^2$ uder 25 °C, while the value of $\gamma_{CL}$ is in the order of $10^{-4}$ J/ $m^2$. Therefore, in the above-described formula 1, contribution of $\gamma_{CL}$ to $\Delta G$ is small, and can be virtually ignored. Accordingly, the above-described formula 1 can be considered as below:

[Formula 2]    $\Delta G = \gamma_{GC} - \gamma_{GL}$

That is, the value of $\Delta G$ may be considered to be the difference obtained by subtracting E2 from E1 (E1 - E2). Note that, the difference obtained by subtracting E2 from E1 represents (E1 - E2), as apparent from the above-described formula 2, and it is to be noted that it is not |E1 - E2|, that is, the absolute value of the difference (E1 - E2) obtained by subtracting E2 from E1.

Controlling the surface free energy may be controlling the value of $\Delta G$ described above, that is, the difference obtained by subtracting E2 from E1. For example, controlling the surface free energy may be controlling the value of $\Delta G$ in the above-described formula 2 to be decreased, or controlling to suppress the value of $\Delta G$ not to increase. Since the cell is made easier to be attached to the gas-liquid interface 255 by performing control in this manner, the above-described control is effective when it is desired to perform manipulation to attach the cell to the gas-liquid interface 255.

For example, controlling the surface free energy may be controlling the value of $\Delta G$ in the above-described formula 2 to be increased, or controlling to suppress the value of $\Delta G$ not to be decreased. Since the cell is made more difficult to be attached to the gas-liquid interface 255 by performing control in this manner, the above-described control is effective when it is desired to perform manipulation to withdraw, from the gas-liquid interface 255, the attached cell from the gas-liquid interface 255. In addition, the above-described control is effective when it is not desired to strongly attach the cell to the gas-liquid interface 255, such as when it is desired to move the cell in the same container 25, when it is desired to use an air bubble to squeeze the cell and analyze it, or the like.

Further, since the value of $\gamma_{GC}$ (E1) has a narrow width for aritificial control, the value of $\gamma_{GC}$ (E1) is approximately constant. Therefore, controlling the value of $\Delta G$ in the above-described formula 2 may be performed by controlling the value of $\gamma_{GL}$ (E2), that is, the surface free energy between gas and liquid. Note that, the value of $\Delta G$ in the above-described formula 2 may be controlled not only by controlling the value of $\gamma_{GL}$ (E2), but also by performing modification of the sugar chain, lipid or the like on the surface of the cell to change the value of $\gamma_{GC}$ (E1) and $\gamma_{CL}$.

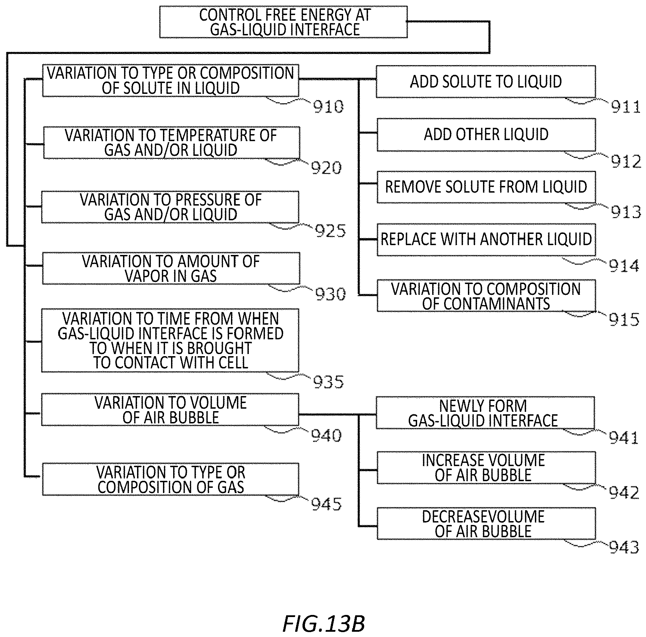

Fig. 13B illustrates a method for varying the surface free energy of a gas-liquid interface 255. The method for varying the surface free energy of the gas-liquid interface 255 may be performed by combining one or more methods shown in Fig. 13B.

Here, the mechanism of varying the surface free energy of the gas-liquid interface 255 will be described first. At the gas-liquid interface 255 of the air bubble, intermolecular force is applied between molecules which constitute the liquid. Although the molecules interact with each other to be stabilized inside the liquid, there is excessive energy (which is referred to as surface free energy or surface tension) at the surface between the air bubble (the gas-liquid interface 255) since there is a lack of interacting molecules. Therefore, the larger the intermolecular force between molecules constituting the liquid, the larger the surface free energy becomes when there is a lack of molecules to interact with.

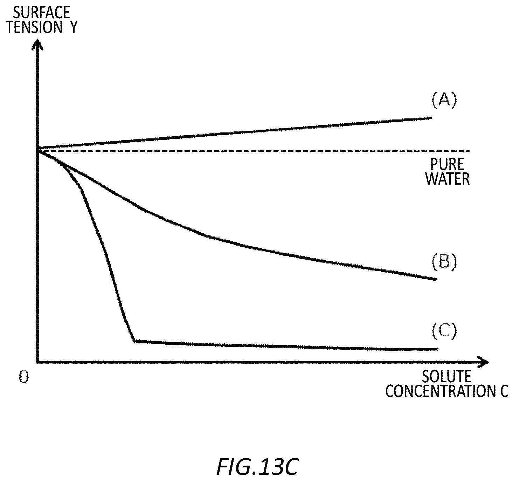

Fig. 13C illustrates a mechanism by which surface free energy of the gas-liquid interface 255 may be varied due to the concentration of a solute. The horizontal axis represents the concentration c of the solute in the liquid, and the vertical axis represents the surface free energy (surface tension y). The surface free energy (surface tension y) when the solute concentration is zero is set as the value of surface tension of pure water, which is displayed with a dotted line. Fig. 13C illustrates a case where the liquid is an aqueous solution.

(A) illustrates variation of the surface tension $\gamma$ when inorganic salt is added to the liquid as a solute. The inorganic salt may be a chemical compound which ionizes the liquid such as an aqueous solution into cation and anion. For example, the inorganic salt may be metal salt such as sodium chloride, potassium chloride, phosphate and alum. Inorganic salt is ionized in the solution into cation and anion, and these ions are caused to be hydrated by being surrounded by water molecules, to be stabilized. The water molecules are stabilized better by interacting with cations and anions having Coulomb force, rather than interacting with another water molecule to form a hydrogen bond. Therefore, it is impossible for the cation and the anion derived from the inorganic salt to be attached to the gas-liquid interface 255. As a result, inside the liquid, an interaction occurs between the cation and anion derived from the inorganic salt and the water molecules, which is caused by a stable and stronger Coulomb force. On the other hand, the gas-liquid interface 255 with the air bubble is at a state closer to pure water with only hydrogen bond, energy difference occurs between the inside of the liquid and the liquid at the surface of the gas-liquid interface 255, which results in increase of the surface free energy at the gas-liquid interface 255. That is, surface free energy is increased by adding inorganic salt to the liquid.

(B) illustrates variation of the surface tension $\gamma$ when a polar organic compound is added to the liquid as a solute. The polar organic compound may be an organic chemical compound having a functional group with high polarity, such as an amino group, a carboxyl group, or hydroxyl group. For example, the polar organic compound may be alcohol, fatty acid, amino acid, peptide, protein, sugar or the like. Since the polar organic compound has a hydrophobic functional group and a hydrophilic functional group, it attaches to the gas-liquid interface 255. For example, the hydrophobic portion of the polar organic compound interacts with gas and the hydrophilic portion interacts with liquid to attach to the gas-liquid interface 255, that is, by adding the polar organic compound to the solution, the gas-liquid interface 255 is covered with molecules of the polar organic compound, which results in decrease in water molecules at the surface in contact with the vapor phase and decrease in the surface free energy.

(C) illustrates variation of the surface tension $\gamma$ when a surfactant is added to the liquid as a solute. The surfactant may be an amphipathic chemical compound having both a hydrophobic portion and a hydrophilic portion within the molecule. For example, the surfactant may be a cationic surfactant (such as a cationic soap), an anionic surfactant (such as fatty acid sodium), an amphoteric surfactant (such as a betaine-based surfactant), or a non-ionic surfactant (such as octyl glycoside). Similar to the polar organic compound, the surfactant also interacts with gas and water molecule at the gas-liquid interface 255, but the surface free energy decreases at an extremely lower concentration compared to a case of the polar organic compound. Increasing the concentration of the surfactant causes the gas-liquid interface 255 to be almost completely covered with molecules of the surfactant, which results in an extreme decrease of water molecules at the surface in contact with the vapor phase. In addition, by adding a surfactant, the surfactant interacts with the surface of the organism, and the surface free energy $\gamma_{GC}$ and $\gamma_{CL}$ may decrease.

That is, at 910 in Fig. 13B, the size of surface free energy of the gas-liquid interface 255 can be regulated by causing variation to the type or composition of the solute included in the liquid. For example, at 911, by adding a solute to the liquid, the size of the surface free energy at the gas-liquid interface 255 can be regulated. Specifically, as described for Fig. 13C above, the surface free energy at the gas-liquid interface 255 can be increased by add inorganic salt to the liquid as a solute, or alternatively, the surface free energy at the gas-liquid interface 255 can be decreased by adding a polar organic compound or a surfactant to the liquid as a solute.

For example, at 912, the size of the surface free energy may be controled by adding another liquid to the liquid in the container 25. When the liquid is a complete medium, a buffer, a basal medium, or water may be added as another liquid. In this case, the concentration of the polar organic compound included in the complete medium is diluted by adding another liquid. Therefore, the surface free energy at the gas-liquid interface 255 may be regulated to be increased.

For example, at 913, the size of the surface free energy may be regulated by removing the inorganic salt or the polar organic compound from the liquid. Removal of the inorganic salt may be performed by adding a chelating agent such as EDTA to the liquid. Removal of the polar organic compound or the surfactant may be performed by injecting a substrate such as a filter, a column, or a bead in the liquid and the substrate adsorbing the polar organic compound or the surfactant in the liquid, or may be performed by forming a gas-liquid interface 255 in the liquid and causing the polar organic compound or the surfactant to be adsorbed to the gas-liquid interface 255. In this manner, the concentration of the polar organic compound or the surfactant in the liquid can be decreased.

Table 1 is a table indicating the variation of whether the surface free energy at the gas-liquid interface 255 decreases or increases by adjusting various factors of the liquid. As described above, the size of the surface free energy at the gas-liquid interface 255 may be regulated by controlling the concentration of the inorganic salt, the polar organic compound or the surfactant in the liquid.

[Table 1]

| LIQUID ADJUSTMENT FACTOR | AMPHIPHATHIC SUBSTANCE CONCENTRATION | | INORGANIC SALT CONCENTRATION | | TEMPERATURE (PRESSURE) | |
|---|---|---|---|---|---|---|
| CONTROL | REDUCED | RAISED | REDUCED | RAISED | REDUCED | RAISED |
| GAS/LIQUID SURFACE FREE ENERGY | INCREASE | DECREASE | DECREASE | INCREASE | INCREASE | DECREASE |

For example, at 914 in Fig. 13B, consider a case where liquid having surface free energy of E2 at the gas-liquid interface 255 between the liquid and the air bubble is replaced with liquid having surface free energy of E3 at the gas-liquid interface 255 between the liquid and the air bubble. Here, when E3 is greater than E2, the surface free energy at the gas-liquid interface 255 is increased by the replacement of liquid. For example, by replacing the liquid with liquid having a lower concentration of amphipathic substances such as the polar organic compound or the surfactant in the liquid, as compared to the existing liquid, the surface free energy at the gas-liquid interface 255 is increased. For example, by replacing the liquid with liquid having a higher concentration of the inorganic salt in the liquid, as compared to the existing liquid, the surface free energy at the gas-liquid interface 255 is increased.

For example, at 914, consider a case where liquid having surface free energy of E2 at the gas-liquid interface 255 between the liquid and the air bubble is replaced with liquid having surface free energy of E4 at the gas-liquid interface 255 between the liquid and the air bubble. Here, when E4 is smaller than E2, the surface free energy at the gas-liquid interface 255 is decreased by the replacement of liquid. For example, by replacing the liquid with liquid having a higher concentration of amphipathic substances such as the polar organic compound or the surfactant in the liquid, as compared to the existing liquid, the surface free energy at the gas-liquid interface 255 is decreased. For example, by replacing the liquid with liquid having a lower concentration of the inorganic salt in the liquid, as compared to the existing liquid, the surface free energy at the gas-liquid interface 255 is decreased.

In addition, at 920, by varying the temperature of the gas and/or liquid, the surface free energy at the gas-liquid interface 255 may be regulated. When the temperature of the gas and/or liquid is raised, molecular motion at the gas-liquid interface 255 is intensified, and the impact of the intermolecular force which was acted upon between molecules at the gas-liquid interface 255, in particular, between molecules inside the liquid is decreased. Accordingly, when the temperature of the gas and/or liquid is raised, the surface free energy at the gas-liquid interface 255 is decreased, and when the temperature of the gas and/or liquid falls, the surface free energy at the gas-liquid interface 255 is increased. In this manner, the gas and/or liquid may be controlled by the temperature control unit 520 to be warmed or cooled. By varying the temperature of the gas and/or liquid, the surface free energy at the gas-liquid interface 255 may be regulated.

In addition, at 925, the same effect as when the temperature of the gas and/or liquid is raised can be obtained by raising the pressure of the gas and/or liquid, since it also intensifies the molecular motion at the gas-liquid interface 255. That is, when the pressure of the gas and/or liquid is raised, the surface free energy at the gas-liquid interface 255 is decreased, and when the pressure of the gas and/or liquid falls, the surface free energy at the gas-liquid interface 255 is increased. In this manner, the surface free energy at the gas-liquid interface 255 may be regulated also by controlling the pressure of the gas and/or liquid.

Further, at 930, the surface free energy at the gas-liquid interface 255 may be regulated also by varying the amount of water molecules (humidity) included in the gas. When water molecules are included in the gas, since the water molecule in the liquid and the water molecule in the gas interacts with each other at the gas-liquid interface 255, the gas-liquid interface 255 is stabilized. That is, when the amount of water molecule (humidity) included in the gas is increased, the surface free energy at the gas-liquid interface 255 is decreased, and when the humidity is decreased, the surface free energy at the gas-liquid interface 255 is increased. In this manner, the surface free energy at the gas-liquid interface 255 may be regulated also by controlling the humidity of the gas. In addition, the surface free energy at the gas-liquid interface 255 may be regulated in a manner similar to a case of water molecule, by including, in the gas, a volatile substance which may interact with the water molecule in the liquid, other than the water molecule, such as ethyl alcohol.

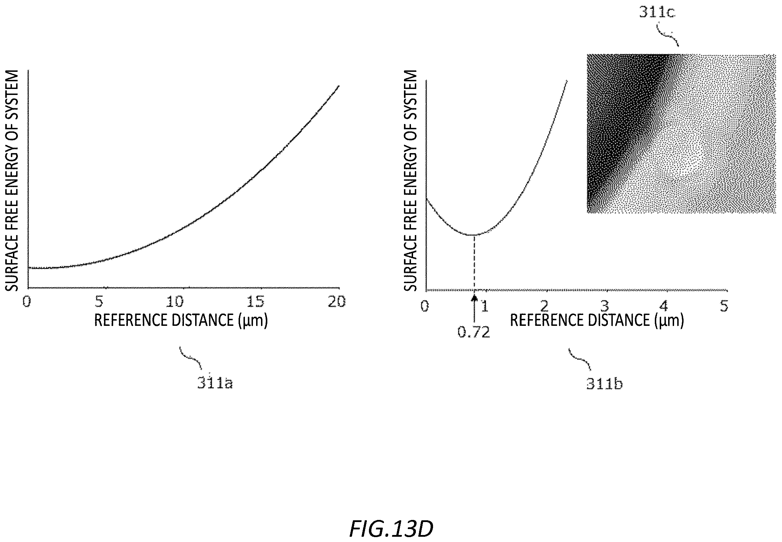

Fig. 13D illustrates the free energy variation of the system over the course of the cell in the buffer coming into contact with the air bubble and completely entering inside air bubble. The surface free energy of the system is the sum of surface free energy between the cell and liquid, the surface free energy between gas and liquid, and the surface free energy between gas and the cell. Since the area of each interface varies over the course of the cell coming into contact with the air bubble to completely enter to the inside of the air bubble, each area is multiplied by the each sufrace free energy to calculate the sum thereof, which allows the free energy of the system at that moment to be obtained. It is assumed that the air bubble is a sphere, and the diameter thereof is 500 $\mu$m. In addition, it is assumed that the cell is a sphere, and the diameter thereof is 20 $\mu$m. 311a represents a graph in which the horizontal axis is the break-in distance of the cell into the air bubble as the reference distance, and the vertical axis is the free energy of the system.

311b is an enlarged view of a portion in 311a having a reference distance of 0 to 5 $\mu$m. According to 311b, the free energy of the system is minimized when the break-in distance is 0.72$\mu$m. That is, when deformation of the cell is ignored, the free energy of the system becomes the lowest and $\Delta G$ becomes a negative minimum value when the cell breaks into the air bubble to a degree of about 3.5 %. That is, since a most stable state is obtained when the cell breaks into the air bubble to a degree of about 3.5 %, the cell, the cell being attached to the air bubble and breaking very slightly into the air bubble may progress spontaneously (the photograph in 311c illustrates how the cell was actually attached to the air bubble). However, it is impossible for the cell to spontaneously enter the air bubble further, or conversely, for the cell to leave the air bubble. When deformation of the cell is considered and the cell is brought into contact to be aligned to the curved surface of the air bubble, since the state in which the product of the contact surfaces of the air bubble and the cell results in the negative minimum value of $\Delta G$, as long as the condition that $\Delta G$ becomes negative for each sufrace free energy is satisfied, which brings the most stable state, the air bubble and the cell is stabilized in a state of being attached to each other.

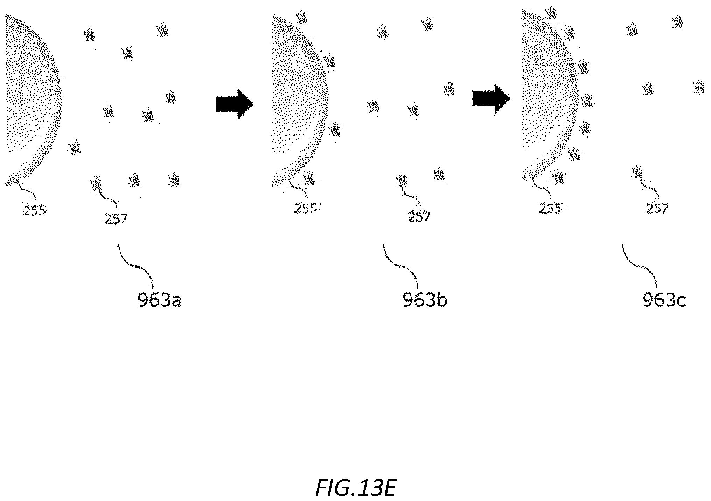

Fig. 13E illustrates the air bubble formed in the liquid including contaminant over time. 963a illustrates the state immediately after the air bubble is formed in the liquid including the contaminant 257 such as protein. At this step, since it is immediately after the air bubble is formed, there is hardly any contaminant 257 attached to the gas-liquid interface 255. Then, 963b illustrates a state after a certain amount of time has elapsed after the air bubble is formed. At this step, a certain amount of contaminant 257 is attached to the gas-liquid interface 255. Then, 963c illustrates a state after a further time has elapsed after the air bubble is formed. At this step, a considerable amount of contaminant 257 is attached to the gas-liquid interface 255.

Since protein which is the contaminant 257 is a polar organic compound having a hydrophobic functional group and a hydrophilic functional group in the molecule, the contaminant 257 may interact with gas and the water molecule at the gas-liquid interface 255. That is, when the contaminant 257 incidentally collides with the gas-liquid interface 255, the contaminant 257 is caused to be attached to the gas-liquid interface 255, and covers the gas-liquid interface 255.

Therefore, the water molecule on the surface in contact with the vapor phase is decreased, and the surface free energy at the gas-liquid interface 255 falls. As a result, it becomes harder for the cell to be attached to the gas-liquid interface 255.

In this manner, when contaminants 257 are included in the liquid, over time, the gas-liquid interface 255 becomes covered with the contaminant 257, and the surface free energy at the gas-liquid interface 255 falls, making it harder for the cell to be attached thereto. Using this phenomenon, at 935 in Fig. 13B, the degree of easiness for the cell to be attached to the gas-liquid interface 255 can be regulated by the time that has elapsed since the air bubble was formed.

At 935, by regulating the time from when the gas-liquid interface 255 is formed to when it comes into contact with the cell, the surface free energy at the gas-liquid interface 255 may be regulated. For example, by bringing the gas-liquid interface 255 into contact with the cell immediately after being formed, the cell can be attached to the gas-liquid interface 255 before the contaminant 257 is attached to the gas-liquid interface 255 and the surface free energy at the gas-liquid interface 255 falls. For example, the cell may be attached to the gas-liquid interface 255 by bringing the gas-liquid interface 255 into contact with the cell within ten seconds after the gas-liquid interface 255 being formed. The time between when the gas-liquid interface is formed and when it comes into contact with the cell to have the cell attached thereto may be ten seconds or less, may be five seconds or less, or further may be one second or less, but it is not limited thereto, and any time can be set.

In addition, at 935, the cell attached to the gas-liquid interface 255 can be made easier to be withdrawn from the gas-liquid interface 255 by decreasing the surface free energy at the gas-liquid interface 255. For example, the cell may be attached to the gas-liquid interface 255 after a preset time has elapsed since the gas-liquid interface 255 is formed. Over time, the gas-liquid interface 255 becomes covered with the contaminant 257, and the surface free energy at the gas-liquid interface 255 falls, which results in the cell becoming harder to be attached to the gas-liquid interface 255. As an example, the cell may be brought into contact with the gas-liquid interface 255 to be attached thereto ten seconds or more, 15 seconds or more, 20 seconds or more, or 30 seconds or more has elapsed since the gas-liquid interface 255 is formed. In this manner, the surface free energy at the gas-liquid interface 255 can be decreased by causing a preset time to elapse since the gas-liquid interface 255 is formed. In this case, an air bubble suitable for a case where there is less desire to cause the cell to be attached to the gas-liquid interface 255, or where it is desired to make it easier form the cell to be withdrawn from the gas-liquid interface 255 can be provided.

In addition, at 915, the size of surface free energy can be regulated also by causing variation to the type or composition of the contaminant 257 included in the liquid. By using this, the degree of easiness of attachment of the cell to the gas-liquid interface 255 can be regulated by varying the type or composition of the contaminant 257 included in the liquid.

For example, liquid containing the contaminant 257 may be a basal medium. The basal medium may be one containing a very small amount of protein or amino acid. As an example, the basal medium is a DMEM (Dulbecco's modified Eagle medium) or Ham's F-12 (Ham F-12 medium). For example, the liquid containing a large amount of the contaminant 257 may be a complete medium. The complete medium may be one obtained by adding, to the basal medium, protein such as serum or cell growth factor, or an amino acid such as L-glutamine.

For example, liquid containing little contaminant 257 may be a buffer. The buffer may be a solution with salt concentration, pH, or the osmotic pressure is regulated to be suitable for the cell. As an example, the buffer may be PBS (phosphate buffered saline), a HANKS buffer, or a HEPES (hydroxyethyl piperazine ethanesulfonic acid) buffer.

For example, the easiness of attachment of the cell to the gas-liquid interface 255 may be regulated by using a plurality of type of liquid having different concentration, types or the like of the contaminant 257 to replace the liquid therewith. For example, when a complete medium is used as the medium in which the cell is cultured, since the complete medium includes a large amount of the contaminant 257, the surface free energy at the gas-liquid interface 255 is low. Therefore, when the liquid is a complete medium, it is hard for the cell to be attached to the gas-liquid interface 255. Therefore, the surface free energy at the gas-liquid interface 255 can be increased to make it easier for the cell to be attached to the gas-liquid interface 255 by replacing the complete medium with liquid containing less contaminant 257 (amphipathic substances such as a polar organic compound), or by replacing it with a solution containing a large amount of inorganic salt.

For example, in order to perform regulation such that it becomes easier for the cell to be attached to the gas-liquid interface 255, the complete medium filled in the container 25 may be entirely or partially (for example, by half the amount) removed, and add a basal medium or a buffer instead to the container 25. For example, in order to perform regulation such that it becomes easier for the cell to be attached to the gas-liquid interface 255, an adequate amount of a basal medium or a buffer may further be added to the complete medium filled in the container 25.

Further, when a basal medium or a buffer is used as the liquid, the liquid may be entirely or at least partially replaced with a complete medium, or an adequate amount of a complete medium may be added to the liquid. By performing replacement or adding in this manner, the surface free energy at the gas-liquid interface 255 can be decreased, and the cell attached to the gas-liquid interface 255 can be made easier to be withdrawn.

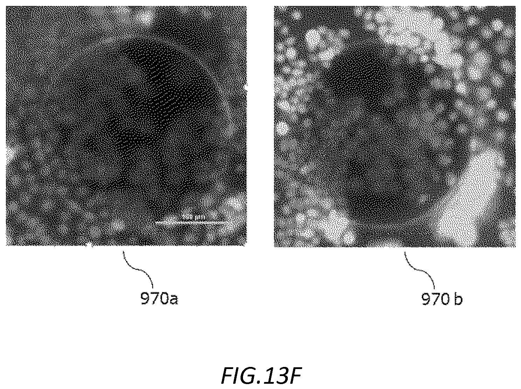

Fig. 13F illustrates an experiment example by which the cell was made easier to be attached to the gas-liquid interface 255 by replacing the liquid from a complete medium to a buffer. For a HeLa cell cultured in a complete medium, the complete medium (970a) was replaced with a PBS buffer (970b). As a result, it was shown that, after the replacement with the PBS buffer, cells were attached to the gas-liquid interface 255 (the white mass-like objects in 970b are the cells).

In addition, at 940, the surface free energy at the gas-liquid interface 255 may be regulated by varying the volume of the air bubble. For example, at 941, by newly forming a gas-liquid interface 255, the surface free energy at the gas-liquid interface 255 can be increased to make it easier for the cell to be attached to the gas-liquid interface 255. For example, the cell may be brought into contact with the gas-liquid interface 255 immediately after increasing the volume (size) of the air bubble to enlarge the gas-liquid interface 255, or within a preset time thereafter. As an example, the gas-liquid interface 255 may be brought into contact with the cell within ten seconds or less after increasing the volume of the air bubble to enlarge the surface area of the gas-liquid interface 255. In this manner, the cell can be caused to be attached to a fresh gas-liquid interface 255 generated by enlarging of the surface area, before the contaminant 257 is attached to the gas-liquid interface 255.

For example, at 942, the stage position control unit or the nozzle position control unit may move the container 25 or the flow channel 51 to a position at which the air bubble can be brought into contact with the organism to form an air bubble, and may bring the gas-liquid interface 255 into contact with the cell while increasing the volume of the air bubble to enlarge the surface area of the gas-liquid interface 255. By doing so, the cell can also be caused to be attached to a fresh gas-liquid interface 255 generated by enlargement of the surface area before the contaminant 257 is attached to the gas-liquid interface 255. Enlarging the surface area of the gas-liquid interface 255 may be performed by controlling the charging of gas to the pressure generation unit 47 by the volume control unit 200 to be at a preset amount.

Fi.g. 13G illustragtes an example where the gas-liquid interface 255 is brought into contact with the cell while the volume of the air bubble is increased to enlarge the surface area of the gas-liquid interface 255. Adjustment is made such that a proximity of the outside of the inside of the nozzle is positioned right above the nerve cell differentiated from the human iPS cell being cultured in the complete medium (975a), and the air bubble was enlarged to bring the gas-liquid interface 255 into contact with the cell (975b). Then, with the gas-liquid interface 255 in contact with the cell, the volume of the air bubble was increased, and the surface area of the gas-liquid interface 255 was enlarged (975c). As a result, it was possible to cause the cell to be attached to the gas-liquid interface 255. At 976d, since the cell is attached to the gas-liquid interface 255 to be captured in the flow channel of the nozzle, the cell is moved upwardly and the image of the cell is defocused.

In addition, at 943, by decreasing the volume of the air bubble and reducing the surface area of the gas-liquid interface 255, the cell attached to the gas-liquid interface 255 may be made easier to be withdrawn from the gas-liquid interface 255. Since the surface free energy at the gas-liquid interface 255 falls due to reduction of the surface area of the gas-liquid interface 255, the occupied area of the contaminant 257 attached to the gas-liquid interface 255 is increased, and the cell is made easier to be withdrawn from the gas-liquid interface 255. In addition, decreasing of the area to which the cell is attached is also cause an impact. Reduction of the gas-liquid interface 255 may be performed by controlling, by the volume control unit 200, the pressure generation unit 47 to intake a preset amout of gas.

Table 2 shows the decrease amount ($\Delta G$) of free energy of the system when the diameter of the air bubble, the shape of the cell, or the diameter of the cell is changed, and the break-in distance at which a most stable state is obtained when the cell breaks into the air bubble. At this time, deformation of the air bubble and the cell is not taken into consideration. It is shown that the llarger the decrease amount ($\Delta G$) of free energy of the system, the more stable the attachment of the cell to the gas-liquid interface 255 is. It is apparent from the result for a case where the air bubble diameter is 500 $\mu$m, the cell shape is spherical, and the cell diameter is 20 $\mu$m and the result for a case where the air bubble diameter is 100 $\mu$m in Table 2, that the cell shape is spherical, and the cell diameter is 20 $\mu$m that the diameter of the air bubble, that is, the size of the air bubble provides little impact to the reduction in free energy of the system.

[Table 2]

| AIR BUBBLE DIAMETER ($\mu$m) | CELL SHAPE | CELL DIAMETER ($\mu$m) | DECREAESE AMOUNT OF FREE ENERGY OF SYSTEM (nJ) | MOST STABLE BREAK-IN DISTANCE ($\mu$m) |
|---|---|---|---|---|
| 500 | SPHERICAL | 20 | $-1.2 \times 10^{-4}$ | 0.72 |
| 500 | SPHERICAL | 40 | $-4.9 \times 10^{-4}$ | 1.72 |
| 500 | SPHERICAL | 100 | $-32.4 \times 10^{-4}$ | 4.57 |
| 500 | FLAT | DIAMETER: 100 THICKNESS: 10 | $-87.0 \times 10^{-4}$ | 3.86 |
| 100 | SPHERICAL | 20 | $-1.3 \times 10^{-4}$ | 0.84 |

In addition, It is apparent from the result for a case where the air bubble diameter is 500 $\mu$m, the cell shape is spherical, and the cell diameter is 100 $\mu$m and the result for a case where the air bubble diameter is 500 $\mu$m, the cell shape is flat, and the cell diameter is 100 $\mu$m in Table 2 that, compared to a spherical shape, a flat shape of the cell the contributes more to the reduction in free energy of the system. At this time, a flat cell is assumed to be in a state where the cell surface(the surface having the largest surface area) is attached to the air bubble. Further, It is apparent from the result for a case where the air bubble diameter is 500 $\mu$m, the cell shape is spherical, and the cell diameter is 20 $\mu$m and the result for a case where the air bubble diameter is 500 $\mu$m, the cell shape is spherical, and the cell diameter is 100 $\mu$m in Table 2 that, a larger diameter of the cell, that is, a larger size of the cell contributes more to the reduction in free energy of the system. Accordingly, it is apparent from the results in Table 2 that it is easier for the cell to be attached to the gas-liquid interface 255 when the shape of the cell is flat and/or the size of the cell is larger.

In addition, at 945, since the values of $\gamma_{GC}$ (E1) and $\gamma_{GL}$ (E2) are different depending on the type of gas, the size of surface free energy at the gas-liquid interface 255 can be regulated by varying the type and composition of the gas. For example, when the existing gas is air, gas to be changed may be monoatomic molecule gas (for example, helium, neon, argon or the like), diatomic molecule (for example, nitrogen, oxygen or the like), polyatomic molecule (for example, carbon dioxide, methane or the like), or mixed gas thereof. The volume control unit 200 controls the pressure generation unit 47 and the nozzle actuator 40 to perform intake of the gas to be changed. For example, the volume control unit 200 may send an instruction to the nozzle actuator 40 to take the nozzle 49 out from the liquid. After the nozzle actuator 40 has taken the nozzle 49 out from the liquid, the pressure generation unit 47 may pull the plunger of the syringe pump to intake the gas to be changed. For example, the volume control unit 200 may send an instruction to the nozzle actuator 40 to put the nozzle 49 in the liquid. After the nozzle actuator 40 has put the nozzle 49 in the liquid, the pressure generation unit 47 may press the plunger of the syringe pump to charge the gas to be changed.

[Examples]

[Example 1] A HeLa cell which is an established cell was cultured for two days in a culture dish with a DMEM medium having 10 % fetal bovine serume added to the complete medium. The complete medium was removed and replaced with PBS buffer. Using 10 to 100 HeLa cells as manipulation targets, the relative position between the cell which is the manipulation target and the nozzle was adjusted such that the end of the nozzle comes to a position near the cell which is the manipulation target. At 100 $\mu$m from the bottom surface. Then, air was charged from the pump at a rate of 10 $\mu$L/second, and an air bubble was formed at the end of the nozzle (the inner diameter of the flow channel being 0.5 mm). The cell which is the manipulation target was brought into contact with the gas-liquid interface of the air bubble having a volume of about 0.02 mm$^3$ (the air bubble volume from the nozzle end), for the cell to be exfoliated and attached thereto, and the attached cell was captured in the flow channel of the nozzle. The captured cell was moved by being released into another culture dish containing a DMEM medium, which is a complete medium, having 10% fetal bovine serum added thereto, to be subcultured.

[Example 2] A nerve cell obtained by differentiating a human iPS cell was cultured in a culture dish for seven days with Neurobasal medium having appropriate cytokine added thereto. Using one nerve cell as the manipulation target, the relative position of the cell which is the manipulation target and the nozzle was adjusted such that the end of the nozzle comes to a position near the cell which is the manipulation target and 30 $\mu$m from the bottom surface. Then, air was charged from the pump at a rate of 2 $\mu$L/second, and an air bubble was formed at the end of the nozzle (the inner diameter of the flow channel being 0.1 mm). Two seconds after starting to form the air bubble, the cell which is the manipulation target is brought into contact with the gas-liquid interface of the air bubble having a volume of 0.0002 mm$^3$ (the air bubble volume from the nozzle end), and caused to be exfoliated and attached. The attached cell was captured in the flow channel of the nozzle, and released into a PCR tube containing 12.5 $\mu$L of cell lysate (in manner similar to 835b in Fig. 11G), to analyze the target mRNA.

[Example 3] A nerve cell obtained by differentiating a human iPS cell was cultured in a culture dish for seven days with Neurobasal medium having appropriate cytokine added thereto. Using one nerve cell as the manipulation target, the relative position between the cell which is the manipulation target and the nozzle was adjusted such that the end of the nozzle is at 30 $\mu$m from the bottom surface and the cell which is the manipulation target is at a proximity on the outside of the flow channel outlet inside of the end of the nozzle. Then, air was charged from the pump at a rate of 2 $\mu$L/second, and an air bubble was formed at the end of the nozzle (the inner diameter of the flow channel being 0.1 mm). At this time, the increasing speed of the surface area of the air bubble was 0.0015 mm$^2$/second. The cell which is the manipulation target is brought into contact with the gas-liquid interface of the air bubble over the course of forming the air bubble to be enlarged to an air bubble volume of 0.0002 mm$^3$ (the airbubble volume from the nozzle end), and was exfoliated and attached thereto. The attached cell was captured in the flow channel of the nozzle, released to a PCR tube containing 12.5 $\mu$L of cell lysate (similar to 835b in Fig. 11G), to analyze the target mRNA.

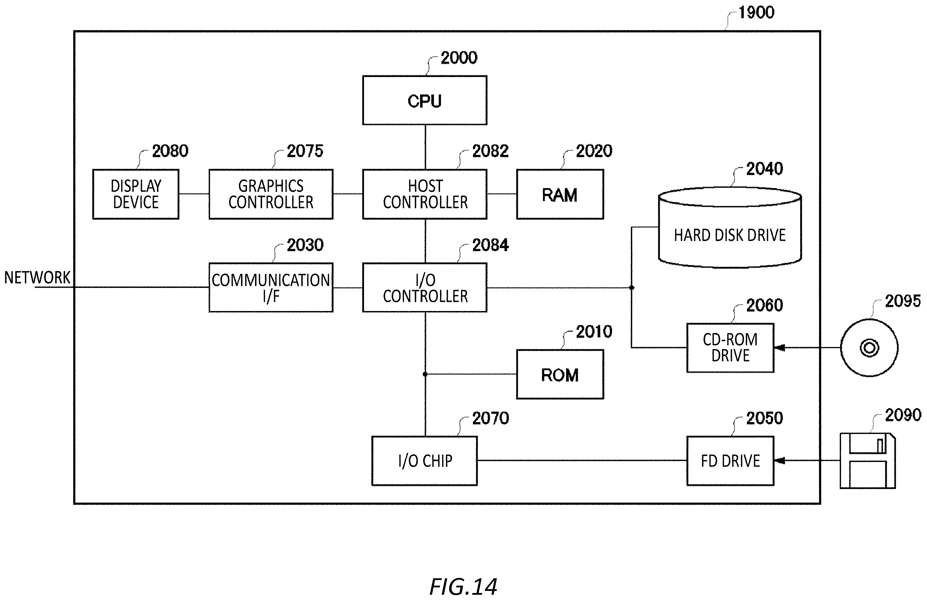

Fig. 14 illustrates an example of a hardware configuration of a computer 1900 that functions as the information processing device 170. The computer 1900 according to the present embodiment includes: a CPU-peripheral portion having a CPU 2000, a RAM 2020, a graphics controller 2075, and a display device 2080 interconnected by a host controller 2082; and an input/output unit having communication interface 2030, a hard disk drive 2040, and a CD-ROM drive 2060 connected to the host controller 2082 by an input/output controller 2084; and a legacy input/output unit having a ROM 2010, a flexible disk drive 2050, and an input/output chip 2070 connected to the input/output controller 2084.

The host controller 2082 connects the RAM 2020 with the CPU 2000 and the graphics controller 2075 accessing the RAM 2020 at a high transfer rate. The CPU 2000 operates based on programs stored in the ROM 2010 and the RAM 2020, and controls each unit. The graphics controller 2075 acquires image data generated by the CPU 2000 or the like on a frame buffer provided inside the RAM 2020 and display the image data on the display device 2080. Alternatively, the graphics controller 2075 may include therein a frame buffer storing the image data generated by the CPU 2000 or the like. Various pieces of information (for example, an image, location information of the manipulation target 35 or the like) generated inside the information processing device 170 can be displayed on the display device 2080.

The input/output controller 2084 connects the communication interface 2030, the hard disk drive 2040, and the CD-ROM drive 2060 which are relatively fast input/output devices to the host controller 2082. The communication interface 2030 communicates with other devices via a network by wire or wirelessly. In addition, the communication interface functions as a hardware to perform communications. The hard disk drive 2040 stores a program and data to be used by the CPU 2000 in the computer 1900. The CD-ROM drive 2060 reads a program or data from the CD-ROM 2095 and provide the hard disk drive 2040 via the RAM 2020.

In addition, the ROM 2010, and the flexible disk drive 2050 and input/output chip 2070, which are relatively low-speed input/output devices, are connected to the input/output controller 2084. The ROM 2010 stores a boot program performed when the computer 1900 starts up, and/or a program relying on the hardware of the computer 1900, and the like. The flexible disk drive 2050 reads out a program or data from the flexible disk 2090, and provide it to the hard disk drive 2040 via the RAM 2020. The input/output chip 2070 connects the flexible disk drive 2050 to the input/output controller 2084, and connects various types of input/output devices to the input/output controller 2084, for example, via a parallel port, a serial port, a keyboard port, a mouse port, or the like.

The program provided to the hard disk drive 2040 via the RAM 2020 is stored in a recording medium, such as the flexible disk 2090, the CD-ROM 2095, or an IC card, and provided by a user. The program is read out from the recording medium, installed on the hard disk drive 2040 in the computer 1900 via the RAM 2020, and executed in the CPU 2000.

The programs installed on the computer 1900 to cause the computer 1900 to function as the information processing device 170 include an air bubble forming module, an energy controlling module, and a manipulating module. These programs or modules may act on the CPU 2000 or the like to cause the computer 1900 to function as the volume control unit 200, the liquid control unit 260 or the like.

The information processing described in these programs are read by the computer 1900 to function as the volume control unit 200, the liquid control unit 260 or the like, which are specific means in which a software and various hardware resources described above cooperate with one another. Using these specific means, by achieving an operation or fabrication of information according to the intended use of the computer 1900 in the present embodiment, the information processing device 170 specific for the intended use is constructed.

By way of example, when communication is performed between the computer 1900 and an external device or the like, the CPU 2000 executes the communication program loaded on the RAM 2020, and provide the communication interface 2030 with communication processing instructions based on the content of the process written in the communication program. In response to the control by the CPU 2000, the communication interface 2030 reads out the transmission data stored in the transmission buffer region or the like provided on the storage device, such as the RAM 2020, the hard disk drive 2040, the flexible disk 2090, the CD-ROM 2095, or the like, and transmit this transmission data to the network, and write reception data received from the network onto a reception buffer region or the like provided on the storage device. In this way, the communication interface 2030 may transfer transmission/reception data to the storage device through DMA (Direct Memory Access) scheme, and alternatively, the CPU 2000 may transfer the transmission/reception data by reading the data from the storage device or communication interface 2030 that are the origins of transfer, and writing the data onto the communication interface 2030 or the storage device that are the destinations of transfer.

In addition, the CPU 2000 causes all or necessary portions of files or database stored in an external storage device such as the hard disk drive 2040, the CD-ROM drive 2060 (CD-ROM 2095), and the flexible disk drive 2050 (flexible disk 2090) to be read into the RAM 2020 by means of DMA transfer or the like, and then performs various types of processing on the data in the RAM 2020. The CPU 2000 writes back the data on which processing is completed into an external storage device by DMA transfer or the like. In such processing, the RAM 2020 can be regarded as carrying contents of the external storage device temporarily, and thus the RAM 2020, the external storage device and the like are collectively called a memory, a recording unit, a storage device or the like in the present embodiment.

Here, the storage device or the like stores information required for information processing by the information processing device 170, for example, moving image data, as required, and supplies the same to each component of the information processing device 170, as required.

Various types of information such as various types of programs, data, tables, databases or the like in the present embodiment according to the present embodiment are stored on such a storage device, and are subjected to information processing. Note that, the CPU 2000 can carry a part of the RAM 2020 in a cache memory and read from or write to the cache memory. In such a configuration as well, the cache memory serves a part of the function of the RAM 2020, and therefore the cache memory is also included with the RAM 2020, the memory, and/or the storage device in the present embodiment, except when it is shown with distinction.

In addition, the CPU 2000 executes various types of processing including various types of computations, information processing, conditional determination, information search/replacement, or the like described in the present embodiment for the data read from the RAM 2020, as specified by the instruction sequence of the program, and writes the result back onto the RAM 2020. For example, when performing conditional determination, the CPU 2000 compares various types of variables shown in the present embodiment to determine whether they satisfy conditions such as being larger than, smaller than, equal to or greater than, less than or equal to, equal to or like other variables or constants, and if a condition is satisfied (or if it is not satisfied) branches to a different instruction sequence or calls up a subroutine.

In addition, the CPU 2000 can search for information stored in a file in the storage device or the database, or the like. For example, if a plurality of entries, each having an attribute value of a second attribute associated with an attribute value of a first attribute, are stored in a storage device, the CPU 2000 searches, from among the plurality of entries stored in the storage device, an entry having an attribute value of the first attribute that matches a specified condition, and reads out the attribute value of the second attribute stored in the entry, and it is thereby possible to obtain the attribute value of the second attribute associated with the first attribute that satisfies a predetermined condition.

The programs or modules shown above may also be stored in an external recording medium. As a recording medium, other than the flexible disk 2090 and the CD-ROM 2095, an optical recording medium such as DVD or CD, a magneto-optical recording medium such as MO, a tape medium, a semiconductor memory, such as IC card, or the like can be used. Also, a storage device such as a hard disk or RAM that is provided with a server system connected to the Internet or a specialized communication network may be used as the recording medium to provide the programs to the computer 1900 via the network.

Although a configuration in which the information processing device 170 includes the CPU 2000 as a processor has been illustrated in the present disclosure, the type of the processor is not particularly limited therto. For example, as a processor, GPU, ASIA, FPGA or the like can be used appropriately. In addition, although a configuration in which the information processing device 170 includes the hard disk drive 2040 as an auxiliary storage device in present disclosure, the type of auxiliary storage device is not particularly limited thereto. For example, instead of the hard disk drive 2040 or in addition to the hard disk drive 2040, another storage device such as a solid state drive may be used.

While the present invention has been described with the embodiments, the technical scope of the present invention is not limited to the above-described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an device, system, program, and method shown in the claims, embodiments, or diagrams can be performed in any order as long as the order is not indicated by "prior to," "before," or the like and as long as the output from a previous process is not used in a later process. Even if the operation flow is described by using phrases such as "first" or "next" in the scope of the claims, specification, or drawings, it does not necessarily mean that the process must be performed in this order.

(Supplementary note)

[Item 1] A manipulation method of an organism, comprising:

forming an air bubble by introducing gas into liquid in which an organism is submerged;

controlling of energy before, during or after the forming of the air bubble, including controlling of difference (E1 - E2) obtained by subtracting, from surface free energy E1 at an interface between the gas and the organism, surface free energy E2 at an interface between the gas and the liquid; and

manipulating the organism using the air bubble, by bringing the air bubble into contact with the organism during or after the controlling of energy.

[Item 2] The manipulation method according to item 1, wherein

the controlling of energy includes decreasing the difference (E1 - E2) or suppressing the difference (E1 - E2) from being increased, and

the manipulating includes allowing the organism to be attached to a gas-liquid interface between the air bubble and the liquid.

[Item 3] The manipulation method according to item 1 or 2, wherein
the controlling of energy includes increasing the difference (E1 - E2) or suppressing the difference (E1 - E2) from being decreased.

[Item 4] The manipulation method according to item 3, wherein
the manipulating includes the organism being squeezed by a gas-liquid interface between the air bubble and the liquid.

[Item 5] The manipulation method according to any one of items 1 to 4, wherein
the controlling of energy includes controlling the surface free energy E2.

[Item 6] The manipulation method according to item 5, wherein
the controlling of energy includes replacing at least a part of the liquid with another liquid.

[Item 7] The manipulation method according to item 6, wherein the controlling of energy includes replacing the liquid with another liquid having surface free energy E3 at the interface with the air bubble, wherein E3 is greater than E2.

[Item 8] The manipulation method according to item 5, wherein the controlling of energy includes add another liquid to the liquid.

[Item 9] The manipulation method according to item 5, wherein the controlling of energy includes adding inorganic salt to the liquid.

[Item 10]The manipulation method according to item 5, wherein the controlling of energy includes removing a polar organic compound from the liquid.

[Item 11]The manipulation method according to item 6, wherein the controlling of energy includes replacing the liquid with another liquid having surface free energy E4 at the interface with the gas, wherein E4 is smaller than E2.

[Item 12]The manipulation method according to item 5, wherein the controlling of energy includes adding a polar organic compound to the liquid.

[Item 13]The manipulation method according to any one of items 1 to 4, wherein the controlling of energy includes replacing at least a part of the gas with another gas.

[Item 14]The manipulation method according to any one of items 1 to 13, wherein the controlling of energy includes allowing an air bubble formed in the forming of the air bubble to be attached to the organism within ten seconds after the air bubble being formed.

[Item 15]The manipulation method according to any one of items 1 to 13, wherein
the controlling of energy includes allowing an air bubble formed in the forming of the air bubble to be attached to the organism after fifteen seconds or more has elapsed since the air bubble was formed.

[Item 16]The manipulation method according to any one of items 1 to 5, wherein
the controlling of energy includes enlarging or reducing a gas-liquid interface of the air bubble formed in the forming of the air bubble.

[Item 17]The manipulation method according to any one of items 1 to 5, wherein
the controlling of energy includes controlling introduction speed or suction speed of the gas in the forming of the air bubble.

[Item 18]The manipulation method according to any one of items 1 to 17, wherein the controlling of energy includes controlling the temperature of the liquid.

[Item 19]The manipulation method according to any one of items 1 to 18, wherein the controlling of energy includes controlling the humidity of the air bubble.

[Item 20]The manipulation method according to any one of items 1 to 19, wherein
the manipulating includes manipulating the organism by bringing the interface between the liquid and the air bubble into contact with the organism and moving the interface.

[Item 21]The manipulation method according to item 20, wherein

the forming of the air bubble includes submerging an end of a flow channel to which the gas is injectable in liquid, and introducing the gas into the liquid from the end,

the forming of the air bubble further including moving relative positions of the flow channel and the organism to become closer.

[Item 22]The manipulation method according to item 21, wherein
the manipulating includes bringing the interface between the liquid and the air bubble into contact with the organism, and collecting the organism in contact with the interface by capturing the interface into the flow channel.

[Item 23]The manipulation method according to item 22, wherein
the manipulating includes capturing the interface into the flow channel within ten seconds after bringing the interface between the liquid and the air bubble into contact with the organism.

[Item 24]An organism manipulation device for manipulating an organism, comprising:

a flow channel into which gas is introduced from an end arranged in liquid in which the organism is submerged, to form an air bubble at the end;

an energy control unit which controls a difference (E1 - E2) obtained by subtracting, from surface free energy E1 at an interface between the gas and the organism, surface free energy E2 at an interface between the gas and the liquid; and

a manipulation unit which manipulates the organism with the air bubble.

[Item 25]The organism manipulation device according to item 24, wherein
the energy control unit has a volume control unit which causes the manipulation unit to control a pump connected to the flow channel, thereby controlling volume of the air bubble in the liquid.

[Item 26]The organism manipulation device according to item 24 or 25, further comprising:

a liquid storage unit which stores another liquid that may change the difference (E1-E 2) of surface free energy; and

a liquid flow channel which allows another liquid to flow therethrough from the liquid storage unit, wherein

the energy control unit includes a liquid control unit which adds another liquid to the liquid or replaces the liquid at least in part with another liquid, via the liquid flow channel.

[Item 27]The organism manipulation device according to any one of items 24 to 26, wherein the energy control unit has a temperature control unit which controls the temperature of the liquid.

[Item 28]The organism manipulation device according to any one of items 24 to 27, wherein the energy control unit has a humidity control unit which controls the humidity of the gas.

[Item 29]The organism manipulation device according to any one of items 24 to 28, further comprising
a microscope unit which displays the organism in an enlarged manner.

[Item 30]The organism manipulation device according to any one of items 24 to 29, wherein

the manipulation unit manipulates the organism by bringing the interface between the liquid and the air bubble into contact with the organism and moving the interface.

[Item 31]The organism manipulation device according to item 30, wherein
the manipulation unit includes collecting the organism by capturing the interface in the flow channel.

[Item 32]The organism manipulation device according to any one of items 24 to 31, wherein
the volume control unit controls the introduction speed or suction speed of the gas.

[Item 33]A computer program having instructions inside, wherein

when the instruction is executed by the processor or the programmable circuit,

the processor or the programmable circuit controls operations including:

forming an air bubble by introducing gas into liquid in which an organism is submerged;

controlling of energy before, during or after the forming of the air bubble, including controlling of difference (E1 - E2) obtained by subtracting, from surface free energy E1 at an interface between the gas and the organism, surface free energy E2 at an interface between the gas and the liquid; and

forming an airbubble to manipulate the organism at an end during or after the controlling of energy.

[Item 34] An organism manipulation device comprising:

a flow channel having an end arranged in liquid including an organism, which is supplied in a container;

a pump which introduces gas into the flow channel to form an air bubble at the end; and

a position control unit which controls a position of the container or the flow channel, wherein

the position control unit moves the container or the flow channel to a position at which the air bubble can be brought into contact with the organism, and

the pump forms the air bubble at the position and to bring the air bubble into contact with the organism while increasing the air bubble's volume.

EXPLANATION OF REFERENCES

1: fluorescence image observation light source,
2: dichroic mirror,
3: optical deflector,
4: relay lens,
5: dichroic mirror,
6: objective lens,
7: condenser lens,
8: condenser lens,
9: band-pass filter
10: transmission image observation light source,
11: barrier filter,
12: projection lens,
13: barrier filter,
14: projection lens,
15: pinhole,
16: light source,
17: light source,

25: container,
31: Nomarski prism,
32: analyzer (polarizing plate),
35: manipulation target,
37: polarizer (polarizing plate),
38: Nomarski prism,
39: ring diaphragm,
40: nozzle actuator,
41: sample actuator,
42: flow channel capturing camera,
45: light source,
46: light source,
47: pressure generation unit,
48: sensor unit,
49: nozzle,
50: microscope unit,
51: flow channel,
51a: first flow channel,
51b: second flow channel,
53: flow channel replacement unit,
54: liquid storage unit,
58: sample lid,
59: sample lid retaining unit,
60: camera,
70: camera,
100: organism manipulation device,
101: manipulation unit,
111: display area,
112: display area,
113: display area,
114: display area,
115: display area,
160: output unit,
170: information processing device,
171: imaging control unit,
180: input unit,
190: recording unit,
200: volume control unit,
250: flow channel control unit,
251: pump,
251a: first pump,
251b: second pump,
253: cylindrical portion,
253a: outer cylinder,
253b: inner cylinder,
254: end,
255: gas-liquid interface,
256: air bubble,
257: contaminant,
260: liquid control unit,
261: liquid,
300: image processing unit,
500: energy control unit,
1900: computer,
2000: CPU,
2010: ROM,
2020: RAM,
2030: communication interface,

2040 hard disk drive,
2050: flexible disk drive,
2060: CD-ROM drive,
2070: input/output chip,
2075: graphics controller,
2080: display device,
2082: host controller,
2084: input/output controller,
2090: flexible disk,
2095: CD-ROM.

**Claims**

1. A manipulation method of an organism, comprising:

    forming an air bubble by introducing gas into liquid in which an organism is submerged;
    controlling of energy before, during or after the forming of the air bubble, including controlling of difference (E1 - E2) obtained by subtracting, from surface free energy E1 at an interface between the gas and the organism, surface free energy E2 at an interface between the gas and the liquid; and
    manipulating the organism using the air bubble, by bringing the air bubble into contact with the organism during or after the controlling of energy.

2. The manipulation method according to claim 1, wherein

    the controlling of energy includes decreasing the difference (E1 - E2) or suppressing the difference (E1 - E2) from being increased, and
    the manipulating includes allowing the organism to be attached to a gas-liquid interface between the air bubble and the liquid.

3. The manipulation method according to claim 1 or 2, wherein
   the controlling of energy includes increasing the difference (E1 - E2) or suppressing the difference (E1 - E2) from being decreased.

4. The manipulation method according to claim 3, wherein
   the manipulating includes the organism being squeezed by a gas-liquid interface between the air bubble and the liquid.

5. The manipulation method according to any one of claims 1 to 4, wherein
   the controlling of energy includes controlling the surface free energy E2.

6. The manipulation method according to claim 5, wherein
   the controlling of energy includes replacing at least a part of the liquid with another liquid.

7. The manipulation method according to claim 5, wherein
   the controlling of energy includes adding another liquid to the liquid.

8. The manipulation method according to claim 5, wherein
   the controlling of energy includes adding a polar organic compound or inorganic salt to the liquid.

9. The manipulation method according to any one of claims 1 to 4, wherein
   the controlling of energy includes replacing at least part of the gas with another gas.

10. The manipulation method according to any one of claims 1 to 9, wherein
    the controlling of energy includes allowing an air bubble formed in the forming of the air bubble to be attached to the organism within ten seconds after the air bubble being formed.

11. The manipulation method according to any one of claims 1 to 9, wherein
    the controlling of energy includes allowing an air bubble formed in the forming of the air bubble to be attached to the organism after fifteen seconds or more has elapsed since the air bubble was formed.

12. The manipulation method according to any one of claims 1 to 5, wherein
the controlling of energy includes enlarging or reducing a gas-liquid interface of the air bubble formed in the forming of the air bubble.

13. The manipulation method according to any one of claims 1 to 5, wherein
the controlling of energy includes controlling introduction speed or suction speed of the gas in the forming of the air bubble.

14. The manipulation method according to any one of claims 1 to 13, wherein
the manipulating includes manipulating the organism by bringing the interface between the liquid and the air bubble into contact with the organism and moving the interface.

15. The manipulation method according to claim 14, wherein

the forming of the air bubble includes submerging an end of a flow channel to which the gas is injectable in liquid, and introducing the gas into the liquid from the end,
the forming of the air bubble further including moving relative positions of the flow channel and the organism to become closer.

16. The manipulation method according to claim 15, wherein
the manipulating includes bringing the interface between the liquid and the air bubble into contact with the organism, and collecting the organism in contact with the interface by capturing the interface into the flow channel.

17. The manipulation method according to claim 16, wherein
the manipulating includes capturing the interface into the flow channel within ten seconds after bringing the interface between the liquid and the air bubble into contact with the organism.

18. An organism manipulation device for manipulating an organism, comprising:

a flow channel into which gas is introduced from an end arranged in liquid in which the organism is submerged, to form an air bubble at the end;
an energy control unit which controls a difference (E1 - E2) obtained by subtracting, from surface free energy E1 at an interface between the gas and the organism, surface free energy E2 at an interface between the gas and the liquid; and
a manipulation unit whjch manipulates the organism with the air bubble.

19. The organism manipulation device according to claim 18, wherein
the energy control unit has a volume control unit causes the manipulation unit to control a pump connected to the flow channel, thereby controlling volume of the air bubble in the liquid.

20. An organism manipulation device comprising:

a flow channel having an end arranged in liquid including an organism, which is supplied in a container;
a pump which introduces gas into the flow channel to form an air bubble at the end; and
a position control unit which controls a position of the container or the flow channel, wherein
the position control unit is which moves the container or the flow channel to a position at which the air bubble can be brought into contact with the organism, and
the pump forms the air bubble at the position and to bring the air bubble into contact with the organism while increasing the air bubble's volume.

*FIG.1A*

*FIG.1B*

FIG.2A

*FIG.2B*

<u>49</u>

251a
251b

253
51a
51b
254

253a
253b

*FIG.3A*

49

253
51a
51b
261
254
255

*FIG.3B*

FIG.4

INFORMATION
PROCESSING DEVICE

IMAGING CONTROL UNIT
171

RECORDING UNIT
190

FLOW CHANNEL CONTROL UNIT
250

IMAGE PROCESSING UNIT
300

VOLUME CONTROL UNIT
200

LIQUID CONTROL UNIT
260

TEMPERATURE CONTROL UNIT
520

HUMIDITY CONTROL UNIT
530

ENERGY CONTROL UNIT
500

170

*FIG.5*

FIG.6

*FIG.7A*

*FIG.7B*

| I D | COORDINATE | SIZE | MOVEMENT DESTINATION |
|---|---|---|---|
| aaa | $(X_{aaa}, Y_{aaa})$ | AAA | A1 |
| bbb | $(X_{bbb}, Y_{bbb})$ | BBB | A2 |
| ccc | $(X_{ccc}, Y_{ccc})$ | CCC | A3 |
| | | | |
| | | | |

*FIG.7C*

FIG.7D

APPLICATION

| CELL COLLECTION (ANALYSIS) ▼ | ⎤113

MANIPULATED CELL FORM

◯ CYTOPLASM ⦿ SINGLE CELL ◯ CELL POPULATION (COLONY) ◯ SPHEROID ⎤114

MANIPULATION

⦿ COLLECTION ◯ RETAINING ◯ REMOVAL ◯ SQUEEZING ⎤115

*FIG.7E*

S600

○ FROM S160

LIQUID TO BE REMOVED? — Yes → REMOVE LIQUILD

S610     S615

No

ADD ADHESIVE FORCE
ADJUSTMENT REAGENT

S620

REPEAT? — Yes

S630

No

○ TO S180

*FIG.8*

S200

○ FROM S180

| OPERATE XYZ POSITION CONTROL ACTUATOR |

S210

| CAPTURE IMAGE OF NOZZLE END |

S215

DIFFERENT
FROM SET XYZ POSITION?    Yes →  | DECIDE OPERATING AMOUNT OF XYZ POSITION CONTROL ACTUATOR |

S220    S225

No

○ TO S300

*FIG.9A*

S200

○ FROM S180

OPERATE Z POSITION CONTROL ACTUATOR — S230

MEASURE LOAD OF Z POSITION CONTROL ACTUATOR — S235

LESS THAN OR EQUAL TO SET LOAD? — S240

No / Yes

Yes → DECIDE OPERATION AMOUNT OF Z POSITION CONTROL ACTUATOR — S242

SET INITIAL Z POSITION — S245

OPERATE XYZ POSITION CONTROL ACTUATOR — S250

CAPTURE IMAGE OF NOZZLE END — S252

DIFFERENT FROM SET XYZ POSITION? — S254

No / Yes

Yes → DECIDE OPERATING AMOUNT OF XYZ POSITION CONTROL ACTUATOR — S256

○ TO S300

*FIG.9B*

S200

○ FROM S180

| FORM AIR BUBBLE | S260

OPERATE Z POSITION CONTROL ACTUATOR — S262

MEASURE INNER PRESSURE OF AIR BUBBLE — S264

SET INNER PRESSURE CHANGED? — No → DECIDE OPERATIN AMOUNT OF Z POSITION CONTROL ACTUATOR — S268

S266

Yes

| REMOVE AIR BUBBLE | S270

SET INITIAL Z POSITION — S272

OPERATE XYZ POSITION CONTROL ACTUATOR — S274

CAPTURE IMAGE OF NOZZLE END — S276

DIFFERENT FROM SET XYZ POSITION? — Yes → DECIDE OPERATING AMOUNT OF XYZ POSITION CONTROL ACTUATOR — S282

S280

No

○ TO S300

FIG.9C

S300

○ FROM S200

OPERATE PUMP    S320

CAPTURE IMAGE OF NOZZLE END    S330

DIFFERENT FROM SET AIR BUBBLE SHAPE?    S340

Yes

DECIDE OPERATING AMOUNT OF PUMP    S342

No

○ TO S400

*FIG.10A*

S300

FROM S200

OPERATE PUMP  ~ S370

MEASURE INNER PRESSURE OF NOZZLE  ~ S380

DIFFERENT FROM SET INNER PRESSURE?  ~ S390

Yes

DECIDE OPERATING AMOUNT OF PUMP  ~ S392

No

TO S400

*FIG.10B*

S400

○ FROM S300

```
┌─────────────────────┐  Yes   ┌──────────────────────────────┐
│    UNNECESSARY      ├───────▶│  REMOVE UNNECESSARY CELL     ├──────── S412
│ CELL TO BE REMOVED? │        └──────────────────────────────┘
└──────────┬──────────┘ S410
        No │
```

FORM AIR BUBBLE ◀ S437 ◀ SUCK GAS S436 ◀ REMOVE AIR BUBBLE S435

```
┌─────────────────────┐  Yes   ┌──────────────────────────────┐        ┌─────────────────┐
│     CYTOPLASM       ├───────▶│ SEPARATE AND ATTACH CYTOPLASM├───────▶│  SUCCESSIVELY   │  Yes
│  TO BE COLLECTED?   │        └──────────────────────────────┘        │    COLLECT?     │
└──────────┬──────────┘ S420              S422                         └────────┬────────┘ S434
        No │                                                                 No │
```

```
┌─────────────────────┐  Yes   ┌──────────────────────────────┐
│  CELL TO BE COLLECTED? ├─────▶│    ATTACH CELL (EXFOLIATE)   │
└──────────┬──────────┘ S430   └──────────────────────────────┘ S432
        No │
```

```
┌─────────────────────┐  Yes   ┌──────────────────────┐   ┌──────────────────┐
│   CELL RETAINING    ├───────▶│ ATTACH CELL (EXFOLIATE)│─▶│  CAPTURE IMAGE   │
│    OBSERVATION?     │        └──────────────────────┘   └──────────────────┘ S444
└──────────┬──────────┘ S440              S442
        No │
```

```
┌─────────────────────┐  Yes   ┌──────────────┐   ┌──────────────────────────┐
│   CELL SQUEEZING    ├───────▶│ SQUEEZE CELL │──▶│     CAPTURE IMAGE,        │
│    OBSERVATION?     │        └──────────────┘   │  AND MEASURE PRESSURE     │
└──────────┬──────────┘ S450         S452         └──────────────────────────┘ S454
        No │
```

```
┌─────────────────────────┐
│ OTHER CELL MANIPULATION  ├──────── S460
└──────────┬──────────────┘
```

○ TO S500

*FIG.11A*

EP 4 219 673 A1

802a

802b

802c

802d

FIG.11B

FIG.11C

FIRST TIME

(AIR)

SECOND TIME

810a     810b     810c

*FIG.11D*

EP 4 219 673 A1

FIG.11E

|  | Day 4 :<br>TRANSMISSION<br>IMAGE OBSERVATION | Day 10 :<br>ALP STAIN (P1) | Day 30 :<br>ALP STAIN (P3) |
|---|---|---|---|
| EXAMPLE | 830a | 831a | 832a |
| COMPARATIVE<br>EXAMPLE | 830b<br>(n=4) | 831b<br>(n=4) | 832b<br>(n=3) |

*FIG.11F*

*FIG.11G*

FIG.11H

CELL WIDTH

CELL THICKNESS

CELL WIDTH

CELL THICKNESS

0.0 kPa

2.0 kPa

850a

850b

*FIG.11I*

S500

FROM S400

OPERATE PUMP —S510

CAPTURE IMAGE OF NOZZLE END —S520

DIFFERENT FROM SET GAS-LIQUID INTERFACE POSITION? — Yes → DECIDE OPERATING AMOUNT OF PUMP —S532

S530

No

WITHDRAW CELL FROM INTERFACE? — Yes → TAKE NOZZLE OUT FROM LIQUID —S542 → MOVE INTERFACE AT HIGH SPEED —S544

S540

No

TO S640

*FIG.12A*

73

## S500

○ FROM S400

```
OPERATE PUMP                                    S560

MEASURE INNER PRESSURE OF NOZZLE                S570
```

DIFFERENT
FROM SET INNER
PRESSURE?                    Yes →    DECIDE OPERATING
                                     AMOUNT OF PUMP
S580                                       S582

No

WITHDRAW
CELL FROM INTERFACE?         Yes →    TAKE NOZZLE        →    MOVE INTERFACE
                                     OUT FROM LIQUID          AT HIGH SPEED
S590                                       S592                    S594

No

○ TO S640

*FIG.12B*

$Y_{CL} \rightarrow$ CELL
LIQUID

$Y_{GL} \rightarrow$ GAS

$Y_{GC} \rightarrow$ CELL
GAS

961a

961b

*FIG.13A*

CONTROL FREE ENERGY AT
GAS-LIQUID INTERFACE

VARIATION TO TYPE OR COMPOSITION
OF SOLUTE IN LIQUID
910

VARIATION TO TEMPERATURE OF
GAS AND/OR LIQUID
920

VARIATION TO PRESSURE OF
GAS AND/OR LIQUID
925

VARIATION TO AMOUNT OF
VAPOR IN GAS
930

VARIATION TO TIME FROM WHEN
GAS-LIQUID INTERFACE IS FORMED
TO WHEN IT IS BROUGHT
TO CONTACT WITH CELL
935

VARIATION TO VOLUME
OF AIR BUBBLE
940

VARIATION TO TYPE OR
COMPOSITION OF GAS
945

ADD SOLUTE TO LIQUID
911

ADD OTHER LIQUID
912

REMOVE SOLUTE FROM LIQUID
913

REPLACE WITH ANOTHER LIQUID
914

VARIATION TO COMPOSITION
OF CONTAMINANTS
915

NEWLY FORM
GAS-LIQUID INTERFACE
941

INCREASE VOLUME
OF AIR BUBBLE
942

DECREASEVOLUME
OF AIR BUBBLE
943

FIG.13B

*FIG.13C*

FIG.13D

FIG.13E

970a    970b

FIG.13F

975a

975b

975c

975d

FIG.13G

*FIG.14*

EP 4 219 673 A1

## INTERNATIONAL SEARCH REPORT

| International application No. |
| --- |
| **PCT/JP2021/035192** |

### A. CLASSIFICATION OF SUBJECT MATTER

*C12M 1/00*(2006.01)i; *C12M 1/04*(2006.01)i; *C12M 1/26*(2006.01)i
FI:  C12M1/00 Z; C12M1/26; C12M1/04

According to International Patent Classification (IPC) or to both national classification and IPC

### B. FIELDS SEARCHED

Minimum documentation searched (classification system followed by classification symbols)

C12M1/00; C12M1/04; C12M1/26

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | JP 07-047259 A (HITACHI LTD) 21 February 1995 (1995-02-21) paragraphs [0001], [0101]-[0114], fig. 21 | 1-14, 18, 19 |
| A | entire text | 15-17, 20 |
| X | WO 2015/098919 A1 (SEKISUI CHEMICAL CO LTD) 02 July 2015 (2015-07-02) claims, fig. 1, 2 | 1-10, 13, 14, 18 |
| A | entire text | 15-17, 19, 20 |
| A | JP 2005-538287 A (MEMSFLOW APS) 15 December 2005 (2005-12-15) entire text, all drawings | 1-20 |
| A | JP 3187489 B2 (CANON INC) 11 May 2001 (2001-05-11) entire text, all drawings | 1-20 |
| A | WO 2014/057713 A1 (FURUKAWA ELECTRIC CO., LTD) 17 April 2014 (2014-04-17) entire text, all drawings | 1-20 |
| A | JP 2006-158335 A (OLYMPUS CORP) 22 June 2006 (2006-06-22) entire text, all drawings | 1-20 |

☐ Further documents are listed in the continuation of Box C.        ☑ See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **22 October 2021** | **09 November 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/035192**

| Patent document cited in search report | | | Publication date (day/month/year) | Patent family member(s) | | | Publication date (day/month/year) |
|---|---|---|---|---|---|---|---|
| JP | 07-047259 | A | 21 February 1995 | US 6216538 B1 column 1, lines 9-22, column 21, line 5 to column 23, line 48, fig. 21 | | | |
| WO | 2015/098919 | A1 | 02 July 2015 | (Family: none) | | | |
| JP | 2005-538287 | A | 15 December 2005 | WO 2004/016948 A1 entire text, all drawings EP 001534954 A1 US 2006/0051214 A1 | | | |
| JP | 3187489 | B2 | 11 May 2001 | (Family: none) | | | |
| WO | 2014/057713 | A1 | 17 April 2014 | EP 002908116 A1 entire text, all drawings US 2015/0198537 A1 | | | |
| JP | 2006-158335 | A | 22 June 2006 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H713083 B
- JP 3814869 B